# EUROPEAN PATENT APPLICATION

(11) **EP 2 966 086 A1**
(43) Date of publication of application: **13.01.2016**
(21) Application number: 14743735.4
(22) Date of filing: 28.01.2014
(51) Int. Cl.: C07K 16/10, A61K 39/155, A61P 31/14, C12N 1/15, C12N 1/19, C12N 1/21, C12N 5/10, C12N 15/09

(54) **HUMAN ANTIBODY SPECIFIC TO HUMAN METAPNEUMOVIRUS, OR ANTIGEN-BINDING FRAGMENT THEREOF**

(30) Priority: 28.01.2013 JP 2013013599
(71) Applicant: Evec Inc., Sapporo-shi Hokkaido 0600042 (JP)
(72) Inventor: TAKADA, Kenzo, Sapporo-shi Hokkaido 060-0042 (JP); NAKAJIMA, Kantou, Sapporo-shi Hokkaido 060-0042 (JP)
(74) Representative: EIP
(86) International application number: PCT/JP2014/051866
(87) International publication number: WO 2014/115893

(57) **Abstract**

The present invention provides a novel human-derived monoclonal antibody specifically binding to human metapneumovirus F protein and neutralizing the human metapneumovirus, and an antigen-binding fragment thereof. The present invention also provides a pharmaceutical composition comprising the antibody or an antigen-binding fragment thereof.

SEQUENCE LISTING

<110>  Evec Incorporated

<120>  Human Metapneumovirus-Specific Human Antibody or Antigen Binding
       Fragment Thereof

<130>  PCT13-0067

<150>  JP 2013-013599
<151>  2013-01-28

<160>  134

<170>  PatentIn version 3.5

<210>  1
<211>  457
<212>  PRT
<213>  Homo sapiens

<400>  1

Gln Val Gln Leu Val Glu Ser Gly Gly Gly Leu Val Arg Pro Gly Gly
1               5                   10                  15

Ser Leu Arg Leu Ser Cys Ala Ala Ser Gly Phe Thr Phe Ser Asp Tyr
            20                  25                  30

Phe Met Thr Trp Ile Arg Gln Ala Pro Gly Lys Gly Leu Glu Trp Val
        35                  40                  45

Ser Tyr Ile Asn Ser Ile Gly Ser Val Ile Phe Tyr Ala Asp Ser Val
    50                  55                  60

Glu Gly Arg Phe Thr Val Ser Arg Asp Asn Thr Met Lys Ser Leu Phe
65                  70                  75                  80

Leu Gln Met Asn Ser Leu Thr Ala Glu Asp Thr Ala Val Tyr Tyr Cys
                85                  90                  95

Ala Arg Ser Cys Arg Ile Ser Tyr Gly Ala Ser Gly Gly Phe Ser Cys
            100                 105                 110

Gly Met Asp Val Trp Gly Gln Gly Thr Pro Val Thr Val Ser Ser Ala
        115                 120                 125

Ser Thr Lys Gly Pro Ser Val Phe Pro Leu Ala Pro Ser Ser Lys Ser
    130                 135                 140

Thr Ser Gly Gly Thr Ala Ala Leu Gly Cys Leu Val Lys Asp Tyr Phe
145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Gln Thr Tyr
        195                 200                 205

Ile Cys Asn Val Asn His Lys Pro Ser Asn Thr Lys Val Asp Lys Lys
    210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Asp Glu Leu
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Gln Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro Gly Lys
    450                 455

<210> 2
<211> 127
<212> PRT
<213> Homo sapiens

<400> 2

Gln Val Gln Leu Val Glu Ser Gly Gly Gly Leu Val Arg Pro Gly Gly
1               5                   10                  15

Ser Leu Arg Leu Ser Cys Ala Ala Ser Gly Phe Thr Phe Ser Asp Tyr
            20                  25                  30

Phe Met Thr Trp Ile Arg Gln Ala Pro Gly Lys Gly Leu Glu Trp Val
        35                  40                  45

Ser Tyr Ile Asn Ser Ile Gly Ser Val Ile Phe Tyr Ala Asp Ser Val
    50                  55                  60

Glu Gly Arg Phe Thr Val Ser Arg Asp Asn Thr Met Lys Ser Leu Phe
65                  70                  75                  80

Leu Gln Met Asn Ser Leu Thr Ala Glu Asp Thr Ala Val Tyr Tyr Cys
                85                  90                  95

Ala Arg Ser Cys Arg Ile Ser Tyr Gly Ala Ser Gly Gly Phe Ser Cys
            100                 105                 110

Gly Met Asp Val Trp Gly Gln Gly Thr Pro Val Thr Val Ser Ser
        115                 120                 125

<210> 3
<211> 5
<212> PRT
<213> Homo sapiens

<400> 3

Asp Tyr Phe Met Thr
1               5

<210>  4
<211>  17
<212>  PRT
<213>  Homo sapiens

<400>  4

Tyr Ile Asn Ser Ile Gly Ser Val Ile Phe Tyr Ala Asp Ser Val Glu
1               5                   10                  15

Gly

<210>  5
<211>  18
<212>  PRT
<213>  Homo sapiens

<400>  5

Ser Cys Arg Ile Ser Tyr Gly Ala Ser Gly Gly Phe Ser Cys Gly Met
1               5                   10                  15

Asp Val

<210>  6
<211>  213
<212>  PRT
<213>  Homo sapiens

<400>  6

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Arg Glu Asn
            20                  25                  30

Ile Asn Trp Tyr Gln Gln Lys Thr Gly Lys Ala Pro Lys Leu Leu Ile
        35                  40                  45

Tyr Asp Ala Ser Ile Leu Glu Thr Gly Val Pro Ser Arg Phe Ser Gly
    50                  55                  60

Ser Gly Ser Gly Thr His Phe Thr Phe Thr Ile Ser Ser Leu Gln Pro
65                  70                  75                  80

Glu Asp Met Ala Thr Tyr Tyr Cys Gln Gln Tyr His His Leu Ile Thr
                85                  90                  95

Phe Gly Gln Gly Thr Arg Leu Asp Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  7
<211>  106
<212>  PRT
<213>  Homo sapiens

<400>  7

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Ser Ile Arg Glu Asn
            20                  25                  30

Ile Asn Trp Tyr Gln Gln Lys Thr Gly Lys Ala Pro Lys Leu Leu Ile
        35                  40                  45

Tyr Asp Ala Ser Ile Leu Glu Thr Gly Val Pro Ser Arg Phe Ser Gly
    50                  55                  60

Ser Gly Ser Gly Thr His Phe Thr Phe Thr Ile Ser Ser Leu Gln Pro
65                  70                  75                  80

Glu Asp Met Ala Thr Tyr Tyr Cys Gln Gln Tyr His His Leu Ile Thr
                85                  90                  95

Phe Gly Gln Gly Thr Arg Leu Asp Ile Lys
            100                 105

<210>  8
<211>  11
<212>  PRT
<213>  Homo sapiens

<400>  8

Gln Ala Ser Gln Ser Ile Arg Glu Asn Ile Asn
1               5                   10


<210>  9
<211>  7
<212>  PRT
<213>  Homo sapiens

<400>  9

Asp Ala Ser Ile Leu Glu Thr
1               5


<210>  10
<211>  8
<212>  PRT
<213>  Homo sapiens

<400>  10

Gln Gln Tyr His His Leu Ile Thr
1               5


<210>  11
<211>  448
<212>  PRT
<213>  Homo sapiens

<400>  11

Gln Val Gln Leu Val Gln Ser Gly Ala Glu Val Lys Lys Pro Gly Ala
1               5                   10                  15


Ser Val Lys Val Ser Cys Lys Ala Ser Gly Tyr Thr Phe Ser Val Tyr
            20                  25                  30


Gly Ile Ser Trp Val Arg Gln Ala Pro Gly His Gly Leu Glu Trp Met
        35                  40                  45


Gly Trp Ile Ser Ala Tyr Asn Gly Asp Arg Gly Tyr Ala Gln Lys Val
    50                  55                  60


Gln Gly Arg Ile Thr Met Thr Thr Asp Thr Ser Ala Ser Thr Ala Tyr
65                  70                  75                  80


Met Glu Leu Arg Ser Leu Thr Ser Asp Asp Thr Ala Val Tyr Tyr Cys

85                  90                  95

Ala Thr Thr Ser Ser Gly Val Pro Gly Ile Tyr Trp Gly Gln Gly Ser
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  12
<211>  118
<212>  PRT
<213>  Homo sapiens

<400>  12

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Val Tyr
            20                  25                  30

Gly Ile Ser Trp Val Arg Gln Ala Pro Gly His Gly Leu Glu Trp Met
        35                  40                  45

Gly Trp Ile Ser Ala Tyr Asn Gly Asp Arg Gly Tyr Ala Gln Lys Val
    50                  55                  60

Gln Gly Arg Ile Thr Met Thr Thr Asp Thr Ser Ala Ser Thr Ala Tyr
65                  70                  75                  80

Met Glu Leu Arg Ser Leu Thr Ser Asp Asp Thr Ala Val Tyr Tyr Cys
                85                  90                  95

Ala Thr Thr Ser Ser Gly Val Pro Gly Ile Tyr Trp Gly Gln Gly Ser
            100                 105                 110

Leu Val Thr Val Ser Ser
        115

<210>  13
<211>  5
<212>  PRT
<213>  Homo sapiens

<400>  13

Val Tyr Gly Ile Ser
1               5

<210>  14
<211>  17
<212>  PRT
<213>  Homo sapiens

<400>  14

Trp Ile Ser Ala Tyr Asn Gly Asp Arg Gly Tyr Ala Gln Lys Val Gln
1               5                   10                  15

Gly

<210>  15
<211>  9
<212>  PRT
<213>  Homo sapiens

<400>  15

Thr Ser Ser Gly Val Pro Gly Ile Tyr
1               5

<210>  16
<211>  217
<212>  PRT
<213>  Homo sapiens

<400>  16

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Tyr Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Asn Leu
                85                  90                  95

Ser Gly Pro Trp Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  17
<211>  111
<212>  PRT
<213>  Homo sapiens

<400>  17

Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Ser Asn
            20                  25                  30

Tyr Val Tyr Trp Tyr Gln Gln Leu Pro Gly Thr Ala Pro Lys Leu Leu
        35                  40                  45

Ile Tyr Arg Asn Asn Gln Arg Pro Ser Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Ala Ala Trp Asp Asp Asn Leu
                85                  90                  95

Ser Gly Pro Trp Val Phe Gly Gly Gly Thr Lys Leu Thr Val Leu
            100                 105                 110

<210>  18
<211>  13
<212>  PRT
<213>  Homo sapiens

<400>  18

Ser Gly Ser Ser Ser Asn Ile Gly Ser Asn Tyr Val Tyr
1               5                   10

<210>  19
<211>  7
<212>  PRT
<213>  Homo sapiens

<400>  19

Arg Asn Asn Gln Arg Pro Ser
1               5

<210>  20
<211>  12
<212>  PRT
<213>  Homo sapiens

<400>  20

Ala Ala Trp Asp Asp Asn Leu Ser Gly Pro Trp Val
1               5                   10

<210>  21
<211>  449
<212>  PRT
<213>  Homo sapiens

<400>  21

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Arg Asp
            20                  25                  30

Tyr Trp Ser Phe Ile Arg Gln Pro Ala Gly Lys Gly Leu Glu Trp Ile
        35                  40                  45

Gly Arg Ile Tyr His Asn Gly Asp Thr Asn Tyr Asn Pro Ser Leu Lys
    50                  55                  60

Ser Arg Val Thr Met Ser Gln Asp Thr Ser Lys Asn Gln Phe Phe Leu
65                  70                  75                  80

Arg Leu Arg His Val Thr Ala Ala Asp Thr Ala Val Tyr Tyr Cys Ala
                85                  90                  95

Arg Glu Gly Ser Ala Gly Thr Phe Gly Gln Ala Asp Trp Gly Gln Gly
            100                 105                 110

Thr Leu Val Ile Val Tyr Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val

290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  22
<211>  119
<212>  PRT
<213>  Homo sapiens

<400>  22

Gln Val Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Arg Asp
            20                  25                  30

Tyr Trp Ser Phe Ile Arg Gln Pro Ala Gly Lys Gly Leu Glu Trp Ile
        35                  40                  45

Gly Arg Ile Tyr His Asn Gly Asp Thr Asn Tyr Asn Pro Ser Leu Lys

50                  55                  60

Ser Arg Val Thr Met Ser Gln Asp Thr Ser Lys Asn Gln Phe Phe Leu
65                  70                  75                  80

Arg Leu Arg His Val Thr Ala Ala Asp Thr Ala Val Tyr Tyr Cys Ala
                85                  90                  95

Arg Glu Gly Ser Ala Gly Thr Phe Gly Gln Ala Asp Trp Gly Gln Gly
            100                 105                 110

Thr Leu Val Ile Val Tyr Ser
        115

<210>  23
<211>  5
<212>  PRT
<213>  Homo sapiens

<400>  23

Arg Asp Tyr Trp Ser
1               5

<210>  24
<211>  16
<212>  PRT
<213>  Homo sapiens

<400>  24

Arg Ile Tyr His Asn Gly Asp Thr Asn Tyr Asn Pro Ser Leu Lys Ser
1               5                   10                  15

<210>  25
<211>  11
<212>  PRT
<213>  Homo sapiens

<400>  25

Glu Gly Ser Ala Gly Thr Phe Gly Gln Ala Asp
1               5                   10

<210>  26
<211>  215
<212>  PRT
<213>  Homo sapiens

<400>  26

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Ile Ser Asn Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Gly Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Glu Asn Trp Leu Leu
                85                  90                  95

Tyr Thr Phe Gly Gln Gly Thr Lys Leu Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  27
<211>  108
<212>  PRT
<213>  Homo sapiens

<400>  27

Glu Ile Val Met Thr Gln Ser Pro Ala Thr Leu Ser Val Ser Pro Gly
1               5                   10                  15

Glu Arg Ala Thr Leu Ser Cys Arg Ala Ser Gln Ser Ile Ser Asn Asn
            20                  25                  30

Leu Ala Trp Tyr Gln Gln Lys Pro Gly Gln Ala Pro Arg Leu Leu Ile
        35                  40                  45

Tyr Gly Ala Ser Thr Arg Ala Thr Gly Ile Pro Ala Arg Phe Ser Gly
    50                  55                  60

Gly Gly Ser Gly Thr Glu Phe Thr Leu Thr Ile Ser Ser Leu Gln Ser
65                  70                  75                  80

Glu Asp Phe Ala Val Tyr Tyr Cys Gln Gln Tyr Glu Asn Trp Leu Leu
                85                  90                  95

Tyr Thr Phe Gly Gln Gly Thr Lys Leu Glu Ile Lys
            100                 105

<210>  28
<211>  11
<212>  PRT
<213>  Homo sapiens

<400>  28

Arg Ala Ser Gln Ser Ile Ser Asn Asn Leu Ala
1               5                   10

<210>  29
<211>  7
<212>  PRT
<213>  Homo sapiens

<400>  29

Gly Ala Ser Thr Arg Ala Thr
1               5

<210>  30
<211>  10
<212>  PRT
<213>  Homo sapiens

<400>  30

Gln Gln Tyr Glu Asn Trp Leu Leu Tyr Thr
1               5                   10

<210>  31
<211>  453
<212>  PRT
<213>  Homo sapiens

<400>  31

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Leu
    50                  55                  60

Lys Gly Arg Phe Thr Val Ser Arg Asp Asn Ala Lys Lys Ser Leu Tyr
65                  70                  75                  80

Leu Gln Met Asn Ser Leu Arg Ala Glu Asp Thr Ala Val Tyr Tyr Cys
                85                  90                  95

Ala Arg Val Asp Tyr Gly Thr Asn Ser Glu Val Gly Arg Phe Asp Pro
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210> 32
<211> 123
<212> PRT
<213> Homo sapiens

<400> 32

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
                20                  25                  30

Ser Met Asn Trp Val Arg Gln Ala Pro Gly Lys Gly Leu Glu Trp Val
        35                  40                  45

Ser Ser Ile Ser Ser Ser Ser Ser Tyr Ile Tyr Tyr Ala Asp Ser Leu
    50                  55                  60

Lys Gly Arg Phe Thr Val Ser Arg Asp Asn Ala Lys Lys Ser Leu Tyr
65                  70                  75                  80

Leu Gln Met Asn Ser Leu Arg Ala Glu Asp Thr Ala Val Tyr Tyr Cys
                85                  90                  95

Ala Arg Val Asp Tyr Gly Thr Asn Ser Glu Val Gly Arg Phe Asp Pro
            100                 105                 110

Trp Gly Gln Gly Thr Leu Val Thr Val Ser Ser
        115                 120

<210>  33
<211>  5
<212>  PRT
<213>  Homo sapiens

<400>  33

Ser Tyr Ser Met Asn
1               5

<210>  34
<211>  17
<212>  PRT
<213>  Homo sapiens

<400>  34

Ser Ile Ser Ser Ser Ser Ser Tyr Ile Tyr Tyr Ala Asp Ser Leu Lys
1               5                   10                  15

Gly

<210>  35
<211>  14
<212>  PRT
<213>  Homo sapiens

<400>  35

Val Asp Tyr Gly Thr Asn Ser Glu Val Gly Arg Phe Asp Pro
1               5                   10

<210>  36
<211>  217
<212>  PRT
<213>  Homo sapiens

<400>  36

Gln Ser Val Leu Thr Gln Pro Pro Ser Val Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Ala Val His Trp Tyr Gln Gln Leu Pro Gly Thr Ala Pro Lys Leu
        35                  40                  45

Leu Ile Tyr Asp Ser Ile Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Thr Glu Asp Glu Ala Asp Tyr Tyr Cys His Ser Tyr Asp Arg Ser
                85                  90                  95

Leu Ser Gly Ser Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Lys Ser Tyr Ser Cys Gln Val Thr His Glu Gly Ser Thr Val Glu
        195                 200                 205

Lys Thr Val Ala Pro Thr Glu Cys Ser
    210                 215

<210>  37
<211>  111
<212>  PRT
<213>  Homo sapiens

<400>  37

Gln Ser Val Leu Thr Gln Pro Pro Ser Val Ser Gly Thr Pro Gly Gln
1               5                   10                  15

Arg Val Thr Ile Ser Cys Thr Gly Ser Ser Ser Asn Ile Gly Ala Gly
            20                  25                  30

Tyr Ala Val His Trp Tyr Gln Gln Leu Pro Gly Thr Ala Pro Lys Leu
        35                  40                  45

Leu Ile Tyr Asp Ser Ile Asn Arg Pro Ser Gly Val Pro Asp Arg Phe
    50                  55                  60

Ser Gly Ser Lys Ser Gly Thr Ser Ala Ser Leu Ala Ile Thr Gly Leu
65                  70                  75                  80

Gln Thr Glu Asp Glu Ala Asp Tyr Tyr Cys His Ser Tyr Asp Arg Ser
                85                  90                  95

Leu Ser Gly Ser Val Phe Gly Gly Gly Thr Lys Leu Thr Val Leu
            100                 105                 110

<210>  38
<211>  14
<212>  PRT
<213>  Homo sapiens

<400>  38

Thr Gly Ser Ser Ser Asn Ile Gly Ala Gly Tyr Ala Val His
1               5                   10

<210>  39
<211>  7
<212>  PRT
<213>  Homo sapiens

<400>  39

Asp Ser Ile Asn Arg Pro Ser
1               5

<210>  40
<211>  11

<212>  PRT
<213>  Homo sapiens

<400>  40

His Ser Tyr Asp Arg Ser Leu Ser Gly Ser Val
1               5                   10


<210>  41
<211>  453
<212>  PRT
<213>  Homo sapiens

<400>  41

Gln Val Gln Leu Val Gln Ser Gly Thr Glu Leu Lys Lys Pro Gly Ala
1               5                   10                  15

Ser Val Lys Val Ser Cys Lys Ala Ser Gly Tyr Thr Phe Thr Asp Tyr
            20                  25                  30

Ser Met His Trp Leu Arg Gln Ala Pro Gly Glu Ser Leu Glu Trp Met
        35                  40                  45

Gly Trp Val Asn Gly Gly Pro Leu Asp Thr Lys Tyr Ser Pro Thr Phe
    50                  55                  60

Gln Asp Arg Val Ser Phe Ser Arg Asp Val Phe Ala Thr Thr Ala Tyr
65                  70                  75                  80

Met Asp Leu Ser Ser Leu Gly Pro Glu Asp Thr Ala Val Tyr Tyr Cys
                85                  90                  95

Ala Arg Gly Trp Gly Ile Thr Ala Tyr Arg Gly Asp Ala Phe Asp Leu
            100                 105                 110

Trp Gly Gln Gly Thr Met Val Ile Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Asp Glu Leu Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450

<210>  42
<211>  123
<212>  PRT
<213>  Homo sapiens

<400>  42

Gln Val Gln Leu Val Gln Ser Gly Thr Glu Leu Lys Lys Pro Gly Ala
1               5                   10                  15

Ser Val Lys Val Ser Cys Lys Ala Ser Gly Tyr Thr Phe Thr Asp Tyr
            20                  25                  30

Ser Met His Trp Leu Arg Gln Ala Pro Gly Glu Ser Leu Glu Trp Met
        35                  40                  45

Gly Trp Val Asn Gly Gly Pro Leu Asp Thr Lys Tyr Ser Pro Thr Phe
    50                  55                  60

Gln Asp Arg Val Ser Phe Ser Arg Asp Val Phe Ala Thr Thr Ala Tyr
65                  70                  75                  80

Met Asp Leu Ser Ser Leu Gly Pro Glu Asp Thr Ala Val Tyr Tyr Cys
                85                  90                  95

Ala Arg Gly Trp Gly Ile Thr Ala Tyr Arg Gly Asp Ala Phe Asp Leu
            100                 105                 110

Trp Gly Gln Gly Thr Met Val Ile Val Ser Ser
        115                 120

<210>  43
<211>  5
<212>  PRT
<213>  Homo sapiens

<400>  43

Asp Tyr Ser Met His
1               5

<210>  44
<211>  17
<212>  PRT
<213>  Homo sapiens

<400>  44

Trp Val Asn Gly Gly Pro Leu Asp Thr Lys Tyr Ser Pro Thr Phe Gln
1               5                   10                  15

Asp

<210>  45
<211>  14
<212>  PRT
<213>  Homo sapiens

<400>  45

Gly Trp Gly Ile Thr Ala Tyr Arg Gly Asp Ala Phe Asp Leu
1               5                   10

<210>  46
<211>  214
<212>  PRT
<213>  Homo sapiens

<400>  46

Ser Phe Val Leu Thr Gln Pro Pro Ser Val Ser Val Ala Pro Gly Lys
1               5                   10                  15

Ser Ala Arg Ile Thr Cys Gly Ala Ser Asn Ile Glu Ser Lys Ser Val
            20                  25                  30

His Trp Tyr Gln Gln Lys Pro Gly Gln Ala Pro Val Val Val Val Tyr
        35                  40                  45

Asp Asp Asp Asp Arg Pro Ser Gly Ile Pro Glu Arg Phe Ser Gly Ser
    50                  55                  60

Asn Ser Gly Asn Thr Ala Thr Leu Ile Ile Asn Arg Val Glu Val Gly
65                  70                  75                  80

Asp Glu Ala Asp Tyr Tyr Cys Gln Val Trp Asp Ser Thr Ser Asp Met
                85                  90                  95

Tyr Val Phe Gly Ala Gly Thr Lys Val Thr Val Leu Gly Gln Pro Lys
            100                 105                 110

Ala Asn Pro Thr Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Gly Ser Pro Val Lys Ala Gly

145                 150                 155                 160

Val Glu Thr Thr Lys Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  47
<211>  108
<212>  PRT
<213>  Homo sapiens

<400>  47

Ser Phe Val Leu Thr Gln Pro Pro Ser Val Ser Val Ala Pro Gly Lys
1               5                   10                  15

Ser Ala Arg Ile Thr Cys Gly Ala Ser Asn Ile Glu Ser Lys Ser Val
            20                  25                  30

His Trp Tyr Gln Gln Lys Pro Gly Gln Ala Pro Val Val Val Val Tyr
        35                  40                  45

Asp Asp Asp Asp Arg Pro Ser Gly Ile Pro Glu Arg Phe Ser Gly Ser
    50                  55                  60

Asn Ser Gly Asn Thr Ala Thr Leu Ile Ile Asn Arg Val Glu Val Gly
65                  70                  75                  80

Asp Glu Ala Asp Tyr Tyr Cys Gln Val Trp Asp Ser Thr Ser Asp Met
                85                  90                  95

Tyr Val Phe Gly Ala Gly Thr Lys Val Thr Val Leu
            100                 105

<210>  48
<211>  11
<212>  PRT
<213>  Homo sapiens

<400>  48

Gly Ala Ser Asn Ile Glu Ser Lys Ser Val His
1               5                   10

<210>  49
<211>  7
<212>  PRT
<213>  Homo sapiens

<400>  49

Asp Asp Asp Asp Arg Pro Ser
1               5


<210>  50
<211>  11
<212>  PRT
<213>  Homo sapiens

<400>  50

Gln Val Trp Asp Ser Thr Ser Asp Met Tyr Val
1               5                   10


<210>  51
<211>  450
<212>  PRT
<213>  Homo sapiens

<400>  51

Gln Val Gln Leu Val Glu Ser Gly Gly Gly Val Ala Gln Pro Gly Arg
1               5                   10                  15

Ser Leu Arg Leu Ser Cys Ala Ala Ser Gly Phe Thr Leu Ser Asn Tyr
            20                  25                  30

Gly Met His Trp Val Arg Gln Ala Pro Gly Lys Gly Leu Glu Trp Val
        35                  40                  45

Ala Val Ile Trp Tyr Asp Gly Arg Asn Lys Tyr Tyr Ala Asp Ser Val
    50                  55                  60

Lys Gly Arg Phe Thr Ile Ser Arg Asp Asp Phe Lys Asn Thr Leu Asp
65                  70                  75                  80

Leu Gln Met Asn Ser Leu Arg Ala Glu Asp Thr Ala Val Tyr Tyr Cys
                85                  90                  95

Ala Arg Asp Gln His Ala Gly Ile Thr Met Ile Glu Asp Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Asp Glu Leu Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys
    450

<210>  52
<211>  120
<212>  PRT
<213>  Homo sapiens

<400>  52

Gln Val Gln Leu Val Glu Ser Gly Gly Gly Val Ala Gln Pro Gly Arg
1               5                   10                  15

Ser Leu Arg Leu Ser Cys Ala Ala Ser Gly Phe Thr Leu Ser Asn Tyr
            20                  25                  30

Gly Met His Trp Val Arg Gln Ala Pro Gly Lys Gly Leu Glu Trp Val
        35                  40                  45

Ala Val Ile Trp Tyr Asp Gly Arg Asn Lys Tyr Tyr Ala Asp Ser Val
    50                  55                  60

Lys Gly Arg Phe Thr Ile Ser Arg Asp Asp Phe Lys Asn Thr Leu Asp
65                  70                  75                  80

Leu Gln Met Asn Ser Leu Arg Ala Glu Asp Thr Ala Val Tyr Tyr Cys
                85                  90                  95

Ala Arg Asp Gln His Ala Gly Ile Thr Met Ile Glu Asp Trp Gly Gln
            100                 105                 110

Gly Thr Leu Val Thr Val Ser Ser
        115                 120

<210>  53
<211>  5
<212>  PRT

<213>  Homo sapiens

<400>  53

Asn Tyr Gly Met His
1               5


<210>  54
<211>  17
<212>  PRT
<213>  Homo sapiens

<400>  54

Val Ile Trp Tyr Asp Gly Arg Asn Lys Tyr Tyr Ala Asp Ser Val Lys
1               5                   10                  15


Gly


<210>  55
<211>  11
<212>  PRT
<213>  Homo sapiens

<400>  55

Asp Gln His Ala Gly Ile Thr Met Ile Glu Asp
1               5                   10


<210>  56
<211>  216
<212>  PRT
<213>  Homo sapiens

<400>  56

Gln Ser Ala Leu Thr Gln Pro Pro Ser Ala Ser Gly Ser Pro Gly Gln
1               5                   10                  15


Ser Val Thr Ile Ser Cys Thr Gly Thr Ser Ser Asp Val Gly Tyr Phe
            20                  25                  30


Lys Tyr Val Ser Trp Tyr Gln Gln His Pro Gly Lys Ala Pro Lys Leu
        35                  40                  45


Leu Ile Tyr Glu Val Ser Lys Arg Pro Ser Gly Val Pro Asp Arg Phe
    50                  55                  60


Ser Gly Ser Arg Ser Gly Asn Thr Ala Ser Leu Thr Val Ser Gly Leu
65                  70                  75                  80


Gln Ala Asp Asp Glu Ala Asp Tyr Tyr Cys Gly Ser Tyr Ala Gly Asn
                85                  90                  95

Asn Asn Ile Ile Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  57
<211>  110
<212>  PRT
<213>  Homo sapiens

<400>  57

Gln Ser Ala Leu Thr Gln Pro Pro Ser Ala Ser Gly Ser Pro Gly Gln
1               5                   10                  15

Ser Val Thr Ile Ser Cys Thr Gly Thr Ser Ser Asp Val Gly Tyr Phe
            20                  25                  30

Lys Tyr Val Ser Trp Tyr Gln Gln His Pro Gly Lys Ala Pro Lys Leu
        35                  40                  45

Leu Ile Tyr Glu Val Ser Lys Arg Pro Ser Gly Val Pro Asp Arg Phe
    50                  55                  60

Ser Gly Ser Arg Ser Gly Asn Thr Ala Ser Leu Thr Val Ser Gly Leu
65                  70                  75                  80

Gln Ala Asp Asp Glu Ala Asp Tyr Tyr Cys Gly Ser Tyr Ala Gly Asn
                85                  90                  95

Asn Asn Ile Ile Phe Gly Gly Gly Thr Lys Leu Thr Val Leu
            100                 105                 110

<210>  58
<211>  14
<212>  PRT
<213>  Homo sapiens

<400>  58

Thr Gly Thr Ser Ser Asp Val Gly Tyr Phe Lys Tyr Val Ser
1               5                   10

<210>  59
<211>  7
<212>  PRT
<213>  Homo sapiens

<400>  59

Glu Val Ser Lys Arg Pro Ser
1               5

<210>  60
<211>  10
<212>  PRT
<213>  Homo sapiens

<400>  60

Gly Ser Tyr Ala Gly Asn Asn Asn Ile Ile
1               5                   10

<210>  61
<211>  449
<212>  PRT
<213>  Homo sapiens

<400>  61

Gln Glu Gln Leu Val Glu Ser Gly Gly Asp Leu Val Lys Pro Gly Gly
1               5                   10                  15

Ser Leu Arg Ile Thr Cys Ala Ala Ser Gly Phe Thr Leu Ser Gly Tyr
            20                  25                  30

Ala Leu Val Trp Val Arg Gln Ala Pro Gly Arg Gly Leu Glu Trp Ile
        35                  40                  45

Ala Asp Ile Thr Ala Ser Ser Thr Tyr Ile Asn Tyr Ala Ala Ser Val
    50                  55                  60

Lys Gly Arg Phe Thr Ile Ser Arg Asp Asn Ala Gln Ser Ser Leu Phe

65                  70                  75                  80

Leu Gln Met Ser Ser Leu Arg Ala Asp Asp Thr Gly Val Tyr Phe Cys
                85                  90                  95

Ala Arg Leu Pro His Leu Pro Gly Phe Phe Asp Leu Trp Gly Arg Gly
            100                 105                 110

Thr Leu Val Ser Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Asp Glu Leu Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro Gly
        435                 440                 445

Lys

<210>  62
<211>  119
<212>  PRT
<213>  Homo sapiens

<400>  62

Gln Glu Gln Leu Val Glu Ser Gly Gly Asp Leu Val Lys Pro Gly Gly
1               5                   10                  15

Ser Leu Arg Ile Thr Cys Ala Ala Ser Gly Phe Thr Leu Ser Gly Tyr
            20                  25                  30

Ala Leu Val Trp Val Arg Gln Ala Pro Gly Arg Gly Leu Glu Trp Ile
        35                  40                  45

Ala Asp Ile Thr Ala Ser Ser Thr Tyr Ile Asn Tyr Ala Ala Ser Val
    50                  55                  60

Lys Gly Arg Phe Thr Ile Ser Arg Asp Asn Ala Gln Ser Ser Leu Phe
65                  70                  75                  80

Leu Gln Met Ser Ser Leu Arg Ala Asp Asp Thr Gly Val Tyr Phe Cys
                85                  90                  95

Ala Arg Leu Pro His Leu Pro Gly Phe Phe Asp Leu Trp Gly Arg Gly
            100                 105                 110

Thr Leu Val Ser Val Ser Ser
        115

<210>  63
<211>  5
<212>  PRT
<213>  Homo sapiens

<400>  63

Gly Tyr Ala Leu Val
1               5

<210>  64
<211>  17
<212>  PRT
<213>  Homo sapiens

<400>  64

Asp Ile Thr Ala Ser Ser Thr Tyr Ile Asn Tyr Ala Ala Ser Val Lys
1               5                   10                  15

Gly

<210>  65
<211>  10
<212>  PRT
<213>  Homo sapiens

<400>  65

Leu Pro His Leu Pro Gly Phe Phe Asp Leu
1               5                   10

<210>  66
<211>  217
<212>  PRT
<213>  Homo sapiens

<400>  66

Gln Ser Val Leu Thr Gln Pro Pro Ser Val Ser Gly Ala Pro Gly Gln
1               5                   10                  15

Arg Val Thr Ile Ser Cys Thr Gly Asn Ser Ser Asn Ile Gly Ala Gly
            20                  25                  30

Tyr Asp Val Asn Trp Tyr Gln His Leu Pro Gly Thr Ala Pro Lys Leu
        35                  40                  45

Leu Ile Ser Val Asn Thr Asn Arg Pro Ser Gly Val Pro Asp Arg Phe
    50                  55                  60

Ser Gly Ser Met Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Arg Arg
                85                  90                  95

Leu Ser Gly Tyr Val Phe Gly Thr Gly Thr Lys Val Thr Val Leu Gly
            100                 105                 110

Gln Pro Lys Ala Asn Pro Thr Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Gly Ser Pro Val
145                 150                 155                 160

Lys Ala Gly Val Glu Thr Thr Lys Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  67
<211>  111
<212>  PRT
<213>  Homo sapiens

<400>  67

Gln Ser Val Leu Thr Gln Pro Pro Ser Val Ser Gly Ala Pro Gly Gln
1               5                   10                  15

Arg Val Thr Ile Ser Cys Thr Gly Asn Ser Ser Asn Ile Gly Ala Gly
            20                  25                  30

Tyr Asp Val Asn Trp Tyr Gln His Leu Pro Gly Thr Ala Pro Lys Leu
        35                  40                  45

Leu Ile Ser Val Asn Thr Asn Arg Pro Ser Gly Val Pro Asp Arg Phe
    50                  55                  60

Ser Gly Ser Met Ser Gly Thr Ser Ala Ser Leu Ala Ile Thr Gly Leu
65                  70                  75                  80

Gln Ala Glu Asp Glu Ala Asp Tyr Tyr Cys Gln Ser Tyr Asp Arg Arg
                85                  90                  95

Leu Ser Gly Tyr Val Phe Gly Thr Gly Thr Lys Val Thr Val Leu
            100                 105                 110

<210>  68
<211>  14
<212>  PRT
<213>  Homo sapiens

<400>  68

Thr Gly Asn Ser Ser Asn Ile Gly Ala Gly Tyr Asp Val Asn
1               5                   10

<210>  69
<211>  7
<212>  PRT
<213>  Homo sapiens

<400>  69

Val Asn Thr Asn Arg Pro Ser
1               5

<210>  70
<211>  11
<212>  PRT
<213>  Homo sapiens

<400>  70

Gln Ser Tyr Asp Arg Arg Leu Ser Gly Tyr Val
1               5                   10

<210>  71
<211>  455
<212>  PRT
<213>  Homo sapiens

<400>  71

Gln Val Gln Leu Val Gln Ser Gly Ala Glu Met Lys Lys Pro Gly Ala
1               5                   10                  15

Ser Val Lys Val Ser Cys Lys Ala Ser Gly Tyr Thr Phe Thr Asp Tyr
            20                  25                  30

Phe Ile His Trp Val Arg Gln Ala Pro Gly Gln Gly Leu Glu Trp Met
        35                  40                  45

Gly Trp Val Asn Pro Asn Ser Gly Ala Thr Asn Tyr Ala Gln Lys Phe
    50                  55                  60

Gln Gly Arg Val Thr Met Thr Gly Asp Thr Ser Leu Ser Ala Ala Tyr
65                  70                  75                  80

Val Asp Leu Ser Arg Leu Ser Ser Asp Asp Ser Ala Val Tyr Tyr Cys
                85                  90                  95

Ala Arg Asp Arg Gly Leu Gly Ile Ser Ser Thr Arg Asn Asp Ala Phe
            100                 105                 110

Asp Ile Trp Gly Gln Gly Thr Thr Val Thr Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn His Lys Pro Ser Asn Thr Lys Val Asp Lys Lys Val Glu
    210                 215                 220

Pro Lys Ser Cys Asp Lys Thr His Thr Cys Pro Pro Cys Pro Ala Pro
225                 230                 235                 240

Glu Leu Leu Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val

260                 265                 270

Asp Val Ser His Glu Asp Pro Glu Val Lys Phe Asn Trp Tyr Val Asp
        275                 280                 285

Gly Val Glu Val His Asn Ala Lys Thr Lys Pro Arg Glu Glu Gln Tyr
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Pro Ala Pro Ile Glu Lys Thr Ile Ser Lys Ala Lys Gly Gln Pro Arg
            340                 345                 350

Glu Pro Gln Val Tyr Thr Leu Pro Pro Ser Arg Asp Glu Leu Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
        435                 440                 445

Leu Ser Leu Ser Pro Gly Lys
    450                 455

<210>  72
<211>  125
<212>  PRT
<213>  Homo sapiens

<400>  72

Gln Val Gln Leu Val Gln Ser Gly Ala Glu Met Lys Lys Pro Gly Ala
1               5                   10                  15

Ser Val Lys Val Ser Cys Lys Ala Ser Gly Tyr Thr Phe Thr Asp Tyr

20                  25                  30

Phe Ile His Trp Val Arg Gln Ala Pro Gly Gln Gly Leu Glu Trp Met
        35                  40                  45

Gly Trp Val Asn Pro Asn Ser Gly Ala Thr Asn Tyr Ala Gln Lys Phe
    50                  55                  60

Gln Gly Arg Val Thr Met Thr Gly Asp Thr Ser Leu Ser Ala Ala Tyr
65                  70                  75                  80

Val Asp Leu Ser Arg Leu Ser Ser Asp Asp Ser Ala Val Tyr Tyr Cys
                85                  90                  95

Ala Arg Asp Arg Gly Leu Gly Ile Ser Ser Thr Arg Asn Asp Ala Phe
            100                 105                 110

Asp Ile Trp Gly Gln Gly Thr Thr Val Thr Val Ser Ser
        115                 120                 125

<210>  73
<211>  5
<212>  PRT
<213>  Homo sapiens

<400>  73

Asp Tyr Phe Ile His
1               5

<210>  74
<211>  17
<212>  PRT
<213>  Homo sapiens

<400>  74

Trp Val Asn Pro Asn Ser Gly Ala Thr Asn Tyr Ala Gln Lys Phe Gln
1               5                   10                  15

Gly

<210>  75
<211>  16
<212>  PRT
<213>  Homo sapiens

<400>  75

Asp Arg Gly Leu Gly Ile Ser Ser Thr Arg Asn Asp Ala Phe Asp Ile
1               5                   10                  15

<210>  76
<211>  214
<212>  PRT
<213>  Homo sapiens

<400>  76

Asp Ile Gln Leu Thr Gln Ser Pro Ser Ser Leu Ser Ala Ser Val Gly
1               5                   10                  15

Asp Arg Val Thr Ile Thr Cys Gln Pro Ser Gln Gly Ile Arg Asn Phe
            20                  25                  30

Leu Asn Trp Tyr Gln Gln Lys Pro Gly Lys Ala Pro Asn Leu Leu Ile
        35                  40                  45

Tyr Asp Ala Ser Asn Leu Glu Thr Gly Val Ala Ser Arg Phe Ser Gly
    50                  55                  60

Ser Gly Ser Gly Thr His Phe Thr Leu Thr Ile Ser Ser Leu Gln Pro
65                  70                  75                  80

Glu Asp Ile Ala Thr Tyr Phe Cys Gln Gln Tyr His Ser Leu Pro Leu
                85                  90                  95

Thr Phe Gly Gly Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  77
<211>  107
<212>  PRT
<213>  Homo sapiens

<400>  77

Asp Ile Gln Leu Thr Gln Ser Pro Ser Ser Leu Ser Ala Ser Val Gly
1               5                   10                  15

Asp Arg Val Thr Ile Thr Cys Gln Pro Ser Gln Gly Ile Arg Asn Phe
            20                  25                  30

Leu Asn Trp Tyr Gln Gln Lys Pro Gly Lys Ala Pro Asn Leu Leu Ile
        35                  40                  45

Tyr Asp Ala Ser Asn Leu Glu Thr Gly Val Ala Ser Arg Phe Ser Gly
    50                  55                  60

Ser Gly Ser Gly Thr His Phe Thr Leu Thr Ile Ser Ser Leu Gln Pro
65                  70                  75                  80

Glu Asp Ile Ala Thr Tyr Phe Cys Gln Gln Tyr His Ser Leu Pro Leu
                85                  90                  95

Thr Phe Gly Gly Gly Thr Lys Val Glu Ile Lys
            100                 105


<210>  78
<211>  11
<212>  PRT
<213>  Homo sapiens

<400>  78

Gln Pro Ser Gln Gly Ile Arg Asn Phe Leu Asn
1               5                   10


<210>  79
<211>  7
<212>  PRT
<213>  Homo sapiens

<400>  79

Asp Ala Ser Asn Leu Glu Thr
1               5


<210>  80
<211>  9
<212>  PRT
<213>  Homo sapiens

<400>  80

Gln Gln Tyr His Ser Leu Pro Leu Thr
1               5


<210>  81
<211>  451
<212>  PRT
<213>  Homo sapiens

<400>  81

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ala Asp Tyr
            20                  25                  30

Tyr Ile His Trp Leu Arg Gln Ala Pro Gly Gln Gly Leu Glu Trp Leu
        35                  40                  45

Gly Arg Ile Asn Pro Ser Asn Ser Arg Thr Thr Tyr Pro Gln Lys Phe
    50                  55                  60

Gln Gly Arg Val Thr Val Thr Arg Asp Thr Ser Thr Ser Thr Val Tyr
65                  70                  75                  80

Met Glu Leu Ser Ser Leu Arg Ser Ala Asp Thr Ala Val Tyr Tyr Cys
                85                  90                  95

Ala Arg Asp Gly Leu Arg Met Gly Tyr Tyr Tyr Met Asp Val Trp Gly
            100                 105                 110

Lys Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Asp Glu Leu Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Gln Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro Gly Lys

    450

<210>  82
<211>  121
<212>  PRT
<213>  Homo sapiens

<400>  82

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ala Asp Tyr
            20                  25                  30

Tyr Ile His Trp Leu Arg Gln Ala Pro Gly Gln Gly Leu Glu Trp Leu
        35                  40                  45

Gly Arg Ile Asn Pro Ser Asn Ser Arg Thr Thr Tyr Pro Gln Lys Phe
    50                  55                  60

Gln Gly Arg Val Thr Val Thr Arg Asp Thr Ser Thr Ser Thr Val Tyr
65                  70                  75                  80

Met Glu Leu Ser Ser Leu Arg Ser Ala Asp Thr Ala Val Tyr Tyr Cys
                85                  90                  95

Ala Arg Asp Gly Leu Arg Met Gly Tyr Tyr Tyr Met Asp Val Trp Gly
            100                 105                 110

Lys Gly Thr Thr Val Thr Val Ser Ser
        115                 120


<210>  83
<211>  5
<212>  PRT
<213>  Homo sapiens

<400>  83

Asp Tyr Tyr Ile His
1               5


<210>  84
<211>  17
<212>  PRT
<213>  Homo sapiens

<400>  84

Arg Ile Asn Pro Ser Asn Ser Arg Thr Thr Tyr Pro Gln Lys Phe Gln
1               5                   10                  15

Gly

<210>  85
<211>  12
<212>  PRT
<213>  Homo sapiens

<400>  85

Asp Gly Leu Arg Met Gly Tyr Tyr Tyr Met Asp Val
1               5                   10

<210>  86
<211>  217
<212>  PRT
<213>  Homo sapiens

<400>  86

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val His Trp Tyr Gln Gln Ile Pro Gly Thr Ala Pro Lys Leu
        35                  40                  45

Leu Ile Phe Gly Asn Tyr Asn Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Glu Asp Glu Ala His Tyr Tyr Cys Gln Ser Tyr Asp Thr Thr
                85                  90                  95

Leu Asp Gly Ala Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Lys Ser Tyr Ser Cys Gln Val Thr His Glu Gly Ser Thr Val Glu
        195                 200                 205

Lys Thr Val Ala Pro Thr Glu Cys Ser
    210                 215

<210>  87
<211>  111
<212>  PRT
<213>  Homo sapiens

<400>  87

Gln Ser Val Leu Thr Gln Pro Pro Ser Val Ser Gly Ala Pro Gly Gln
1               5                   10                  15

Arg Val Thr Ile Ser Cys Thr Gly Ser Ser Ser Asn Ile Gly Ala Gly
            20                  25                  30

Tyr Asp Val His Trp Tyr Gln Gln Ile Pro Gly Thr Ala Pro Lys Leu
        35                  40                  45

Leu Ile Phe Gly Asn Tyr Asn Arg Pro Ser Gly Val Pro Asp Arg Phe
    50                  55                  60

Ser Ala Ser Lys Ser Gly Thr Ser Ala Ser Leu Ala Ile Thr Gly Leu
65                  70                  75                  80

Gln Ala Glu Asp Glu Ala His Tyr Tyr Cys Gln Ser Tyr Asp Thr Thr
                85                  90                  95

Leu Asp Gly Ala Val Phe Gly Gly Gly Thr Lys Leu Thr Val Leu
            100                 105                 110

<210>  88
<211>  14
<212>  PRT
<213>  Homo sapiens

<400>  88

Thr Gly Ser Ser Ser Asn Ile Gly Ala Gly Tyr Asp Val His
1               5                   10

<210>  89

<211>  7
<212>  PRT
<213>  Homo sapiens

<400>  89

Gly Asn Tyr Asn Arg Pro Ser
1               5


<210>  90
<211>  11
<212>  PRT
<213>  Homo sapiens

<400>  90

Gln Ser Tyr Asp Thr Thr Leu Asp Gly Ala Val
1               5                   10


<210>  91
<211>  454
<212>  PRT
<213>  Homo sapiens

<400>  91

Gln Val Gln Leu Val Gln Ser Gly Ala Glu Val Lys Lys Pro Gly Ser
1               5                   10                  15

Ser Val Arg Val Ser Cys Lys Ala Ser Gly Gly Thr Phe Ser Thr Tyr
            20                  25                  30

Gly Ile Asn Trp Val Arg Gln Ala Arg Gly Gln Gly Leu Glu Trp Met
        35                  40                  45

Gly Thr Val Ile Pro Thr Phe Glu Lys Thr Tyr Tyr Ala Gln Gly Phe
    50                  55                  60

Gln Gly Arg Val Thr Ile Ala Ala Asp Gly Ser Lys Asn Thr Val Tyr
65                  70                  75                  80

Met Glu Leu Arg Arg Leu Thr Ser Glu Asp Ser Ala Ile Tyr Phe Cys
                85                  90                  95

Thr Arg Phe Phe Thr Pro Tyr Cys Ser Gly Gly Ser Cys Tyr Leu Asp
            100                 105                 110

Leu Trp Gly Pro Gly Thr Leu Val Thr Val Ser Ser Ala Ser Thr Lys
        115                 120                 125

Gly Pro Ser Val Phe Pro Leu Ala Pro Ser Ser Lys Ser Thr Ser Gly
    130                 135                 140

Gly Thr Ala Ala Leu Gly Cys Leu Val Lys Asp Tyr Phe Pro Glu Pro
145                 150                 155                 160

Val Thr Val Ser Trp Asn Ser Gly Ala Leu Thr Ser Gly Val His Thr
                165                 170                 175

Phe Pro Ala Val Leu Gln Ser Ser Gly Leu Tyr Ser Leu Ser Ser Val
            180                 185                 190

Val Thr Val Pro Ser Ser Ser Leu Gly Thr Gln Thr Tyr Ile Cys Asn
        195                 200                 205

Val Asn His Lys Pro Ser Asn Thr Lys Val Asp Lys Lys Val Glu Pro
    210                 215                 220

Lys Ser Cys Asp Lys Thr His Thr Cys Pro Pro Cys Pro Ala Pro Glu
225                 230                 235                 240

Leu Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser His Glu Asp Pro Glu Val Lys Phe Asn Trp Tyr Val Asp Gly
        275                 280                 285

Val Glu Val His Asn Ala Lys Thr Lys Pro Arg Glu Glu Gln Tyr Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Ala Leu Pro
                325                 330                 335

Ala Pro Ile Glu Lys Thr Ile Ser Lys Ala Lys Gly Gln Pro Arg Glu
            340                 345                 350

Pro Gln Val Tyr Thr Leu Pro Pro Ser Arg Asp Glu Leu Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                 390                 395                 400

Thr Pro Pro Val Leu Asp Ser Asp Gly Ser Phe Phe Leu Tyr Ser Lys
                405                 410                 415

Leu Thr Val Asp Lys Ser Arg Trp Gln Gln Gly Asn Val Phe Ser Cys
            420                 425                 430

Ser Val Met His Glu Ala Leu His Asn His Tyr Thr Gln Lys Ser Leu
        435                 440                 445

Ser Leu Ser Pro Gly Lys
    450

<210> 92
<211> 124
<212> PRT
<213> Homo sapiens

<400> 92

Gln Val Gln Leu Val Gln Ser Gly Ala Glu Val Lys Lys Pro Gly Ser
1               5                   10                  15

Ser Val Arg Val Ser Cys Lys Ala Ser Gly Gly Thr Phe Ser Thr Tyr
            20                  25                  30

Gly Ile Asn Trp Val Arg Gln Ala Arg Gly Gln Gly Leu Glu Trp Met
        35                  40                  45

Gly Thr Val Ile Pro Thr Phe Glu Lys Thr Tyr Tyr Ala Gln Gly Phe
    50                  55                  60

Gln Gly Arg Val Thr Ile Ala Ala Asp Gly Ser Lys Asn Thr Val Tyr
65                  70                  75                  80

Met Glu Leu Arg Arg Leu Thr Ser Glu Asp Ser Ala Ile Tyr Phe Cys
                85                  90                  95

Thr Arg Phe Phe Thr Pro Tyr Cys Ser Gly Gly Ser Cys Tyr Leu Asp
            100                 105                 110

Leu Trp Gly Pro Gly Thr Leu Val Thr Val Ser Ser
        115                 120

<210> 93
<211> 5
<212> PRT
<213> Homo sapiens

<400> 93

Thr Tyr Gly Ile Asn
1               5

<210>  94
<211>  17
<212>  PRT
<213>  Homo sapiens

<400>  94

Thr Val Ile Pro Thr Phe Glu Lys Thr Tyr Tyr Ala Gln Gly Phe Gln
1               5                   10                  15

Gly

<210>  95
<211>  15
<212>  PRT
<213>  Homo sapiens

<400>  95

Phe Phe Thr Pro Tyr Cys Ser Gly Gly Ser Cys Tyr Leu Asp Leu
1               5                   10                  15

<210>  96
<211>  214
<212>  PRT
<213>  Homo sapiens

<400>  96

Gln Ser Val Leu Thr Gln Pro Pro Ser Val Ser Gly Ala Pro Gly Gln
1               5                   10                  15

Ser Val Thr Ile Ser Cys Ala Gly Ser Ser Ser Asn Ile Gly Ala Gly
            20                  25                  30

Tyr Asp Val His Trp Tyr Gln His Leu Pro Gly Thr Ala Pro Arg Leu
        35                  40                  45

Leu Ile Tyr Ser Asn Thr Asn Arg Pro Ser Gly Val Pro Asp Arg Phe
    50                  55                  60

Ser Gly Ser Lys Ser Gly Ala Ser Ala Ser Leu Ala Ile Thr Gly Leu
65                  70                  75                  80

Gln Ala Glu Asp Glu Ala Asp Tyr Tyr Cys Gln Ser Tyr Asp Ser Gly
                85                  90                  95

Val Val Phe Gly Gly Gly Thr Lys Leu Thr Val Leu Arg Gln Pro Lys

            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Thr Pro Ser Lys Gln Ser Asn Asn Lys Tyr Ala Ala
                165                 170                 175

Ser Ser Tyr Leu Ser Leu Thr Pro Glu Gln Trp Lys Ser His Arg Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210> 97
<211> 108
<212> PRT
<213> Homo sapiens

<400> 97

Gln Ser Val Leu Thr Gln Pro Pro Ser Val Ser Gly Ala Pro Gly Gln
1               5                   10                  15

Ser Val Thr Ile Ser Cys Ala Gly Ser Ser Ser Asn Ile Gly Ala Gly
            20                  25                  30

Tyr Asp Val His Trp Tyr Gln His Leu Pro Gly Thr Ala Pro Arg Leu
        35                  40                  45

Leu Ile Tyr Ser Asn Thr Asn Arg Pro Ser Gly Val Pro Asp Arg Phe
    50                  55                  60

Ser Gly Ser Lys Ser Gly Ala Ser Ala Ser Leu Ala Ile Thr Gly Leu
65                  70                  75                  80

Gln Ala Glu Asp Glu Ala Asp Tyr Tyr Cys Gln Ser Tyr Asp Ser Gly
                85                  90                  95

Val Val Phe Gly Gly Gly Thr Lys Leu Thr Val Leu

            100                 105

<210> 98
<211> 14
<212> PRT
<213> Homo sapiens

<400> 98

Ala Gly Ser Ser Ser Asn Ile Gly Ala Gly Tyr Asp Val His
1               5                   10

<210> 99
<211> 7
<212> PRT
<213> Homo sapiens

<400> 99

Ser Asn Thr Asn Arg Pro Ser
1               5

<210> 100
<211> 8
<212> PRT
<213> Homo sapiens

<400> 100

Gln Ser Tyr Asp Ser Gly Val Val
1               5

<210> 101
<211> 452
<212> PRT
<213> Homo sapiens

<400> 101

Gln Val Gln Leu Val Gln Ser Gly Ala Glu Val Gln Lys Pro Gly Ser
1               5                   10                  15

Ser Val Lys Val Ser Cys Lys Thr Ser Gly Asp Thr Phe Asn Asn Tyr
            20                  25                  30

Ala Leu Ser Trp Val Arg Gln Ala Pro Gly Gln Gly Pro Glu Trp Met
        35                  40                  45

Gly Arg Ile Ile Pro Leu Val Glu Met Ala Asn Tyr Ala Gln Lys Phe
    50                  55                  60

Gln Gly Arg Val Thr Ile Thr Ala Asp Lys Ser Thr Thr Thr Ile Tyr
65                  70                  75                  80

Met Glu Leu Ser Ser Leu Arg Ser Glu Asp Thr Ala Met Tyr Tyr Cys
                85                  90                  95

Ala Arg Asp Gly Tyr Gln Gly Gly Phe Pro Tyr Phe Phe Asp Ser Trp
            100                 105                 110

Gly Gln Gly Thr Leu Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Leu
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Asn Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Asp Glu Leu Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly Lys
    450

<210>  102
<211>  122
<212>  PRT
<213>  Homo sapiens

<400>  102

Gln Val Gln Leu Val Gln Ser Gly Ala Glu Val Gln Lys Pro Gly Ser
1               5                   10                  15

Ser Val Lys Val Ser Cys Lys Thr Ser Gly Asp Thr Phe Asn Asn Tyr
            20                  25                  30

Ala Leu Ser Trp Val Arg Gln Ala Pro Gly Gln Gly Pro Glu Trp Met
        35                  40                  45

Gly Arg Ile Ile Pro Leu Val Glu Met Ala Asn Tyr Ala Gln Lys Phe
    50                  55                  60

Gln Gly Arg Val Thr Ile Thr Ala Asp Lys Ser Thr Thr Thr Ile Tyr
65                  70                  75                  80

Met Glu Leu Ser Ser Leu Arg Ser Glu Asp Thr Ala Met Tyr Tyr Cys
                85                  90                  95

Ala Arg Asp Gly Tyr Gln Gly Gly Phe Pro Tyr Phe Phe Asp Ser Trp
            100                 105                 110

Gly Gln Gly Thr Leu Val Thr Val Ser Ser
        115                 120

<210>  103
<211>  5
<212>  PRT
<213>  Homo sapiens

<400>  103

Asn Tyr Ala Leu Ser
1               5

<210>  104
<211>  17
<212>  PRT
<213>  Homo sapiens

<400>  104

Arg Ile Ile Pro Leu Val Glu Met Ala Asn Tyr Ala Gln Lys Phe Gln
1               5                   10                  15

Gly

<210>  105
<211>  13
<212>  PRT
<213>  Homo sapiens

<400>  105

Asp Gly Tyr Gln Gly Gly Phe Pro Tyr Phe Phe Asp Ser
1               5                   10

<210>  106
<211>  214
<212>  PRT
<213>  Homo sapiens

<400>  106

Glu Val Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Asn Tyr
            20                  25                  30

Leu Ala Trp Tyr Gln Gln Lys Pro Gly Gln Ala Pro Arg Leu Leu Ile
        35                  40                  45

Ser Ala Ala Ser Ser Arg Ala Thr Gly Ile Pro Asp Arg Phe Gly Gly
    50                  55                  60

Ser Gly Ser Gly Ala Asp Phe Thr Leu Thr Ile Ser Arg Leu Glu Pro
65                  70                  75                  80

Glu Asp Phe Ala Val Tyr Tyr Cys Gln Gln Tyr Gly Phe Ser Pro Gly
                85                  90                  95

Thr Phe Gly Gln Gly Thr Lys Val Glu Ile Arg Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Leu Tyr
            180                 185                 190

Ala Cys Glu Val Thr His Gln Gly Leu Ser Ser Pro Val Thr Lys Ser
        195                 200                 205

Phe Asn Arg Gly Glu Cys
    210


<210>  107
<211>  107
<212>  PRT
<213>  Homo sapiens

<400>  107

Glu Val Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Asn Tyr
            20                  25                  30

Leu Ala Trp Tyr Gln Gln Lys Pro Gly Gln Ala Pro Arg Leu Leu Ile
        35                  40                  45

Ser Ala Ala Ser Ser Arg Ala Thr Gly Ile Pro Asp Arg Phe Gly Gly
    50                  55                  60

Ser Gly Ser Gly Ala Asp Phe Thr Leu Thr Ile Ser Arg Leu Glu Pro
65                  70                  75                  80

Glu Asp Phe Ala Val Tyr Tyr Cys Gln Gln Tyr Gly Phe Ser Pro Gly
                85                  90                  95

Thr Phe Gly Gln Gly Thr Lys Val Glu Ile Arg
            100                 105

<210>  108
<211>  11
<212>  PRT
<213>  Homo sapiens

<400>  108

Arg Ala Ser Gln Ser Val Ser Asn Tyr Leu Ala
1               5                   10

<210>  109
<211>  7
<212>  PRT
<213>  Homo sapiens

<400>  109

Ala Ala Ser Ser Arg Ala Thr
1               5

<210>  110
<211>  9
<212>  PRT
<213>  Homo sapiens

<400>  110

Gln Gln Tyr Gly Phe Ser Pro Gly Thr
1               5

<210>  111
<211>  448
<212>  PRT
<213>  Homo sapiens

<400>  111

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Val Tyr

20                  25                  30

Gly Ile Ser Trp Val Arg Gln Ala Pro Gly His Gly Leu Glu Trp Met
        35                  40                  45

Gly Trp Ile Ser Ala Tyr Asn Gly Asp Arg Gly Tyr Ala Gln Lys Val
    50                  55                  60

Gln Gly Arg Ile Thr Met Thr Thr Asp Thr Ser Ala Ser Thr Ala Tyr
65                  70                  75                  80

Met Glu Leu Arg Ser Leu Thr Ser Asp Asp Thr Ala Val Tyr Tyr Cys
                85                  90                  95

Ala Thr Thr Ser Ser Gly Val Pro Gly Ile Tyr Trp Gly Gln Gly Ser
            100                 105                 110

Leu Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  112
<211>  118
<212>  PRT
<213>  Homo sapiens

<400>  112

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Val Tyr
            20                  25                  30

Gly Ile Ser Trp Val Arg Gln Ala Pro Gly His Gly Leu Glu Trp Met
        35                  40                  45

Gly Trp Ile Ser Ala Tyr Asn Gly Asp Arg Gly Tyr Ala Gln Lys Val
    50                  55                  60

Gln Gly Arg Ile Thr Met Thr Thr Asp Thr Ser Ala Ser Thr Ala Tyr
65                  70                  75                  80

Met Glu Leu Arg Ser Leu Thr Ser Asp Asp Thr Ala Val Tyr Tyr Cys
                85                  90                  95

Ala Thr Thr Ser Ser Gly Val Pro Gly Ile Tyr Trp Gly Gln Gly Ser
            100                 105                 110

Leu Val Thr Val Ser Ser
        115

<210>  113
<211>  5
<212>  PRT
<213>  Homo sapiens

<400>  113

Val Tyr Gly Ile Ser
1               5

<210>  114
<211>  17
<212>  PRT
<213>  Homo sapiens

<400>  114

Trp Ile Ser Ala Tyr Asn Gly Asp Arg Gly Tyr Ala Gln Lys Val Gln
1               5                   10                  15

Gly

<210>  115
<211>  9
<212>  PRT
<213>  Homo sapiens

<400>  115

Thr Ser Ser Gly Val Pro Gly Ile Tyr
1               5

<210>  116
<211>  214
<212>  PRT
<213>  Homo sapiens

<400>  116

Ser Tyr Ala Leu Val Gln Pro Pro Ser Val Ser Val Ser Pro Gly Gln
1               5                   10                  15

Thr Ala Arg Ile Thr Cys Phe Gly Pro Ala Leu Pro Lys Lys Tyr Ala
            20                  25                  30

Tyr Trp Tyr Gln His Lys Ser Gly Gln Ala Pro Met Leu Val Ile Tyr
        35                  40                  45

Glu Asp Thr Lys Arg Pro Ser Gly Ile Pro Gln Arg Phe Ser Gly Ser
    50                  55                  60

Thr Ser Gly Ala Thr Ala Thr Leu Thr Ile Arg Gly Ala Gln Val Glu
65                  70                  75                  80

Asp Glu Ala Asp Tyr Tyr Cys Tyr Ser Thr Asp Ser Ser Gly His Glu
                85                  90                  95

Arg Val Phe Gly Gly Gly Thr Lys Leu Thr Val Leu Gly Gln Pro Lys
            100                 105                 110

Ala Ala Pro Ser Val Thr Leu Phe Pro Pro Ser Ser Glu Glu Leu Gln
        115                 120                 125

Ala Asn Lys Ala Thr Leu Val Cys Leu Ile Ser Asp Phe Tyr Pro Gly
    130                 135                 140

Ala Val Thr Val Ala Trp Lys Ala Asp Ser Ser Pro Val Lys Ala Gly
145                 150                 155                 160

Val Glu Thr Thr Ala Pro Ser Lys Gln Ser Asn Asn Lys Tyr Ala Ala
                165                 170                 175

Ser Ser Tyr Leu Ser Leu Thr Pro Glu Gln Trp Lys Ser His Lys Ser
            180                 185                 190

Tyr Ser Cys Gln Val Thr His Glu Gly Ser Thr Val Glu Lys Thr Val
        195                 200                 205

Ala Pro Thr Glu Cys Ser
    210

<210>  117
<211>  108
<212>  PRT
<213>  Homo sapiens

<400>  117

Ser Tyr Ala Leu Val Gln Pro Pro Ser Val Ser Val Ser Pro Gly Gln
1               5                   10                  15

Thr Ala Arg Ile Thr Cys Phe Gly Pro Ala Leu Pro Lys Lys Tyr Ala
            20                  25                  30

Tyr Trp Tyr Gln His Lys Ser Gly Gln Ala Pro Met Leu Val Ile Tyr
        35                  40                  45

Glu Asp Thr Lys Arg Pro Ser Gly Ile Pro Gln Arg Phe Ser Gly Ser
    50                  55                  60

Thr Ser Gly Ala Thr Ala Thr Leu Thr Ile Arg Gly Ala Gln Val Glu
65                  70                  75                  80

Asp Glu Ala Asp Tyr Tyr Cys Tyr Ser Thr Asp Ser Ser Gly His Glu
                85                  90                  95

Arg Val Phe Gly Gly Gly Thr Lys Leu Thr Val Leu
            100                 105


<210>  118
<211>  11
<212>  PRT
<213>  Homo sapiens

<400>  118

Phe Gly Pro Ala Leu Pro Lys Lys Tyr Ala Tyr
1               5                   10


<210>  119
<211>  7
<212>  PRT
<213>  Homo sapiens

<400>  119

Glu Asp Thr Lys Arg Pro Ser
1               5


<210>  120
<211>  11
<212>  PRT
<213>  Homo sapiens

<400>  120

Tyr Ser Thr Asp Ser Ser Gly His Glu Arg Val
1               5                   10


<210>  121

<211>  455
<212>  PRT
<213>  Homo sapiens

<400>  121

Arg Val Gln Leu Val Gln Ser Gly Ala Glu Val Lys Lys Pro Gly Ser
1               5                   10                  15

Ser Val Arg Val Ser Cys Lys Ala Ser Gly Gly Thr Phe Ser Asn Tyr
            20                  25                  30

Ser Ile Ser Trp Val Arg Gln Val Pro Gly Gln Gly Leu Glu Trp Met
        35                  40                  45

Gly Arg Ile Ile Pro Ile Leu Pro Leu Ser Ser Tyr Pro Gln Gln Phe
    50                  55                  60

Gln Gly Arg Val Ala Ile Thr Ala Asp Lys Ser Ser Thr Thr Ala Tyr
65                  70                  75                  80

Met Glu Leu Ser Ser Leu Arg Ile Glu Asp Thr Ala Ile Tyr Tyr Cys
                85                  90                  95

Ala Arg Asp Ala Leu Arg Pro Glu Gly Gly Tyr Tyr Tyr Tyr Tyr Phe
            100                 105                 110

Asp Val Trp Gly Thr Gly Thr Thr Val Thr Val Ser Ser Ala Ser Thr
        115                 120                 125

Lys Gly Pro Ser Val Phe Pro Leu Ala Pro Ser Ser Lys Ser Thr Ser
    130                 135                 140

Gly Gly Thr Ala Ala Leu Gly Cys Leu Val Lys Asp Tyr Phe Pro Glu
145                 150                 155                 160

Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn His Lys Pro Ser Asn Thr Lys Val Asp Lys Lys Val Glu
    210                 215                 220

Pro Lys Ser Cys Asp Lys Thr His Thr Cys Pro Pro Cys Pro Ala Pro

225                 230                 235                 240

Glu Leu Leu Gly Gly Pro Ser Val Phe Leu Phe Pro Pro Lys Pro Lys
                245                 250                 255

Asp Thr Leu Met Ile Ser Arg Thr Pro Glu Val Thr Cys Val Val Val
            260                 265                 270

Asp Val Ser His Glu Asp Pro Glu Val Lys Phe Asn Trp Tyr Val Asp
        275                 280                 285

Gly Val Glu Val His Asn Ala Lys Thr Lys Pro Arg Glu Glu Gln Tyr
    290                 295                 300

Asn Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Pro Ala Pro Ile Glu Lys Thr Ile Ser Lys Ala Lys Gly Gln Pro Arg
            340                 345                 350

Glu Pro Gln Val Tyr Thr Leu Pro Pro Ser Arg Asp Glu Leu Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Val Leu Asp Ser Asp Gly Ser Phe Phe Leu Tyr Ser
                405                 410                 415

Lys Leu Thr Val Asp Lys Ser Arg Trp Gln Gln Gly Asn Val Phe Ser
            420                 425                 430

Cys Ser Val Met His Glu Ala Leu His Asn His Tyr Thr Gln Lys Ser
        435                 440                 445

Leu Ser Leu Ser Pro Gly Lys
    450                 455

<210>  122
<211>  125
<212>  PRT
<213>  Homo sapiens

<400>  122

Arg Val Gln Leu Val Gln Ser Gly Ala Glu Val Lys Lys Pro Gly Ser
1               5                   10                  15

Ser Val Arg Val Ser Cys Lys Ala Ser Gly Gly Thr Phe Ser Asn Tyr
            20                  25                  30

Ser Ile Ser Trp Val Arg Gln Val Pro Gly Gln Gly Leu Glu Trp Met
        35                  40                  45

Gly Arg Ile Ile Pro Ile Leu Pro Leu Ser Ser Tyr Pro Gln Gln Phe
    50                  55                  60

Gln Gly Arg Val Ala Ile Thr Ala Asp Lys Ser Ser Thr Thr Ala Tyr
65                  70                  75                  80

Met Glu Leu Ser Ser Leu Arg Ile Glu Asp Thr Ala Ile Tyr Tyr Cys
                85                  90                  95

Ala Arg Asp Ala Leu Arg Pro Glu Gly Gly Tyr Tyr Tyr Tyr Tyr Phe
            100                 105                 110

Asp Val Trp Gly Thr Gly Thr Thr Val Thr Val Ser Ser
        115                 120                 125


<210>  123
<211>  5
<212>  PRT
<213>  Homo sapiens

<400>  123

Asn Tyr Ser Ile Ser
1               5


<210>  124
<211>  17
<212>  PRT
<213>  Homo sapiens

<400>  124

Arg Ile Ile Pro Ile Leu Pro Leu Ser Ser Tyr Pro Gln Gln Phe Gln
1               5                   10                  15

Gly


<210>  125
<211>  16

<212>  PRT
<213>  Homo sapiens

<400>  125

Asp Ala Leu Arg Pro Glu Gly Gly Tyr Tyr Tyr Tyr Tyr Phe Asp Val
1               5                   10                  15


<210>  126
<211>  214
<212>  PRT
<213>  Homo sapiens

<400>  126

Glu Ile Glu Leu Thr Gln Ser Pro Gly Thr Leu Ser Leu Ser Pro Gly
1               5                   10                  15

Glu Arg Ala Thr Leu Ser Cys Arg Ala Asn Gln Ile Ile Gly Asp Thr
            20                  25                  30

Ser Leu Ala Trp Tyr Gln Gln Lys Pro Gly Gln Ala Pro Arg Leu Leu
        35                  40                  45

Ile Phe Gly Ala Ser Ser Arg Ala Thr Gly Ile Pro Asp Arg Phe Ser
    50                  55                  60

Gly Ser Gly Phe Gly Ser Asp Phe Thr Leu Thr Ile Thr Ser Leu Glu
65                  70                  75                  80

Pro Glu Asp Phe Ala Val Tyr Tyr Cys Gln Gln Tyr Gly Ser Ser Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  127
<211>  107
<212>  PRT
<213>  Homo sapiens

<400>  127

Glu Ile Glu Leu Thr Gln Ser Pro Gly Thr Leu Ser Leu Ser Pro Gly
1               5                   10                  15

Glu Arg Ala Thr Leu Ser Cys Arg Ala Asn Gln Ile Ile Gly Asp Thr
            20                  25                  30

Ser Leu Ala Trp Tyr Gln Gln Lys Pro Gly Gln Ala Pro Arg Leu Leu
        35                  40                  45

Ile Phe Gly Ala Ser Ser Arg Ala Thr Gly Ile Pro Asp Arg Phe Ser
    50                  55                  60

Gly Ser Gly Phe Gly Ser Asp Phe Thr Leu Thr Ile Thr Ser Leu Glu
65                  70                  75                  80

Pro Glu Asp Phe Ala Val Tyr Tyr Cys Gln Gln Tyr Gly Ser Ser Pro
                85                  90                  95

Thr Phe Gly Gln Gly Thr Lys Val Glu Ile Lys
            100                 105

<210>  128
<211>  12
<212>  PRT
<213>  Homo sapiens

<400>  128

Arg Ala Asn Gln Ile Ile Gly Asp Thr Ser Leu Ala
1               5                   10

<210>  129
<211>  7
<212>  PRT
<213>  Homo sapiens

<400>  129

Gly Ala Ser Ser Arg Ala Thr

1               5


<210>  130
<211>  8
<212>  PRT
<213>  Homo sapiens

<400>  130

Gln Gln Tyr Gly Ser Ser Pro Thr
1               5


<210>  131
<211>  1620
<212>  DNA
<213>  Human Metaneumovirus

<400>  131
atgtcttgga aagtgatgat catcatttcg ttactcataa caccccagca cgggctaaag      60

gagagttatt tggaagaatc atgcagtact ataactgagg gatacctcag tgttttaaga     120

acaggctggt acactaatgt cttcacatta gaagttggtg atgttgaaaa tcttacatgt     180

actgatggac ctagcttaat caaaacagaa cttgacctaa caaaaagtgc tttaagggaa     240

ctcaaaacag tctctgctga tcagttagct agagaggagc aaattgaaaa tcccagacaa     300

tcaagatttg tcctaggtgc aatagctctc ggagttgcta cagcagcagc agtcacagca     360

ggcattgcaa tagccaaaac cataaggctt gagagtgagg tgaatgcaat taaaggtgct     420

ctcaaacaaa ctaatgaagc agtatccaca ttaggaaatg gtgtgcgggt cctagccact     480

gcagtgagag agctaaaaga atttgtgagc aaaaatctga ctagtgcaat caacaggaac     540

aaatgtgaca ttgctgatct gaagatggct gtcagcttca gtcaattcaa cagaagattt     600

ctaaatgttg tgcggcagtt ttcagacaat gcagggataa caccagcaat atcattggac     660

ctaatgactg atgctgaatt ggccagagct gtatcataca tgccaacatc tgcaggacag     720

ataaaactga tgttggagaa ccgcgcaatg gtaaggagaa aaggatttgg aatcctaata     780

ggggtctacg gaagctctgt gatttacatg gttcaattgc cgatctttgg tgtcatagat     840

acaccttgtt ggataatcaa ggcagctccc tcttgctcag aaaaaaacgg gaattatgct     900

tgcctcctaa gagaggatca agggtggtat tgtaaaaatg caggatccac tgtttactac     960

ccaaatgaaa aagactgtga aacaagaggt gatcatgttt tttgtgacac agcagcaggg    1020

atcaatgttg ctgagcaatc aagagaatgc aacatcaaca tatctactac caactaccca    1080

tgcaaagtca gcacaggaag acaccctata agcatggttg cactatcacc tctcggtgct    1140

ttggtggctt gctataaagg ggtaagctgc tcgattggca gcaatcgagt tggaatcatc    1200

aaacaattac ctaaaggctg ctcatacata actaaccagg atgcagacac tgtaacaatt    1260

gacaataccg tgtatcaact aagcaaagtt gaaggtgaac agcatgtaat aaaagggaga    1320

ccagtttcaa gcagttttga tccaatcagg tttcctgagg atcagttcaa tgttgcgctt 1380

gatcaagtct tcgaaagcat tgagaacagt caggcactgg tggaacagtc aaacaaaatt 1440

ctaaacagtg cagaaaaagg aaacactggt ttcattattg taataatttt ggttgctgtt 1500

cttggtttaa ccatgatttc agtgagcatc atcatcataa tcaagaaaac aaggaagccc 1560

acaggagcac ctccagagct gaatggtgtc accaacggcg gtttcatacc acatagttag 1620

<210> 132
<211> 539
<212> PRT
<213> Artificial Sequence

<220>
<223> Fusion Protein Sequence

<400> 132

Met Ser Trp Lys Val Met Ile Ile Ile Ser Leu Leu Ile Thr Pro Gln
1               5                   10                  15

His Gly Leu Lys Glu Ser Tyr Leu Glu Glu Ser Cys Ser Thr Ile Thr
            20                  25                  30

Glu Gly Tyr Leu Ser Val Leu Arg Thr Gly Trp Tyr Thr Asn Val Phe
        35                  40                  45

Thr Leu Glu Val Gly Asp Val Glu Asn Leu Thr Cys Thr Asp Gly Pro
    50                  55                  60

Ser Leu Ile Lys Thr Glu Leu Asp Leu Thr Lys Ser Ala Leu Arg Glu
65                  70                  75                  80

Leu Lys Thr Val Ser Ala Asp Gln Leu Ala Arg Glu Glu Gln Ile Glu
                85                  90                  95

Asn Pro Arg Gln Ser Arg Phe Val Leu Gly Ala Ile Ala Leu Gly Val
            100                 105                 110

Ala Thr Ala Ala Ala Val Thr Ala Gly Ile Ala Ile Ala Lys Thr Ile
        115                 120                 125

Arg Leu Glu Ser Glu Val Asn Ala Ile Lys Gly Ala Leu Lys Gln Thr
    130                 135                 140

Asn Glu Ala Val Ser Thr Leu Gly Asn Gly Val Arg Val Leu Ala Thr
145                 150                 155                 160

Ala Val Arg Glu Leu Lys Glu Phe Val Ser Lys Asn Leu Thr Ser Ala

                165                 170                 175

Ile Asn Arg Asn Lys Cys Asp Ile Ala Asp Leu Lys Met Ala Val Ser
            180                 185                 190

Phe Ser Gln Phe Asn Arg Arg Phe Leu Asn Val Val Arg Gln Phe Ser
        195                 200                 205

Asp Asn Ala Gly Ile Thr Pro Ala Ile Ser Leu Asp Leu Met Thr Asp
    210                 215                 220

Ala Glu Leu Ala Arg Ala Val Ser Tyr Met Pro Thr Ser Ala Gly Gln
225                 230                 235                 240

Ile Lys Leu Met Leu Glu Asn Arg Ala Met Val Arg Arg Lys Gly Phe
                245                 250                 255

Gly Ile Leu Ile Gly Val Tyr Gly Ser Ser Val Ile Tyr Met Val Gln
            260                 265                 270

Leu Pro Ile Phe Gly Val Ile Asp Thr Pro Cys Trp Ile Ile Lys Ala
        275                 280                 285

Ala Pro Ser Cys Ser Glu Lys Asn Gly Asn Tyr Ala Cys Leu Leu Arg
    290                 295                 300

Glu Asp Gln Gly Trp Tyr Cys Lys Asn Ala Gly Ser Thr Val Tyr Tyr
305                 310                 315                 320

Pro Asn Glu Lys Asp Cys Glu Thr Arg Gly Asp His Val Phe Cys Asp
                325                 330                 335

Thr Ala Ala Gly Ile Asn Val Ala Glu Gln Ser Arg Glu Cys Asn Ile
            340                 345                 350

Asn Ile Ser Thr Thr Asn Tyr Pro Cys Lys Val Ser Thr Gly Arg His
        355                 360                 365

Pro Ile Ser Met Val Ala Leu Ser Pro Leu Gly Ala Leu Val Ala Cys
    370                 375                 380

Tyr Lys Gly Val Ser Cys Ser Ile Gly Ser Asn Arg Val Gly Ile Ile
385                 390                 395                 400

Lys Gln Leu Pro Lys Gly Cys Ser Tyr Ile Thr Asn Gln Asp Ala Asp
                405                 410                 415

Thr Val Thr Ile Asp Asn Thr Val Tyr Gln Leu Ser Lys Val Glu Gly
            420                 425                 430

Glu Gln His Val Ile Lys Gly Arg Pro Val Ser Ser Ser Phe Asp Pro
        435                 440                 445

Ile Arg Phe Pro Glu Asp Gln Phe Asn Val Ala Leu Asp Gln Val Phe
    450                 455                 460

Glu Ser Ile Glu Asn Ser Gln Ala Leu Val Glu Gln Ser Asn Lys Ile
465                 470                 475                 480

Leu Asn Ser Ala Glu Lys Gly Asn Thr Gly Phe Ile Ile Val Ile Ile
                485                 490                 495

Leu Val Ala Val Leu Gly Leu Thr Met Ile Ser Val Ser Ile Ile Ile
            500                 505                 510

Ile Ile Lys Lys Thr Arg Lys Pro Thr Gly Ala Pro Pro Glu Leu Asn
        515                 520                 525

Gly Val Thr Asn Gly Gly Phe Ile Pro His Ser
    530                 535

<210>  133
<211>  1738
<212>  DNA
<213>  Artificial Sequence

<220>
<223>  HA-V5-Full

<400>  133
aagcttgcca ccatgtcttg gaaagtgatg atcatcattt cgttactcat aacaccccag      60

cacgggacca tgtacccata cgatgttcca gattacgctc taaaggagag ttatttggaa     120

gaatcatgca gtactataac tgagggatac ctcagtgttt taagaacagg ctggtacact     180

aatgtcttca cattagaagt tggtgatgtt gaaaatctta catgtactga tggacctagc     240

ttaatcaaaa cagaacttga cctaacaaaa agtgctttaa gggaactcaa aacagtctct     300

gctgatcagt tagctagaga ggagcaaatt gaaaatccca gacaatcaag atttgtccta     360

ggtgcaatag ctctcggagt tgctacagca gcagcagtca cagcaggcat tgcaatagcc     420

aaaaccataa ggcttgagag tgaggtgaat gcaattaaag gtgctctcaa acaaactaat     480

gaagcagtat ccacattagg aaatggtgtg cgggtcctag ccactgcagt gagagagcta     540

aaagaatttg tgagcaaaaa tctgactagt gcaatcaaca ggaacaaatg tgacattgct     600

gatctgaaga tggctgtcag cttcagtcaa ttcaacagaa gatttctaaa tgttgtgcgg     660

cagttttcag acaatgcagg gataacacca gcaatatcat tggacctaat gactgatgct 720

gaattggcca gagctgtatc atacatgcca acatctgcag gacagataaa actgatgttg 780

gagaaccgcg caatggtaag gagaaaagga tttggaatcc taataggggt ctacggaagc 840

tctgtgattt acatggttca attgccgatc tttggtgtca tagatacacc ttgttggata 900

atcaaggcag ctccctcttg ctcagaaaaa aacgggaatt atgcttgcct cctaagagag 960

gatcaagggt ggtattgtaa aaatgcagga tccactgttt actacccaaa tgaaaaagac 1020

tgtgaaacaa gaggtgatca tgttttttgt gacacagcag cagggatcaa tgttgctgag 1080

caatcaagag aatgcaacat caacatatct actaccaact acccatgcaa agtcagcaca 1140

ggaagacacc ctataagcat ggttgcacta tcacctctcg gtgctttggt ggcttgctat 1200

aaaggggtaa gctgctcgat tggcagcaat cgagttggaa tcatcaaaca attacctaaa 1260

ggctgctcat acataactaa ccaggatgca gacactgtaa caattgacaa taccgtgtat 1320

caactaagca aagttgaagg tgaacagcat gtaataaaag ggagaccagt ttcaagcagt 1380

tttgatccaa tcaggtttcc tgaggatcag ttcaatgttg cgcttgatca agtcttcgaa 1440

agcattgaga acagtcaggc actggtggaa cagtcaaaca aaattctaaa cagtgcagaa 1500

aaaggaaaca ctggtttcat tattgtaata attttggttg ctgttcttgg tttaaccatg 1560

atttcagtga gcatcatcat cataatcaag aaaacaagga agcccacagg agcacctcca 1620

gagctgaatg gtgtcaccaa cggcggtttc ataccacata gtagtctaga gggcccgcgg 1680

ttcgaaggta agcctatccc taaccctctc ctcggtctcg attctacgta gcggccgc 1738

<210> 134
<211> 572
<212> PRT
<213> Artificial Sequence

<220>
<223> HA-V5-Full

<400> 134

Met Ser Trp Lys Val Met Ile Ile Ile Ser Leu Leu Ile Thr Pro Gln
1               5                   10                  15

His Gly Thr Met Tyr Pro Tyr Asp Val Pro Asp Tyr Ala Leu Lys Glu
            20                  25                  30

Ser Tyr Leu Glu Glu Ser Cys Ser Thr Ile Thr Glu Gly Tyr Leu Ser
        35                  40                  45

Val Leu Arg Thr Gly Trp Tyr Thr Asn Val Phe Thr Leu Glu Val Gly
    50                  55                  60

Asp Val Glu Asn Leu Thr Cys Thr Asp Gly Pro Ser Leu Ile Lys Thr
65                  70                  75                  80

Glu Leu Asp Leu Thr Lys Ser Ala Leu Arg Glu Leu Lys Thr Val Ser
                85                  90                  95

Ala Asp Gln Leu Ala Arg Glu Glu Gln Ile Glu Asn Pro Arg Gln Ser
            100                 105                 110

Arg Phe Val Leu Gly Ala Ile Ala Leu Gly Val Ala Thr Ala Ala Ala
        115                 120                 125

Val Thr Ala Gly Ile Ala Ile Ala Lys Thr Ile Arg Leu Glu Ser Glu
    130                 135                 140

Val Asn Ala Ile Lys Gly Ala Leu Lys Gln Thr Asn Glu Ala Val Ser
145                 150                 155                 160

Thr Leu Gly Asn Gly Val Arg Val Leu Ala Thr Ala Val Arg Glu Leu
                165                 170                 175

Lys Glu Phe Val Ser Lys Asn Leu Thr Ser Ala Ile Asn Arg Asn Lys
            180                 185                 190

Cys Asp Ile Ala Asp Leu Lys Met Ala Val Ser Phe Ser Gln Phe Asn
        195                 200                 205

Arg Arg Phe Leu Asn Val Val Arg Gln Phe Ser Asp Asn Ala Gly Ile
    210                 215                 220

Thr Pro Ala Ile Ser Leu Asp Leu Met Thr Asp Ala Glu Leu Ala Arg
225                 230                 235                 240

Ala Val Ser Tyr Met Pro Thr Ser Ala Gly Gln Ile Lys Leu Met Leu
                245                 250                 255

Glu Asn Arg Ala Met Val Arg Arg Lys Gly Phe Gly Ile Leu Ile Gly
            260                 265                 270

Val Tyr Gly Ser Ser Val Ile Tyr Met Val Gln Leu Pro Ile Phe Gly
        275                 280                 285

Val Ile Asp Thr Pro Cys Trp Ile Ile Lys Ala Ala Pro Ser Cys Ser
    290                 295                 300

Glu Lys Asn Gly Asn Tyr Ala Cys Leu Leu Arg Glu Asp Gln Gly Trp
305                 310                 315                 320

Tyr Cys Lys Asn Ala Gly Ser Thr Val Tyr Tyr Pro Asn Glu Lys Asp
                325                 330                 335

Cys Glu Thr Arg Gly Asp His Val Phe Cys Asp Thr Ala Ala Gly Ile
            340                 345                 350

Asn Val Ala Glu Gln Ser Arg Glu Cys Asn Ile Asn Ile Ser Thr Thr
        355                 360                 365

Asn Tyr Pro Cys Lys Val Ser Thr Gly Arg His Pro Ile Ser Met Val
    370                 375                 380

Ala Leu Ser Pro Leu Gly Ala Leu Val Ala Cys Tyr Lys Gly Val Ser
385                 390                 395                 400

Cys Ser Ile Gly Ser Asn Arg Val Gly Ile Ile Lys Gln Leu Pro Lys
                405                 410                 415

Gly Cys Ser Tyr Ile Thr Asn Gln Asp Ala Asp Thr Val Thr Ile Asp
            420                 425                 430

Asn Thr Val Tyr Gln Leu Ser Lys Val Glu Gly Glu Gln His Val Ile
        435                 440                 445

Lys Gly Arg Pro Val Ser Ser Ser Phe Asp Pro Ile Arg Phe Pro Glu
    450                 455                 460

Asp Gln Phe Asn Val Ala Leu Asp Gln Val Phe Glu Ser Ile Glu Asn
465                 470                 475                 480

Ser Gln Ala Leu Val Glu Gln Ser Asn Lys Ile Leu Asn Ser Ala Glu
                485                 490                 495

Lys Gly Asn Thr Gly Phe Ile Ile Val Ile Ile Leu Val Ala Val Leu
            500                 505                 510

Gly Leu Thr Met Ile Ser Val Ser Ile Ile Ile Ile Ile Lys Lys Thr
        515                 520                 525

Arg Lys Pro Thr Gly Ala Pro Pro Glu Leu Asn Gly Val Thr Asn Gly
    530                 535                 540

Gly Phe Ile Pro His Ser Ser Leu Glu Gly Pro Arg Phe Glu Gly Lys
545                 550                 555                 560

Pro Ile Pro Asn Pro Leu Leu Gly Leu Asp Ser Thr
                565                 570

## Description

### TECHNICAL FIELD

The present invention relates to a monoclonal antibody specifically binding to human metapneumovirus F protein and neutralizing the biological activity of the human metapneumovirus, and an antigen-binding fragment thereof.

### BACKGROUND ART

Respiratory infection is one of the major diseases in humans in terms of morbidity and death rate. Particularly, in children below the age of 5, the respiratory infection is ranked in one of the leading causes of death. In the USA, the respiratory infection, including pneumonia, influenza, and influenza-like diseases, kills 45,000 or more people every year, and a yearly medical cost reportedly reaches 14.6 billion dollars (Non Patent Literature 1).

Respiratory syncytial virus (RSV), parainfluenza virus, influenza virus, coronavirus, rhinovirus, adenovirus, and the like are known as causative viruses of respiratory infection. Recently, viruses causing respiratory infection, including human metapneumovirus (hMPV), SARS coronavirus, and human bocavirus, have been discovered one after another. Among them, hMPV is a virus that was separated and identified for the first time by a Dutch research group in 2001 (Non Patent Literature 2). This hMPV virus had always been found universally in humans, but had been difficult to separate and therefore not been discovered.

The human metapneumovirus (hMPV), a single-stranded (negative strand) RNA virus, is classified into the genus *Metapneumovirus* within the subfamily *Paramyxoviridae* of the family *Paramyxoviridae* and has eight genes respectively encoding N protein, P protein, M protein, F protein, M2 protein, SH protein, G protein, and L protein (Non Patent Literature 2). The gene sequence of hMPV is most analogous to that of avian pneumovirus (APV) and is also analogous to RSV in terms of gene sequence and clinical symptoms among human viruses (Non Patent Literature 3). hMPV is classified according to serological characteristics into two groups, A and B, which are further classified into their two respective subgroups (A1 and A2, and B1 and B2) (Non Patent Literature 4). Among them, the strain A2 is the mainstream in pandemic outbreaks (Non Patent Literature 5). Although the F protein, the G protein, and the SH protein reside on the virus surface, the main neutralizing antibody against hMPV present in serum has been reported to be an antibody against the F protein (Non Patent Literatures 6 and 7).

According to investigation conducted on pediatric patients with respiratory infection below the age of 5 by the group of Williams et al., it has been reported that 20% of the patients with infection of the lower respiratory tract and 15% of the patients with infection of the upper respiratory tract were infected by hMPV (Non Patent Literature 7). Six-month-old to 1-year-old infants having low antibody titers in blood, immune-compromised senior citizens, or immunodeficient patients are at the risk of increase in severity and require particular attention.

Although methods for examining hMPV have already been established, symptomatic treatment is currently the basic treatment of hMPV in the absence of effective antivirus drugs. Research has been conducted on, for example, the development of antibody drugs specific for hMPV F protein (Patent Literatures 1 and 2, and Non Patent Literature 9) and vaccines prepared using attenuated viruses or the like (Patent Literatures 3 and 4) as therapeutic or preventive drugs for hMPV. A human antibody binding both to the F proteins of hMPV and RSV (Patent Literature 5) has been further reported recently. Any of these approaches have only just begun.

### CITATION LIST

### Patent Literature

Patent Literature 1: WO2006/110214
Patent Literature 2: WO2008/043052
Patent Literature 3: WO2002/057302
Patent Literature 4: WO2003/072719
Patent Literature 5: WO2013/140247
Non Patent Literature

Non Patent Literature 1: Clin. Micro. Reviews, 2006 (vol. 19) p. 546
Non Patent Literature 2: Nature Medicine, 2001 (vol. 7) p. 719
Non Patent Literature 3: Virology, 2002 (vol. 295) p. 119
Non Patent Literature 4: Emerg. Infect. Dis., 2004 (vol. 10) p. 658
Non Patent Literature 5: PLoS ONE, 2012 (vol. 7) e34544
Non Patent Literature 6: J. Virol., 2004 (vol. 78) p. 6927
Non Patent Literature 7: J. Gen. Virol., 2004 (vol. 85) p. 1655
Non Patent Literature 8: New Eng. J. Med., 2004 (vol. 350) p. 443
Non Patent Literature 9: J. Virol., 2006 (vol. 80) p. 7799

### SUMMARY OF INVENTION

### TECHNICAL PROBLEM

Human metapneumovirus (hMPV), which was revealed as a causative virus of respiratory infection in 2001, causes this disease in 15 to 20% of pediatric patients with respiratory infection below the age of 5 and incurs the risk of increase in severity, particularly, in 6-month-old to 1-year-old infants, immune-compromised senior citizens, or immunodeficient patients. Incidentally, RSV is found as a human virus analogous to hMPV, and an antibody drug (Synagis) specifically recognizing RSV F protein has been approved as a preventive drug for the disease and is sold in many countries. Still, the treatment of hMPV is merely symptomatic treatment. Meanwhile, for example, research on vaccines prepared using attenuated viruses or the like (Patent Literatures 3 and 4) and research on antibody drugs specific for hMPV F protein have been reported. Only a human Fab antibody DSλ7, etc., recognizing the F protein of the virus, as with Synagis, (Patent Literature 2) has been reported as an example of a human-derived antibody.

The antibody DSλ7, etc., has still low activity in light of affinity, neutralizing activity, or the like, and is still susceptible to improvement as a drug candidate. Particularly, antibodies having high neutralizing activity against a wide range of strains, A1, A2, B1, and B2, if possible, antibodies having high neutralizing activity against the hMPV_A2 strain, which is the mainstream in recent pandemic outbreaks, have been demanded as antibody drugs for hMPV.

In addition, antibodies differing in epitope are also necessary in consideration of the resistance of the virus. All of chimeric antibodies and humanized antibodies have problems associated with immunogenicity. Completely human-derived antibodies have also been strongly demanded as drug candidate anti-hMPV antibodies.

### SOLUTION TO PROBLEM

Under these circumstances, the present inventors have conducted diligent studies with the aim of preparing human-derived monoclonal antibodies that exhibit effectiveness equal to or higher than ever at lower doses. As a result, the present inventors have successfully prepared a plurality of antibodies that specifically bind to hMPV F protein, have high neutralizing activity against a wide range of hMPV strains, and are classified into some different groups by epitope grouping. In this way, the present invention has been completed.

Specifically, the present invention relates to a human monoclonal antibody specific for human metapneumovirus (hMPV) F protein or an antigen-binding fragment thereof, a nucleic acid (polynucleotide) encoding the antibody or an antigen-binding fragment thereof, an expression vector containing the nucleic acid, a host cell containing the vector, and a pharmaceutical composition comprising the antibody of the present invention or an antigen-binding fragment, as described below in [1] to [41].

[1] An antibody capable of specifically binding to human metapneumovirus F protein and neutralizing its biological activity, or an antigen-binding fragment thereof, comprising a heavy chain variable region and a light chain variable region, wherein
   (i) the heavy chain variable region comprises:
      (a) the amino acid sequence of heavy chain CDR1 selected from the group consisting of the amino acid sequences of SEQ ID NOs: 3, 13, 23, 33, 43, 53, 63, 73, 83, 93, 103, 113, and 123 and amino acid sequences derived from any of these amino acid sequences by the deletion, substitution, insertion, and/or addition mutations of one to several amino acid residues,
      (b) the amino acid sequence of heavy chain CDR2 selected from the group consisting of the amino acid sequences of SEQ ID NOs: 4, 14, 24, 34, 44, 54, 64, 74, 84, 94, 104, 114, and 124 and amino acid sequences derived from any of these amino acid sequences by the deletion, substitution, insertion, and/or addition mutations of one to several amino acid residues, and
      (c) the amino acid sequence of heavy chain CDR3 selected from the group consisting of the amino acid sequences of SEQ ID NOs: 5, 15, 25, 35, 45, 55, 65, 75, 85, 95, 105, 115, and 125 and amino acid sequences derived from any of these amino acid sequences by the deletion, substitution, insertion, and/or addition mutations of one to several amino acid residues, and
   (ii) the light chain variable region comprises:
      (a) the amino acid sequence of light chain CDR1 selected from the group consisting of the amino acid sequences of SEQ ID NOs: 8, 18, 28, 38, 48, 58, 68, 78, 88, 98, 108, 118, and 128 and amino acid sequences derived from any of these amino acid sequences by the deletion, substitution, insertion, and/or addition mutations of one to several amino acid residues,
      (b) the amino acid sequence of light chain CDR2 selected from the group consisting of the amino acid sequences of SEQ ID NOs: 9, 19, 29, 39, 49, 59, 69, 79, 89, 99, 109, 119, and 129 and amino acid sequences derived from any of these amino acid sequences by the deletion, substitution, insertion, and/or addition mutations of one to several amino acid residues, and
      (c) the amino acid sequence of light chain CDR3 selected from the group consisting of the amino acid sequences of SEQ ID NOs: 10, 20, 30, 40, 50, 60, 70, 80, 90, 100, 110, 120, and 130 and amino acid sequences derived from any of these amino acid sequences by the deletion, substitution, insertion, and/or addition mutations of one to several amino acid residues.
[2] The antibody or an antigen-binding fragment thereof according to [1], wherein
   (i) the heavy chain variable region comprises:
      (a) the amino acid sequence of heavy chain CDR1 comprising the amino acid sequence of SEQ ID NO: 3,
      (b) the amino acid sequence of heavy chain CDR2 comprising the amino acid sequence of SEQ ID NO: 4, and
      (c) the amino acid sequence of heavy chain CDR3 comprising the amino acid sequence of SEQ ID NO: 5, and
   (ii) the light chain variable region comprises:
      (a) the amino acid sequence of light chain CDR1 comprising the amino acid sequence of SEQ ID NO: 8,
      (b) the amino acid sequence of light chain CDR2 comprising the amino acid sequence of SEQ ID NO: 9, and
      (c) the amino acid sequence of light chain CDR3 comprising the amino acid sequence of SEQ ID NO: 10.
[3] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 13,
      (b) the amino acid sequence of heavy chain CDR2 comprising the amino acid sequence of SEQ ID NO: 14, and
      (c) the amino acid sequence of heavy chain CDR3 comprising the amino acid sequence of SEQ ID NO: 15, and
   (ii) the light chain variable region comprises
      (a) the amino acid sequence of light chain CDR1 comprising the amino acid sequence of SEQ ID NO: 18,
      (b) the amino acid sequence of light chain CDR2 comprising the amino acid sequence of SEQ ID NO: 19, and
      (c) the amino acid sequence of light chain CDR3 comprising the amino acid sequence of SEQ ID NO: 20.
[4] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 23,
      (b) the amino acid sequence of heavy chain CDR2 comprising the amino acid sequence of SEQ ID NO: 24, and
      (c) the amino acid sequence of heavy chain CDR3 comprising the amino acid sequence of SEQ ID NO: 25, and
   (ii) the light chain variable region comprises
      (a) the amino acid sequence of light chain CDR1 comprising the amino acid sequence of SEQ ID NO: 28,
      (b) the amino acid sequence of light chain CDR2 comprising the amino acid sequence of SEQ ID NO: 29, and
      (c) the amino acid sequence of light chain CDR3 comprising the amino acid sequence of SEQ ID NO: 30.
[5] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 33,
      (b) the amino acid sequence of heavy chain CDR2 comprising the amino acid sequence of SEQ ID NO: 34, and
      (c) the amino acid sequence of heavy chain CDR3 comprising the amino acid sequence of SEQ ID NO: 35, and
   (ii) the light chain variable region comprises
      (a) the amino acid sequence of light chain CDR1 comprising the amino acid sequence of SEQ ID NO: 38,
      (b) the amino acid sequence of light chain CDR2 comprising the amino acid sequence of SEQ ID NO: 39, and
      (c) the amino acid sequence of light chain CDR3 comprising the amino acid sequence of SEQ ID NO: 40.
[6] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 43,
      (b) the amino acid sequence of heavy chain CDR2 comprising the amino acid sequence of SEQ ID NO: 44, and
      (c) the amino acid sequence of heavy chain CDR3 comprising the amino acid sequence of SEQ ID NO: 45, and
   (ii) the light chain variable region comprises
      (a) the amino acid sequence of light chain CDR1 comprising the amino acid sequence of SEQ ID NO: 48,
      (b) the amino acid sequence of light chain CDR2 comprising the amino acid sequence of SEQ ID NO: 49, and
      (c) the amino acid sequence of light chain CDR3 comprising the amino acid sequence of SEQ ID NO: 50.
[7] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 53,
      (b) the amino acid sequence of heavy chain CDR2 comprising the amino acid sequence of SEQ ID NO: 54, and
      (c) the amino acid sequence of heavy chain CDR3 comprising the amino acid sequence of SEQ ID NO: 55, and
   (ii) the light chain variable region comprises
      (a) the amino acid sequence of light chain CDR1 comprising the amino acid sequence of SEQ ID NO: 58,
      (b) the amino acid sequence of light chain CDR2 comprising the amino acid sequence of SEQ ID NO: 59, and
      (c) the amino acid sequence of light chain CDR3 comprising the amino acid sequence of SEQ ID NO: 60.
[8] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 63,
      (b) the amino acid sequence of heavy chain CDR2 comprising the amino acid sequence of SEQ ID NO: 64, and
      (c) the amino acid sequence of heavy chain CDR3 comprising the amino acid sequence of SEQ ID NO: 65, and
   (ii) the light chain variable region comprises
      (a) the amino acid sequence of light chain CDR1 comprising the amino acid sequence of SEQ ID NO: 68,
      (b) the amino acid sequence of light chain CDR2 comprising the amino acid sequence of SEQ ID NO: 69, and
      (c) the amino acid sequence of light chain CDR3 comprising the amino acid sequence of SEQ ID NO: 70.
[9] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 73,
      (b) the amino acid sequence of heavy chain CDR2 comprising the amino acid sequence of SEQ ID NO: 74, and
      (c) the amino acid sequence of heavy chain CDR3 comprising the amino acid sequence of SEQ ID NO: 75, and
   (ii) the light chain variable region comprises
      (a) the amino acid sequence of light chain CDR1 comprising the amino acid sequence of SEQ ID NO: 78,
      (b) the amino acid sequence of light chain CDR2 comprising the amino acid sequence of SEQ ID NO: 79, and
      (c) the amino acid sequence of light chain CDR3 comprising the amino acid sequence of SEQ ID NO: 80.
[10] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 83,
      (b) the amino acid sequence of heavy chain CDR2 comprising the amino acid sequence of SEQ ID NO: 84, and
      (c) the amino acid sequence of heavy chain CDR3 comprising the amino acid sequence of SEQ ID NO: 85, and
   (ii) the light chain variable region comprises
      (a) the amino acid sequence of light chain CDR1 comprising the amino acid sequence of SEQ ID NO: 88,
      (b) the amino acid sequence of light chain CDR2 comprising the amino acid sequence of SEQ ID NO: 89, and
      (c) the amino acid sequence of light chain CDR3 comprising the amino acid sequence of SEQ ID NO: 90.
[11] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 93,
      (b) the amino acid sequence of heavy chain CDR2 comprising the amino acid sequence of SEQ ID NO: 94, and
      (c) the amino acid sequence of heavy chain CDR3 comprising the amino acid sequence of SEQ ID NO: 95, and
   (ii) the light chain variable region comprises
      (a) the amino acid sequence of light chain CDR1 comprising the amino acid sequence of SEQ ID NO: 98,
      (b) the amino acid sequence of light chain CDR2 comprising the amino acid sequence of SEQ ID NO: 99, and
      (c) the amino acid sequence of light chain CDR3 comprising the amino acid sequence of SEQ ID NO: 100.
[12] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 103,
      (b) the amino acid sequence of heavy chain CDR2 comprising the amino acid sequence of SEQ ID NO: 104, and
      (c) the amino acid sequence of heavy chain CDR3 comprising the amino acid sequence of SEQ ID NO: 105, and
   (ii) the light chain variable region comprises
      (a) the amino acid sequence of light chain CDR1 comprising the amino acid sequence of SEQ ID NO: 108,
      (b) the amino acid sequence of light chain CDR2 comprising the amino acid sequence of SEQ ID NO: 109, and
      (c) the amino acid sequence of light chain CDR3 comprising the amino acid sequence of SEQ ID NO: 110.
[13] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 113,
      (b) the amino acid sequence of heavy chain CDR2 comprising the amino acid sequence of SEQ ID NO: 114, and
      (c) the amino acid sequence of heavy chain CDR3 comprising the amino acid sequence of SEQ ID NO: 115, and
   (ii) the light chain variable region comprises
      (a) the amino acid sequence of light chain CDR1 comprising the amino acid sequence of SEQ ID NO: 118,
      (b) the amino acid sequence of light chain CDR2 comprising the amino acid sequence of SEQ ID NO: 119, and
      (c) the amino acid sequence of light chain CDR3 comprising the amino acid sequence of SEQ ID NO: 120.
[14] The antibody or an antigen-binding fragment thereof according to [1], wherein
   (i) the heavy chain variable region comprises
      (a) the amino acid sequence of heavy chain CDR1 comprising the amino acid sequence of SEQ ID NO: 123,
      (b) the amino acid sequence of heavy chain CDR2 comprising the amino acid sequence of SEQ ID NO: 124, and
      (c) the amino acid sequence of heavy chain CDR3 comprising the amino acid sequence of SEQ ID NO: 125, and
   (ii) the light chain variable region comprises
      (a) the amino acid sequence of light chain CDR1 comprising the amino acid sequence of SEQ ID NO: 128,
      (b) the amino acid sequence of light chain CDR2 comprising the amino acid sequence of SEQ ID NO: 129, and
      (c) the amino acid sequence of light chain CDR3 comprising the amino acid sequence of SEQ ID NO: 130.
[15] The antibody or an antigen-binding fragment thereof according to any one of [1] to [14], wherein the antibody comprises
   (a) a heavy chain variable region (VH) selected from the group consisting of the amino acid sequences of SEQ ID NOs: 2, 12, 22, 32, 42, 52, 62, 72, 82, 92, 102, 112, and 122 and amino acid sequences having 95% or higher identity to any of these amino acid sequences, and
   (b) a light chain variable region (VL) selected from the group consisting of the amino acid sequences of SEQ ID NOs: 7, 17, 27, 37, 47, 57, 67, 77, 87, 97, 107, 117, and 127 and amino acid sequences having 95% or higher identity to any of these amino acid sequences.
[16] The antibody or an antigen-binding fragment thereof according to [15], wherein the antibody comprises
   (a) a heavy chain variable region (VH) comprising the amino acid sequence of SEQ ID NO: 2 or an amino acid sequence having 95% or higher identity to the amino acid sequence of SEQ ID NO: 2, and
   (b) a light chain variable region (VL) comprising the amino acid sequence of SEQ ID NO: 7 or an amino acid sequence having 95% or higher identity to the amino acid sequence of SEQ ID NO: 7.
[17] The antibody or an antigen-binding fragment thereof according to [15], wherein the antibody comprises
   (a) a heavy chain variable region (VH) comprising the amino acid sequence of SEQ ID NO: 12 or an amino acid sequence having 95% or higher identity to the amino acid sequence of SEQ ID NO: 12, and
   (b) a light chain variable region (VL) comprising the amino acid sequence of SEQ ID NO: 17 or an amino acid sequence having 95% or higher identity to the amino acid sequence of SEQ ID NO: 17.
[18] The antibody or an antigen-binding fragment thereof according to [15], wherein the antibody comprises
   (a) a heavy chain variable region (VH) comprising the amino acid sequence of SEQ ID NO: 22 or an amino acid sequence having 95% or higher identity to the amino acid sequence of SEQ ID NO: 22, and
   (b) a light chain variable region (VL) comprising the amino acid sequence of SEQ ID NO: 27 or an amino acid sequence having 95% or higher identity to the amino acid sequence of SEQ ID NO: 27.
[19] The antibody or an antigen-binding fragment thereof according to [15], wherein the antibody comprises
   (a) a heavy chain variable region (VH) comprising the amino acid sequence of SEQ ID NO: 32 or an amino acid sequence having 95% or higher identity to the amino acid sequence of SEQ ID NO: 32, and
   (b) a light chain variable region (VL) comprising the amino acid sequence of SEQ ID NO: 37 or an amino acid sequence having 95% or higher identity to the amino acid sequence of SEQ ID NO: 37.
[20] The antibody or an antigen-binding fragment thereof according to [15], wherein the antibody comprises
   (a) a heavy chain variable region (VH) comprising the amino acid sequence of SEQ ID NO: 42 or an amino acid sequence having 95% or higher identity to the amino acid sequence of SEQ ID NO: 42, and
   (b) a light chain variable region (VL) comprising the amino acid sequence of SEQ ID NO: 47 or an amino acid sequence having 95% or higher identity to the amino acid sequence of SEQ ID NO: 47.
[21] The antibody or an antigen-binding fragment thereof according to [15], wherein the antibody comprises
   (a) a heavy chain variable region (VH) comprising the amino acid sequence of SEQ ID NO: 52 or an amino acid sequence having 95% or higher identity to the amino acid sequence of SEQ ID NO: 52, and
   (b) a light chain variable region (VL) comprising the amino acid sequence of SEQ ID NO: 57 or an amino acid sequence having 95% or higher identity to the amino acid sequence of SEQ ID NO: 57.
[22] The antibody or an antigen-binding fragment thereof according to [15], wherein the antibody comprises
   (a) a heavy chain variable region (VH) comprising the amino acid sequence of SEQ ID NO: 62 or an amino acid sequence having 95% or higher identity to the amino acid sequence of SEQ ID NO: 62, and
   (b) a light chain variable region (VL) comprising the amino acid sequence of SEQ ID NO: 67 or an amino acid sequence having 95% or higher identity to the amino acid sequence of SEQ ID NO: 67.
[23] The antibody or an antigen-binding fragment thereof according to [15], wherein the antibody comprises
   (a) a heavy chain variable region (VH) comprising the amino acid sequence of SEQ ID NO: 72 or an amino acid sequence having 95% or higher identity to the amino acid sequence of SEQ ID NO: 72, and
   (b) a light chain variable region (VL) comprising the amino acid sequence of SEQ ID NO: 77 or an amino acid sequence having 95% or higher identity to the amino acid sequence of SEQ ID NO: 77.
[24] The antibody or an antigen-binding fragment thereof according to [15], wherein the antibody comprises
   (a) a heavy chain variable region (VH) comprising the amino acid sequence of SEQ ID NO: 82 or an amino acid sequence having 95% or higher identity to the amino acid sequence of SEQ ID NO: 82, and
   (b) a light chain variable region (VL) comprising the amino acid sequence of SEQ ID NO: 87 or an amino acid sequence having 95% or higher identity to the amino acid sequence of SEQ ID NO: 87.
[25] The antibody or an antigen-binding fragment thereof according to [15], wherein the antibody comprises
   (a) a heavy chain variable region (VH) comprising the amino acid sequence of SEQ ID NO: 92 or an amino acid sequence having 95% or higher identity to the amino acid sequence of SEQ ID NO: 92, and
   (b) a light chain variable region (VL) comprising the amino acid sequence of SEQ ID NO: 97 or an amino acid sequence having 95% or higher identity to the amino acid sequence of SEQ ID NO: 97.
[26] The antibody or an antigen-binding fragment thereof according to [15], wherein the antibody comprises
   (a) a heavy chain variable region (VH) comprising the amino acid sequence of SEQ ID NO: 102 or an amino acid sequence having 95% or higher identity to the amino acid sequence of SEQ ID NO: 102, and
   (b) a light chain variable region (VL) comprising the amino acid sequence of SEQ ID NO: 107 or an amino acid sequence having 95% or higher identity to the amino acid sequence of SEQ ID NO: 107.
[27] The antibody or an antigen-binding fragment thereof according to [15], wherein the antibody comprises
   (a) a heavy chain variable region (VH) comprising the amino acid sequence of SEQ ID NO: 112 or an amino acid sequence having 95% or higher identity to the amino acid sequence of SEQ ID NO: 112, and
   (b) a light chain variable region (VL) comprising the amino acid sequence of SEQ ID NO: 117 or an amino acid sequence having 95% or higher identity to the amino acid sequence of SEQ ID NO: 117.
[28] The antibody or an antigen-binding fragment thereof according to [15], wherein the antibody comprises
   (a) a heavy chain variable region (VH) comprising the amino acid sequence of SEQ ID NO: 122 or an amino acid sequence having 95% or higher identity to the amino acid sequence of SEQ ID NO: 122, and
   (b) a light chain variable region (VL) comprising the amino acid sequence of SEQ ID NO: 127 or an amino acid sequence having 95% or higher identity to the amino acid sequence of SEQ ID NO: 127.
[29] The antibody or an antigen-binding fragment thereof according to any one of [1] to [28], wherein the epitope of the antibody belongs to any of epitope groups Group 1 typified by anti-hMPV antibodies EV046115b, EV046130, and EV046147, Group 2 typified by anti-hMPV antibodies EV046113, EV046116, EV046141, and EV0461142, Group 3 typified by anti-hMPV antibodies EV046124, EV046143, and EV046150, Group 4 typified by anti-hMPV antibodies EV046120 and EV046135, and Group 5 typified by an anti-hMPV antibody EV046136 in epitope grouping according to binding activity against a partially deleted peptide of the F protein.
[30] The antibody or an antigen-binding fragment thereof according to any one of [1] to [28], wherein the epitope of the antibody belongs to any of epitope groups Group 2 typified by anti-hMPV antibodies EV046113, EV046116, EV046141, and EV0461142, Group 3 typified by anti-hMPV antibodies EV046124, EV046143, and EV046150, Group 4 typified by anti-hMPV antibodies EV046120 and EV046135, and Group 5 typified by an anti-hMPV antibody EV046136 in epitope grouping according to binding activity against a partially deleted peptide of the F protein.
[31] The antibody or an antigen-binding fragment thereof according to any one of [1] to [28], wherein the epitope of the antibody belongs to an epitope group Group 3 typified by anti-hMPV antibodies EV046124, EV046143, and EV046150 in epitope grouping according to binding activity against a partially deleted peptide of the F protein.
[32] The antibody or an antigen-binding fragment thereof according to any one of [1] to [31], wherein the antibody is of IgG1 (κ) or IgG1 (λ) class (subclass).
[33] The antibody or an antigen-binding fragment thereof according to any one of [1] to [32], wherein the antibody has a neutralizing activity (IC50) of approximately 1 µg/mL (approximately 6.7 nM) or lower against all of human metapneumovirus strains JPS02-76 (type B1), JPS05-21 (type B2), and JPS03-180 (type A1).
[34] The antibody or an antigen-binding fragment thereof according to any one of [1] to [32], wherein the antibody has a neutralizing activity (IC50) of approximately 1 µg/mL (approximately 6.7 nM) or lower against all of human metapneumovirus strains JPS03-180 (type A1), JPS03-178 (type A2), JPS02-76 (type B1), and JPS05-21 (type B2).
[35] The antibody or an antigen-binding fragment thereof according to any one of [1] to [32], wherein the antibody has a neutralizing activity (IC90) of approximately 2 µg/mL (approximately 13.3 nM) or lower against all of human metapneumovirus strains JPS03-180 (type A1), JPS03-178 (type A2), JPS02-76 (type B1), and JPS05-21 (type B2).
[36] The antibody or an antigen-binding fragment thereof according to any one of [1] to [32], wherein the antibody has a neutralizing activity (IC50) of approximately 0.1 µg/mL (approximately 0.67 nM) or lower against a human metapneumovirus JPS03-178 strain (type A2).
[37] A pharmaceutical composition comprising an antibody or an antigen-binding fragment thereof according to any one of [1] to [36] and a pharmaceutically acceptable carrier.
[38] The pharmaceutical composition according to [37], wherein the pharmaceutical composition is intended for the treatment or prevention of human metapneumovirus infection.
[39] An isolated nucleic acid encoding the amino acid sequence of an antibody or an antigen-binding fragment thereof according to any one of [1] to [36], an isolated nucleic acid encoding an amino acid sequence of any of SEQ ID NOs: 1, 6, 11, 16, 21, 26, 31, 36, 41, 46, 51, 56, 61, 66, 71, 76, 81, 86, 91, 96, 101, 106, 111, 116, 121, and 126, or an isolated nucleic acid hybridizing under high stringent conditions to any of these nucleic acids.
[40] A recombinant expression vector comprising an isolated nucleic acid according to [39] incorporated therein.
[41] A host cell comprising a recombinant expression vector according to [40] introduced thereinto.

### ADVANTAGEOUS EFFECTS OF INVENTION

The monoclonal antibody neutralizing the biological activity of human metapneumovirus (hMPV) according to the present invention or an antigen-binding fragment thereof specifically binds to human metapneumovirus (hMPV) F protein and cancels (neutralizes) its biological activity. Thus, the monoclonal antibody according to the present invention or an antigen-binding fragment thereof can be expected to prevent or treat hMPV infection. In an embodiment, the monoclonal antibody according to the present invention or an antigen-binding fragment thereof is a human monoclonal antibody against human metapneumovirus (hMPV) F protein or an antigen-binding fragment thereof and therefore, is advantageously neither immunogenic nor causes immune response. A pharmaceutical composition comprising the particularly preferred human monoclonal antibody according to the present invention is effective in a very small amount and can also be expected to reduce medical cost.

### BRIEF DESCRIPTION OF DRAWINGS

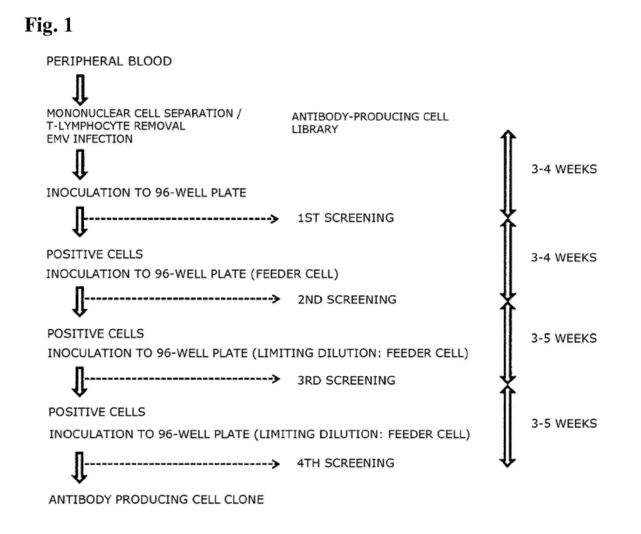

[Figure 1] Figure 1 is a diagram showing, in the form of flow chart, procedures of separating an antibody-producing cell clone producing the anti-hMPV antibody according to the present invention.

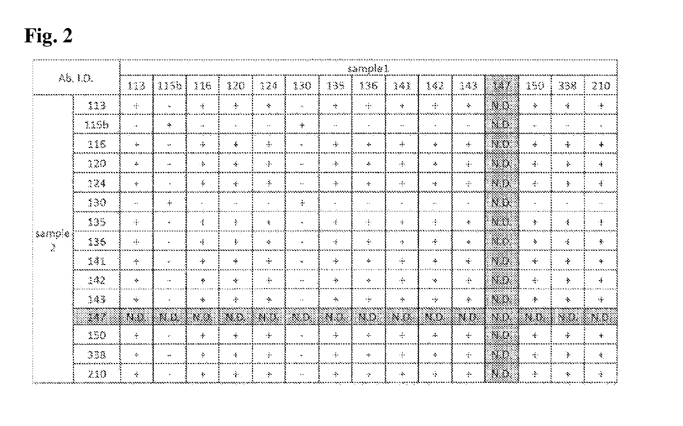

[Figure 2] Figure 2 is a diagram showing the results of conducting the competition assay of anti-MPV antibodies using Biacore T-200. FP-TM(-)-His, which was His-Tag-added hMPV (JPS02-76) FP lacking the TM region (a.a. 491-539), was captured onto Sensor Chip NTA. An anti-MPV antibody was bound as Sample 1 to the sensor chip until a saturation level. Next, another anti-MPV antibody was bound thereto as Sample 2. The presence or absence of competition was determined on the basis of whether or not the binding of the anti-hMPV antibody as Sample 2 was inhibited. Ab. I.D. was indicated by the last 3 digits. The mark + represents competing, and the mark - represents not competing. N.D. represents being immeasurable due to the aggregation reaction of antibodies on the sensor chip. In the diagram, Ab. I.D. 338 and 210 represent mAb388 and HMB3210, respectively, used as controls.

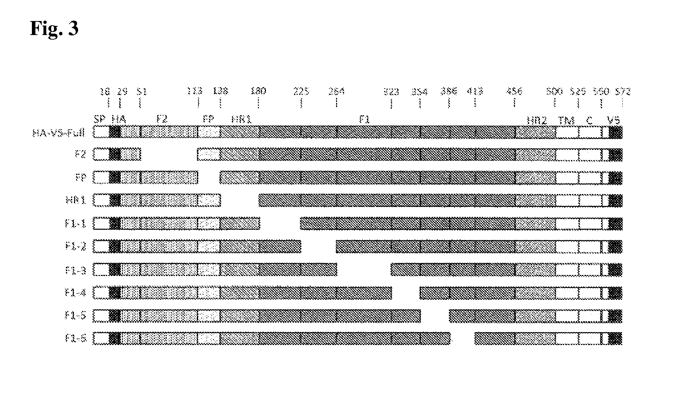

[Figure 3] Figure 3 is a diagram showing various deletion mutants (hMPV-FP deletion mutants) prepared for the epitope mapping of anti-hMPV antibodies. HA-V5-Full is hMPV (JPS02-76) FP containing HA-Tag (HA) added downstream of the signal peptide (SP) and V5-Tag (V5) added downstream of the C-tail (C). Each deletion mutant was deficient in the region shown in the diagram and cloned into an expression vector. Other abbreviations in the diagram are as follows: F2 domain (F2), fusion peptide (FP), heptad repeat 1 (HR1), F1 domain (F1), heptad repeat 2 (HR2), and transmembrane domain (TM).

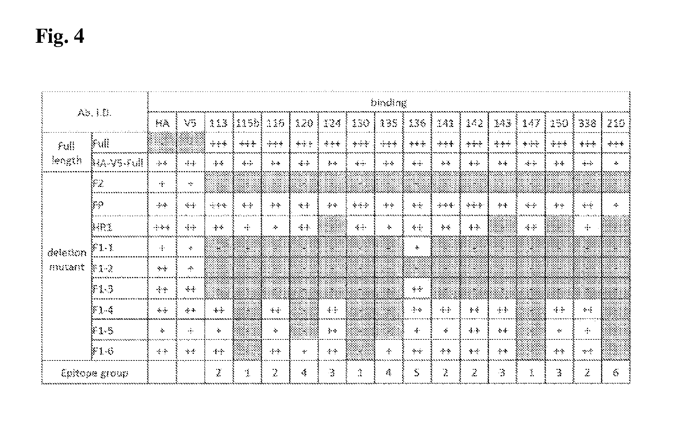

[Figure 4] Figure 4 is a diagram the showing results of the binding assay of anti-hMPV antibodies to deletion mutants. CHO-K1 cells were transfected with each deletion mutant expression vector shown in Figure 3, inoculated to a 96-well plate 16 hours later, and fixed 24 hours later. The resulting deletion mutant-expressing CHO-K1 cells were fluorescently immunostained to confirm the binding of each anti-MPV antibody. The details of this assay are similar to those of cell fluorescent immunostaining screening. Also, HA and V5 antibodies were subjected to cell fluorescent immunostaining to confirm the expression of the deletion mutants themselves. Ab. I.D. was indicated by the last 3 digits. The marks +++, ++, and + represent binding in the order of fluorescence intensity. The mark - represents not binding. Epitope group was determined on the basis of binding patterns to the deletion mutants. In the diagram, Ab. I.D. 338 and 210 represent mAb388 and HMB3210, respectively, used as controls.

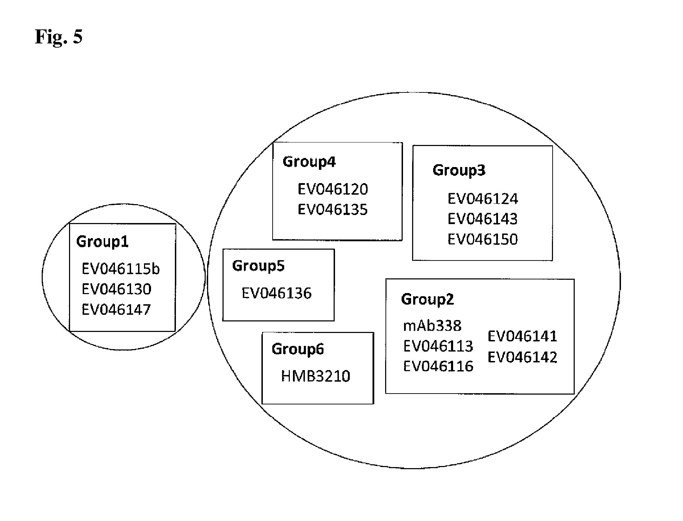

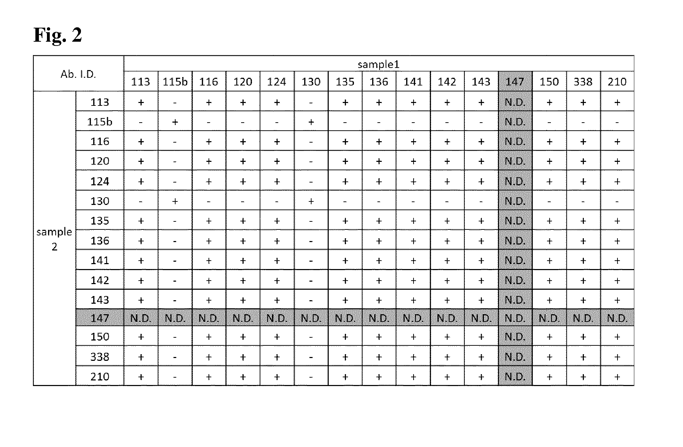

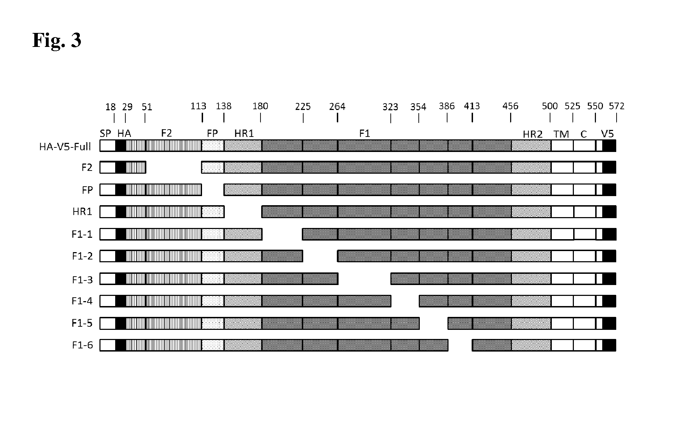

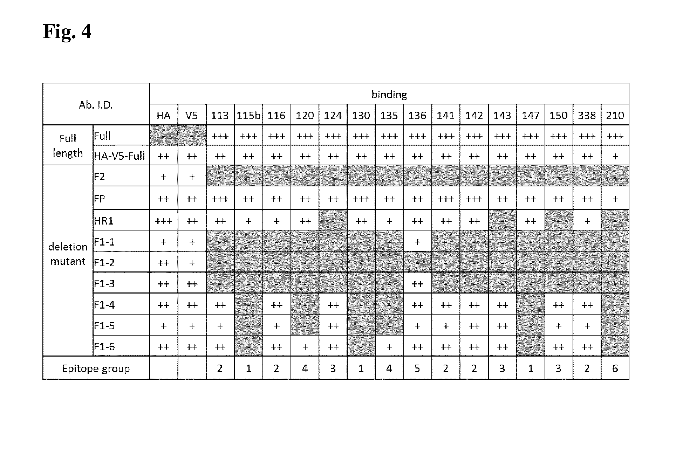

[Figure 5] Figure 5 is a diagram showing the epitope grouping of anti-hMPV antibodies. The anti-hMPV antibodies were epitope-grouped on the basis of the results shown in Figures 3 and 4. The circled groups are based on the competition assay results, and the boxed groups are based on the deletion mutant binding assay results.

### DESCRIPTION OF EMBODIMENTS

### 1. Description of terms

In the present specification, scientific terms and technical terms used in relation to the present invention have meanings generally understood by those skilled in the art. Words in the singular form shall be construed to include the plural and vice versa, unless the context otherwise requires. In general, nomenclatures used in relation to cells and techniques of tissue culture, molecular biology, immunology, microbiology, genetics, protein and nucleic acid chemistries, and hybridization described herein are well known in the art and generally used.

The present invention relates to a monoclonal antibody specifically binding to human metapneumovirus F protein and neutralizing the biological activity of the human metapneumovirus, and an antigen-binding fragment thereof. Hereinafter, the embodiments of the present invention will be described in detail by defining the terms used in the present invention.

### 1) Human metapneumovirus (also abbreviated to hMPV)

The human metapneumovirus is one of the causative viruses of human respiratory infection that was discovered by a Dutch research group in 2001. This virus is classified into the genus *Metapneumovirus* within the subfamily *Paramyxoviridae* of the family *Paramyxoviridae* and, currently, further classified into four subtypes (A1, A2, B1, and B2; also referred to as type A1, type A2, type B1, and type B2, respectively) on the basis of results of phylogenetic analysis using the nucleotide sequences of G and F proteins (Non Patent Literature 4). The subgroup A2 was further classified into two types, hMPV_A2a and hMPV_A2b, in Germany in 2006 (Non Patent Literature 5). Analogous viruses are avian pneumovirus (also abbreviated to APV), which infects bird, human respiratory syncytial virus (also abbreviated to RSV), and the like. hMPV, a 13.35-kb single-stranded (negative strand) RNA virus, has eight genes, N, P, M, F, M2, SH, G, and L, and express three glycoproteins, F, G, and SH on the virus surface ((Non Patent Literature 1). Reportedly, an antibody against the F protein accounts for a major proportion of neutralizing antibodies against hMPV present in serum.

### 2) hMPV F protein

The F protein (hereinafter, also abbreviated to FP) is one of the glycoproteins present on the virus surface of hMPV. The F proteins in typical strains of four hMPV subgroups (A1, A2, B1, and B2) are each composed of 539 amino acid residues. Their typical amino acid sequences are disclosed in GenBank Accession Nos. AF371337 for A1 (NL/1/00 strain), AY304360 for A2 (NL/17/00 strain), AY304361 for B1 (NL/1/99 strain), and AY304362 for B2 (NL/1/94 strain). The amino acid sequence of the A1 F protein, as compared with other viruses, has 81% identity to the amino acid sequence of the F protein of avian pneumovirus type C and 33% identity to the amino acid sequence of the F protein of human RSV (Non Patent Literature 3), suggesting that the hMPV F protein plays the same role as that of the F proteins of these viruses. Specifically, the hMPV F protein is considered to be a fusion protein that is involved in the fusion between the envelope of the virus attached to a cell and the membrane of the host cell. Thus, it has been reported that antibodies having high neutralizing activity are found among antibodies binding to the hMPV F protein (Patent Literatures 1 and 2). Among antibodies against human RSV F protein, an antibody (Synagis) has already been used as a drug.

When the amino acid sequence of the F protein is compared among the four hMPV subgroups, the amino acid sequence of each subgroup (A1, A2, B1, and B2) has 99 to 100% identity to each other and has 97 to 99% identity between the subtypes (between A1 and A2 or between B1 and B2). When the amino acid sequence is compared between groups A and B, 94 to 97% identity is confirmed. Thus, the amino acid sequence of the hMPV F protein is known to be highly conserved among the groups (Non Patent Literature 4). In the present invention, the amino acid sequence of the F protein of a JPS02-76 strain (B1; T285I) used in the screening evaluation of human antibodies exhibited identity as very high as 94% to the amino acid sequence of the A1 (NL/1/00 strain) F protein, 94% to the amino acid sequence of the A2 (NL/17/00 strain) F protein, 99% to the B1 (NL/1/99 strain) F protein, and 99% to the B2 (NL/1/94 strain) F protein, as compared with the typical F proteins mentioned above. This strongly suggests that the evaluation system using the F protein of the JPS02-76 strain used in the present invention is a method capable of obtaining antibodies cross-reactive with the hMPV strain of any subgroup.

### 3) Antibody binding to hMPV F protein

This antibody specifically binds to human metapneumovirus (hMPV) FP and is capable of binding to an epitope site, for example, a linear epitope, a discontinuous epitope, or a conformational epitope, in the FP, or a fragment of the FP, etc. In the present specification, the "anti-hMPV antibody", the "antibody capable of neutralizing hMPV", the "anti-hMPV-F protein antibody", the "antibody specifically binding to human metapneumovirus (hMPV) FP", or the "antibody capable of neutralizing the biological activity of human metapneumovirus (hMPV)" refers to an antibody that inhibits the biological activity of the human metapneumovirus (hMPV) through binding to the hMPV FP. As mentioned above, since the identity of FP is very high among the hMPV subtypes, the antibody specifically binding to hMPV FP is generally screened for by a method using FP of any of the typical strains of four hMPV subgroups (A1, A2, B1, and B2) as an antigen. In the present invention, the FP gene was cloned from the hMPV B1 strain (JPS02-76) and expressed in CHO-K1 cells, and the resulting FP was used. The nucleotide sequence of the obtained FP gene (GenBank Accession No. AY530089) and the amino acid sequence of FP (GenBank Accession No. AAS22077) are shown in SEQ ID NOs: 131 and 132, respectively.

### 4) Antibody

The term "antibody" used herein refers to an immunoglobulin molecule composed of four polypeptide chains, i.e., two heavy (H) chains and two light (L) chains, linked via disulfide bonds. The monoclonal antibody according to the present invention is also composed of an immunoglobulin molecule comprising two heavy chains (H chains) and two light chains (L chains). Each H chain is composed of an H chain variable part region (also referred to as "HCVR" or "VH") and an H chain invariable part region (the H chain invariable part region is composed of three domains, which are also referred to as "CH1", "CH2", and "CH3", respectively (collectively referred to as CH)). Each L chain is composed of an L chain variable part region (also referred to as "LCVR" or "VL") and an L chain invariable part region (the L chain invariable part region is composed of one domain, which is also referred to as "CL"). A region located before each invariable part region (also referred to as invariable region or constant region) is called a variable part region (also referred to as variable region).

Particularly, VH and VL are important because of their involvement in the binding specificity of the antibody. Since the antibody interacts with its target antigen mainly through the amino acid residues of VH and VL, the amino acid sequences within the variable part regions differ more largely among individual antibodies than sequences without the variable part regions. VH and VL can be further subdivided into regions called framework regions (FRs) conserved among various antibodies, and hypervariable regions called complementarity-determining regions (CDRs). Each of VH and VL has three CDRs and four FRs, and these regions are arranged from the amino terminus to the carboxy terminus in the order of FR1, CDR1, FR2, CDR2, FR3, CDR3, and FR4.

The antibody according to the present invention means a whole antibody or an antigen-binding fragment thereof. On the basis of the amino acid sequence representing each variable part region or the amino acid sequence of each complementarity-determining region (CDR), human monoclonal antibodies and monoclonal antibodies (also including chimeric antibodies and humanized antibodies) each capable of specifically binding to hMPV F protein and neutralizing the biological activity of the hMPV, or antigen-binding fragments thereof can be obtained by a technique well known in the art. These antibodies are included in the technical scope of the present invention.

### 5) "Antigen-binding fragment" of the antibody (or simply referred to as "antibody fragment")

The term "antigen-binding fragment" of the antibody (or simply referred to as an "antibody fragment") used herein refers to one or more antibody fragments (e.g., VH) having the ability to specifically bind to the antigen (F proteins of hMPV). The fragment also includes a peptide having the minimum amino acid sequence specifically binding to the antigen. Examples of binding moieties included in the term "antigen-binding fragment" of the antibody include (i) a Fab fragment, (ii) a F(ab')2 fragment, (iii) an Fd fragment composed of VH and CH1 domains, (iv) an Fv fragment composed of VL and VH domains on a single arm of the antibody, (v) a dAb fragment composed of a VH domain (Ward et al., Nature 341: 544-546, 1989), (vi) isolated complementarity-determining regions (CDRs) having frameworks sufficient for the specific binding, (vii) a bispecific antibody, and (viii) a multispecific antibody. In the present specification, the simple term "antibody" used without discrimination includes not only whole antibodies but also such "antigen-binding fragments".

### 6) Isotype and subclass

Heavy chains are divided according to difference in constant region into y, µ, α, δ, and ε chains. Depending on this difference, 5 types of classes (isotypes), IgG, IgM, IgA, IgD, and IgE, respectively, are formed in immunoglobulins. In the case of human IgG, four subclasses, IgG1 to IgG4, are present. On the other hand, light chains are divided according to difference in constant region into κ and λ chains. The antibody according to the present invention is particularly preferably of IgG1 (κ) or IgG1 (λ) class (subclass).

### 2. Antibody according to the present invention or antigen-binding fragment thereof

In one embodiment, the present invention provides an antibody capable of specifically binding to hMPV and neutralizing the biological activity of the hMPV, or an antigen-binding fragment thereof (hereinafter, referred to as the antibody of the present invention). Typically, the antibody of the present invention is an antibody capable of specifically binding to hMPV F protein and neutralizing the biological activity of the protein, or an antigen-binding fragment thereof.

The anti-hMPV antibody of the present invention includes not only an antibody comprising the amino acid sequence of a particular heavy chain or light chain, its variable region, or its CDRs, etc., shown in SEQ ID NO described in the present specification, but an anti-hMPV antibody comprising an amino acid sequence substantially identical to any of these amino acid sequences and capable of specifically binding to hMPV F protein and neutralizing the biological activity of the hMPV. In this context, the term "substantially identical" means the presence of the deletion, substitution, insertion, or addition of one or more amino acid residues in the amino acid sequence of the protein of the present invention, or the combination of any two or more of these means that one or more amino acid residues are deleted, substituted, inserted, or added at one or more arbitrary positions in the same amino acid sequence, and two or more of the deletion, the substitution, the insertion, and the addition may occur at the same time.

Amino acids constituting proteins in the natural world can be grouped according to the properties of their side chains and can be classified into amino acid groups having similar properties, for example, groups of aromatic amino acids (tyrosine, phenylalanine, and tryptophan), basic amino acids (lysine, arginine, and histidine), acidic amino acids (aspartic acid and glutamic acid), neutral amino acids (serine, threonine, asparagine, and glutamine), hydrocarbon chain-containing amino acids (alanine, valine, leucine, isoleucine, and proline), and others (glycine, methionine, and cysteine).

As an example, amino acid residues that can be mutually substituted, also including nonnatural amino acids, may be grouped as follows, and the amino acid residues included in the same group can be mutually substituted: group A: leucine, isoleucine, norleucine, valine, norvaline, alanine, 2-aminobutanoic acid, methionine, o-methylserine, t-butylglycine, t-butylalanine, and cyclohexylalanine; group B: aspartic acid, glutamic acid, isoaspartic acid, isoglutamic acid, 2-aminoadipic acid, and 2-aminosuberic acid; group C: asparagine and glutamine; group D: lysine, arginine, ornithine, 2,4-diaminobutanoic acid, and 2,3-diaminopropionic acid; group E: proline, 3-hydroxyproline, and 4-hydroxyproline; group F: serine, threonine, and homoserine; and group G: phenylalanine, tyrosine, and tryptophan.

The identity of an amino acid sequence or a nucleotide sequence can be determined using the Karlin-Altschul algorithm BLAST (PNAS, 1990 (vol.87) p2264 ; PNAS, 1993 (vol.90) p5873). A program called BLASTN or BLASTX based on the BLAST algorithm has been developed (Altschul SF, et al., J Mol Biol, 1990 (vol.215) p403). In the case of analyzing a nucleotide sequence using BLASTN, parameters are set to, for example, score = 100 and wordlength = 12. In the case of analyzing an amino acid sequence using BLASTX, parameters are set to, for example, score = 50 and wordlength = 3. In the case of using the BLAST and Gapped BLAST programs, default parameters of each program are used. Alternatively, the identity between the amino acid sequences of proteins can also be determined according to the expression "(The number of identical amino acid residues / The number of amino acid residues in the whole protein) x 100 (%)" by aligning the amino acid sequences of the two types of proteins to be compared and counting the number of the same amino acid residues between them.

The correspondence between the human-derived anti-hMPV antibodies found in the present invention and an antigen-binding fragment thereof and SEQ ID NOs of their amino acid sequences in the Sequence Listing is shown in Table 1.

**[Table 1] List of SEQ ID NOs of amino acid sequences related to various human anti-hMPV antibodies**

| | Antibody number | H chain/ L chain | Full-length aa sequence | Variable region aa sequence | CDR aa sequence | | |
|---|---|---|---|---|---|---|---|
| | | | | | CDR-1 | CDR-2 | CDR-3 |
| 01 | EV046113 | H chain | 1 | 2 | 3 | 4 | 5 |
| | | L chain | 6 | 7 | 8 | 9 | 10 |
| 02 | EV046115b | H chain | 11 | 12 | 13 | 14 | 15 |
| | | L chain | 16 | 17 | 18 | 19 | 20 |
| 03 | EV046116 | H chain | 21 | 22 | 23 | 24 | 25 |
| | | L chain | 26 | 27 | 28 | 29 | 30 |
| 04 | EV046120 | H chain | 31 | 322 | 33 | 34 | 35 |
| | | L chain | 36 | 37 | 38 | 39 | 40 |
| 05 | EV046124 | H chain | 41 | 42 | 43 | 44 | 45 |
| | | L chain | 46 | 47 | 48 | 49 | 50 |
| 06 | EV046130 | H chain | 51 | 52 | 53 | 54 | 55 |
| | | Lchain | 56 | 57 | 58 | 59 | 60 |
| 07 | EV046135 | Hchain | 61 | 62 | 63 | 64 | 65 |
| | | Lchain | 66 | 67 | 68 | 69 | 70 |
| 08 | EV046136 | Hchain | 71 | 72 | 73 | 74 | 75 |
| | | Lchain | 76 | 77 | 78 | 79 | 80 |
| 09 | EV046141 | Hchain | 81 | 82 | 83 | 84 | 85 |
| | | Lchain | 86 | 87 | 88 | 89 | 90 |
| 10 | EV046142 | Hchain | 91 | 92 | 93 | 94 | 95 |
| | | Lchain | 96 | 97 | 98 | 99 | 100 |
| 11 | EV046143 | Hchain | 101 | 102 | 103 | 104 | 105 |
| | | Lchain | 106 | 107 | 108 | 109 | 110 |
| 12 | EV046147 | Hchain | 111 | 112 | 113 | 114 | 115 |
| | | Lchain | 116 | 117 | 118 | 119 | 120 |
| 13 | EV046150 | Hchain | 121 | 122 | 123 | 124 | 125 |
| | | Lchain | 126 | 127 | 128 | 129 | 130 |

| | | | | | | | |
|---|---|---|---|---|---|---|---|
| (In the table, the number shown in each cell represents each SEQ ID NO.) | | | | | | | |

Among these obtained antibodies, EV046143 (VH: SEQ ID NO: 102, VL: SEQ ID NO: 107) and EV046150 (VH: SEQ ID NO: 122, VL: SEQ ID NO: 127), whose epitope belonged to the same epitope group and which were very analogous in terms of neutralizing activity and CDR sequences, were analyzed for their sequence identity and consequently found to have approximately 73% identity in the H chain variable regions (SEQ ID NOs: 102 and 122). Approximately 82% identity was confirmed in the amino acid sequences of their L chain variable regions (SEQ ID NOs: 107 and 127). As for the FR regions (FR1 + FR2 + FR3 + FR4) of each chain, 83% identity was confirmed for VH, and approximately 89% identity was confirmed for VL. As for the amino acid sequences of CDRs, 60% (3/5) identity was confirmed in the H chain CDR1 amino acid sequences (SEQ ID NOs: 103 and 123). Approximately 53% (9/17) identity was confirmed in the H chain CDR2 amino acid sequences (SEQ ID NOs: 104 and 124). When the identity was compared between the H chain CDR3 amino acid sequences (SEQ ID NOs: 105 and 125), 37.5% (6/16) identity was observed. In summary, approximately 46% (18/38) identity was confirmed for the whole CDRs of the H chains. On the other hand, when the L chain CDR1 amino acid sequences (SEQ ID NOs: 108 and 128) were compared, approximately 42% (5/12) identity was confirmed. Approximately 87% (6/7) identity was confirmed in the L chain CDR2 amino acid sequences (SEQ ID NOs: 109 and 129). When the L chain CDR3 amino acid sequences (SEQ ID NOs: 120 and 130) were further compared, 75% (6/8) identity was confirmed. In summary, approximately 63% (17/72) identity was confirmed for the whole CDRs of the L chains. Considering these factors together, an antibody having 50% or higher identity to any of the 13 antibodies mentioned above in terms of the whole CDR sequences, or a fragment thereof probably has binding activity and hMPV-neutralizing activity similar to those of the 13 antibodies. Furthermore, an antibody having 60% or higher (preferably 70% or higher, 80% or higher, 90% or higher, 95% or higher, 96% or higher, 97% or higher, 98% or higher, or 99% or higher) identity thereto, in other words, an antibody comprising an amino acid sequence having the deletion, substitution, insertion, or addition of one to several (specifically, 1 to 15, 1 to 10, 1 to 9, 1 to 8, 1 to 7, 1 to 6, 1 to 5, 1 to 4, 1 to 3, 1 to 2, or 1) amino acid residues in the whole three CDR sequences (CDR1, CDR2, and CDR3) of each chain, or the combination of two or more of these mutations, probably has binding activity and neutralizing activity similar to those of the 13 anti-hMPV antibodies.

Thus, the antibody of the present invention or an antigen-binding fragment is preferably an antibody having heavy chain CDR1, CDR2, and CDR3 and light chain CDR1, CDR2, and CDR3 that respectively correspond to 6 amino acid sequences in one combination selected from the combination groups of, for example, SEQ ID NOs: 3 to 5 and 8 to 10, 13 to 15 and 18 to 20, 23 to 25 and 28 to 30, 33 to 35 and 38 to 40, 43 to 45 and 48 to 50, 53 to 55 and 58 to 60, 63 to 65 and 68 to 70, 73 to 75 and 78 to 80, 83 to 85 and 88 to 90, 93 to 95 and 98 to 100, 103 to 105 and 108 to 110, 113 to 115 and 118 to 120, and 123 to 125 and 128 to 130, or an antigen-binding fragment thereof. The CDR sequences, however, may have amino acid sequences derived from the 6 amino acid sequences in one combination selected from the combination groups of SEQ ID NOs: 3 to 5 and 8 to 10, 13 to 15 and 18 to 20, 23 to 25 and 28 to 30, 33 to 35 and 38 to 40, 43 to 45 and 48 to 50, 53 to 55 and 58 to 60, 63 to 65 and 68 to 70, 73 to 75 and 78 to 80, 83 to 85 and 88 to 90, 93 to 95 and 98 to 100, 103 to 105 and 108 to 110, 113 to 115 and 118 to 120, and 123 to 125 and 128 to 130, by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues or 50% or less (preferably 40%, 30%, 20%, or 10% or less) of the number of amino acid residues in the CDR sequences, or the combination of two or more of these mutations, as long as the resulting monoclonal antibody is capable of specifically binding to hMPV F protein and neutralizing its biological activity. Its amino acid sequence except for the CDRs is not particularly limited, and a so-called CDR-grafted antibody having an amino acid sequence, except for CDRs, derived from another antibody, particularly, an antibody of different species is also encompassed by the antibody of the present invention. Of these antibodies, an antibody is more preferred in which the amino acid sequence other than CDRs is also derived from a human. If necessary, its framework regions (FRs) may have the deletion, substitution, insertion, or addition of one to several (specifically, 1 to 30, 1 to 25, 1 to 20, 1 to 15, 1 to 10, 1 to 9, 1 to 8, 1 to 7, 1 to 6, 1 to 5, 1 to 4, 1 to 3, 1 to 2, or 1) amino acid residues, or the combination of two or more of these mutations.

In some cases, the combination of the 6 CDR sequences contained in the antibody of the present invention may be a combination in which 5 to 1 of the 6 amino acid sequences in one combination selected from the combination groups of SEQ ID NOs: 3 to 5 and 8 to 10, 13 to 15 and 18 to 20, 23 to 25 and 28 to 30, 33 to 35 and 38 to 40, 43 to 45 and 48 to 50, 53 to 55 and 58 to 60, 63 to 65 and 68 to 70, 73 to 75 and 78 to 80, 83 to 85 and 88 to 90, 93 to 95 and 98 to 100, 103 to 105 and 108 to 110, 113 to 115 and 118 to 120, and 123 to 125 and 128 to 130 are replaced with corresponding amino acid sequences in any of the other combinations of the 6 amino acid sequences (e.g., in heavy chain CDR1, CDR2, and CDR3 and light chain CDR1, CDR2, and CDR3 represented by SEQ ID NOs: 3, 4, and 5 and 8, 9, and 10, respectively, heavy chain CDR2 (SEQ ID NO: 4) and CDR3 (SEQ ID NO: 5) are replaced with heavy chain CDR2 (SEQ ID NO: 14) and CDR3 (SEQ ID NO: 15), respectively, in the combination of SEQ ID NOs: 13 to 15 and 18 to 20), as long as the resulting monoclonal antibody is capable of specifically binding to hMPV F protein and neutralizing its biological activity. Such an antibody is also included in the scope of the present invention.

In a preferred alternative embodiment, the anti-hMPV antibody according to the present invention is an antibody containing (a) a heavy chain variable region (VH) selected from the group consisting of; the amino acid sequences of SEQ ID NOs: 2, 12, 22, 32, 42, 52, 62, 72, 82, 92, 102, 112, and 122; amino acid sequences derived from any of the amino acid sequences of SEQ ID NOs: 2, 12, 22, 32, 42, 52, 62, 72, 82, 92, 102, 112, and 122 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; and amino acid sequences having 60% or higher (preferably 70% or higher, 80% or higher, 90% or higher, 95% or higher, 96% or higher, 97%, 98% or higher, or 99% or higher) identity to any of the amino acid sequences of SEQ ID NOs: 2, 12, 22, 32, 42, 52, 62, 72, 82, 92, 102, 112, and 122, and (b) a light chain variable region (VL) selected from the group consisting of: the amino acid sequences of SEQ ID NOs: 7, 17, 27, 37, 47, 57, 67, 77, 87, 97, 107, 117, and 127; amino acid sequences derived from any of the amino acid sequences of SEQ ID NOs: 7, 17, 27, 37, 47, 57, 67, 77, 87, 97, 107, 117, and 127 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; and amino acid sequences having 60% or higher (preferably 70% or higher, 80% or higher, 90% or higher, 95% or higher, 96% or higher, 97%, 98% or higher, or 99% or higher) identity to any of the amino acid sequences of SEQ ID NOs: 7, 17, 27, 37, 47, 57, 67, 77, 87, 97, 107, 117, and 127, or an antigen-binding fragment thereof.

In a further preferred alternative embodiment, the anti-hMPV antibody according to the present invention contains (a-1) a heavy chain variable region (VH) represented by the amino acid sequence of SEQ ID NO: 2; an amino acid sequence derived from the amino acid sequence of SEQ ID NO: 2 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 2, and (b-1) a light chain variable region (VL) represented by the amino acid sequence of SEQ ID NO: 7; an amino acid sequence derived from the amino acid sequence of SEQ ID NO: 7 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 7.

In a preferred alternative embodiment, the anti-hMPV antibody contains (a-2) a heavy chain variable region (VH) represented by the amino acid sequence of SEQ ID NO: 12; an amino acid sequence derived from the amino acid sequence of SEQ ID NO: 12 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 12, and (b-2) a light chain variable region (VL) represented by the amino acid sequence of SEQ ID NO: 17; an amino acid sequence derived from the amino acid sequence of SEQ ID NO: 17 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 17.

In a preferred alternative embodiment, the anti-hMPV antibody contains (a-3) a heavy chain variable region (VH) represented by the amino acid sequence of SEQ ID NO: 22; an amino acid sequence derived from the amino acid sequence of SEQ ID NO: 22 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 22, and (b-3) a light chain variable region (VL) represented by the amino acid sequence of SEQ ID NO: 27; an amino acid sequence derived from the amino acid sequence of SEQ ID NO: 27 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 27.

In a preferred alternative embodiment, the anti-hMPV antibody according to the present invention contains (a-4) a heavy chain variable region (VH) represented by the amino acid sequence of SEQ ID NO: 33; an amino acid sequence derived from the amino acid sequence of SEQ ID NO: 33 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 33, and (b-4) a light chain variable region (VL) represented by the amino acid sequence of SEQ ID NO: 38; an amino acid sequence derived from the amino acid sequence of SEQ ID NO: 38 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 38.

In a preferred alternative embodiment, the anti-hMPV antibody contains (a-5) a heavy chain variable region (VH) represented by the amino acid sequence of SEQ ID NO: 43; an amino acid sequence derived from the amino acid sequence of SEQ ID NO: 43 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 43, and (b-5) a light chain variable region (VL) represented by the amino acid sequence of SEQ ID NO: 47; an amino acid sequence derived from the amino acid sequence of SEQ ID NO: 47 by the deletion, substitution, insertion, or addition of one to several (specific numbers are the same as above) amino acid residues, or the combination of two or more of these mutations; or an amino acid sequence having 60% or higher
(specific % is the same as above) identity to the amino acid sequence of SEQ ID NO: 47.

In a preferred alternative embodiment, the anti-hMPV antibody contains (a-6) a heavy chain variable region (VH) represented by the amino acid sequence of SEQ ID NO: 52; an amino acid sequence derived from the amino acid sequence of SEQ ID NO: 57 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 57, and (b-6) a light chain variable region (VL) represented by the amino acid sequence of SEQ ID NO: 62; an amino acid sequence derived from the amino acid sequence of SEQ ID NO: 62 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 62.

In a preferred alternative embodiment, the anti-hMPV antibody according to the present invention contains (a-7) a heavy chain variable region (VH) represented by the amino acid sequence of SEQ ID NO: 62; an amino acid sequence derived from the amino acid sequence of SEQ ID NO: 62 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 62, and (b-7) a light chain variable region (VL) represented by the amino acid sequence of SEQ ID NO: 67; an amino acid sequence derived from the amino acid sequence of SEQ ID NO: 67 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 67.

In a preferred alternative embodiment, the anti-hMPV antibody contains (a-8) a heavy chain variable region (VH) represented by the amino acid sequence of SEQ ID NO: 72; an amino acid sequence derived from the amino acid sequence of SEQ ID NO: 72 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 72, and (b-8) a light chain variable region (VL) represented by the amino acid sequence of SEQ ID NO: 77; an amino acid sequence derived from the amino acid sequence of SEQ ID NO: 77 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 77.

In a preferred alternative embodiment, the anti-hMPV antibody contains (a-9) a heavy chain variable region (VH) represented by the amino acid sequence of SEQ ID NO: 82; an amino acid sequence derived from the amino acid sequence of SEQ ID NO: 82 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 82, and (b-9) a light chain variable region (VL) represented by the amino acid sequence of SEQ ID NO: 87; an amino acid sequence derived from the amino acid sequence of SEQ ID NO: 87 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 87.

In a preferred alternative embodiment, the anti-hMPV antibody according to the present invention contains (a-10) a heavy chain variable region (VH) represented by the amino acid sequence of SEQ ID NO: 92; an amino acid sequence derived from the amino acid sequence of SEQ ID NO: 92 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 92, and (b-10) a light chain variable region (VL) represented by the amino acid sequence of SEQ ID NO: 97; an amino acid sequence derived from the amino acid sequence of SEQ ID NO: 97 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 97.

In a preferred alternative embodiment, the anti-hMPV antibody contains (a-11) a heavy chain variable region (VH) represented by the amino acid sequence of SEQ ID NO: 102; an amino acid sequence derived from the amino acid sequence of SEQ ID NO: 102 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 102, and (b-11) a light chain variable region (VL) represented by the amino acid sequence of SEQ ID NO: 107; an amino acid sequence derived from the amino acid sequence of SEQ ID NO: 107 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 107.

In a preferred alternative embodiment, the anti-hMPV antibody contains (a-12) a heavy chain variable region (VH) represented by the amino acid sequence of SEQ ID NO: 112; an amino acid sequence derived from the amino acid sequence of SEQ ID NO: 112 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 112, and (b-12) a light chain variable region (VL) represented by the amino acid sequence of SEQ ID NO: 117; an amino acid sequence derived from the amino acid sequence of SEQ ID NO: 117 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 117.

In a preferred alternative embodiment, the anti-hMPV antibody contains (a-13) a heavy chain variable region (VH) represented by the amino acid sequence of SEQ ID NO: 122; an amino acid sequence derived from the amino acid sequence of SEQ ID NO: 122 by the deletion, substitution, insertion, or addition of one to several (specifically, 1 to 9, 1 to 8, 1 to 7, 1 to 6, 1 to 5, 1 to 4, 1 to 3, 1 to 2, or 1) amino acid residues, or the combination of two or more of these mutations; or an amino acid sequence having 60% or higher (preferably 70% or higher, 80% or higher, 90% or higher, 95% or higher, 96% or higher, 97% or higher, 98% or higher, or 99% or higher) identity to the amino acid sequence of SEQ ID NO: 122, and (b-13) a light chain variable region (VL) represented by the amino acid sequence of SEQ ID NO: 127; an amino acid sequence derived from the amino acid sequence of SEQ ID NO: 127 by the deletion, substitution, insertion, or addition of one to several (specific numbers are the same as above) amino acid residues, or the combination of two or more of these mutations; or an amino acid sequence having 60% or higher (specific % is the same as above) identity to the amino acid sequence of SEQ ID NO: 127.

A method known in the art can be used as a method for preparing these antibodies (Riechmann L, et al., Reshaping human antibodies for therapy. Nature, 332:323-327, 1988). In the present invention, a completely human antibody is preferred, as a matter of course.

### 3. Nucleic acid encoding antibody according to the present invention, etc.

In another embodiment, the present invention provides a nucleic acid (nucleotide) encoding the anti-hMPV antibody capable of specifically binding to hMPV F protein and neutralizing its biological activity, or an antigen-binding fragment thereof. A nucleic acid encoding an amino acid sequence selected from the group consisting of SEQ ID NOs: 4 to 15, 18 to 29, and 32 to 43, and an isolated nucleic acid having high identity to the nucleic acid are also included in the scope of the present invention. In this context, the term "having high identity" means sequence identity to an extent that permits hybridization under high stringent conditions to the predetermined nucleic acid sequence and means having, for example, 60%, 70%, 80%, 90%, or 95% or higher identity. The present invention provides an isolated nucleic acid selected from nucleic acids hybridizing thereto under high stringent conditions. Preferably, the nucleic acid is DNA or RNA, more preferably DNA.

The "high stringent conditions" are conditions involving, for example, 5 x SSC, 5 x Denhardt's solution, 0.5% SDS, 50% formamide, and 50°C. (See e.g., J. Sambrook et al., Molecular Cloning, A Laboratory Manual 2nd ed., Cold Spring Harbor Laboratory Press (1989), particularly, 11.45 "Conditions for Hybridization of Oligonucleotide Probes"). Under these conditions, it can be expected that a polynucleotide (e.g., DNA) having high identity is more efficiently obtained at a higher temperature. However, possible factors that influence the stringency of hybridization are a plurality of factors such as temperatures, probe concentrations, probe lengths, ionic strengths, times, and salt concentrations. Those skilled in the art can achieve similar stringency by appropriately selecting these factors.

The nucleic acid hybridizing under high stringent conditions includes a nucleic acid having, for example, 70% or higher, 80% or higher, 90% or higher, 95% or higher, 97% or higher, or 99% or higher identity to the nucleic acid encoding the amino acid sequence.

The identity of a nucleotide sequence can be determined by use of the aforementioned identity search algorithm or the like (Proc. Natl. Acad. Sci. USA 872264-2268, 1990; and Proc Natl Acad Sci USA 90: 5873, 1993).

The nucleic acid encoding the anti-hMPV antibody of the present invention is preferably a DNA encoding the H chain or L chain of an antibody comprising CDR sequences in any one of the combinations of 26 CDR sequences of SEQ ID NOs: 3 to 5, 8 to 10, 13 to 15, 18 to 20, 23 to 25, 28 to 30, 33 to 35, 38 to 40, 43 to 45, 48 to 50, 53 to 55, 58 to 60, 63 to 65, 68 to 70, 73 to 75, 78 to 80, 83 to 85, 88 to 90, 93 to 95, 98 to 100, 103 to 105, 108 to 110, 113 to 115, 118 to 120, 123 to 125, and 128 to 130, or an antigen-binding fragment thereof, more preferably a DNA encoding the H chain or L chain of an antibody comprising any VH or VL selected from SEQ ID NOs: 2, 7, 12, 17, 22, 27, 32, 37, 42, 47, 52, 57, 62, 67, 72, 77, 82, 87, 92, 97, 102, 107, 112, 117, 122, and 127, or an antigen-binding fragment thereof, or an isolated nucleic acid hybridizing under high stringent conditions to any of these nucleic acids (DNAs).

The nucleic acid is further preferably a nucleic acid encoding the amino acid sequence of SEQ ID NO: 1, 6, 11, 16, 21, 26, 31, 36, 41, 46, 51, 56, 61, 66, 71, 76, 81, 86, 91, 96, 101, 106, 111, 116, 121, or 126, or an isolated nucleic acid hybridizing under high stringent conditions to any of these nucleic acids.

### 4. Vector, host cell, and antibody preparation method according to the present invention

The present invention also relates to a vector comprising the nucleic acid incorporated therein and a host cell comprising the vector introduced thereinto, and a method for preparing the antibody using the vector and the host cell.

The antibody of the present invention can also be prepared as a recombinant human antibody by use of a method known in the art (see e.g., Nature, 312: 643,1984; and Nature, 321: 522, 1986). The antibody of the present invention can be prepared, for example, by culturing the host cell having the vector according to the present invention incorporated therein and purifying the produced antibody from the culture supernatant or the like. More specifically, VH- and VL-encoding cDNAs can be respectively inserted to expression vectors for animal cells containing human antibody CH- and/or human antibody CL-encoding genes prepared from the same cell or another human cell to construct human antibody expression vectors, which are transferred to animal cells so that the antibody is produced by expression.

The vector to which the nucleic acid encoding VH or VL of the antibody of the present invention is incorporated is not necessarily limited and is preferably a vector that is routinely used for the expression of protein genes or the like and is particularly compatible with the expression of antibody genes, or a vector for high expression. Preferred examples thereof include vectors containing EF promoter and/or CMV enhancer. Usually, expression vectors respectively having VH- or VL-encoding nucleic acids incorporated therein are prepared, and host cells are cotransfected with the expression vectors. Alternatively, these nucleic acids may be incorporated in a single expression vector.

The host cell to which the expression vector is transferred is not necessarily limited and is preferably a cell that is routinely used for the expression of protein genes or the like and is particularly compatible with the expression of antibody genes. Examples thereof include bacteria (*E. coli,* etc.), *Actinomycetes*, yeasts, insect cells (SF9, etc.), and mammalian cells (COS-1, CHO, myeloma cells, etc.).

For the industrial production of the recombinant antibody, a recombinant animal cell line, for example, a CHO cell line, stably highly producing the antibody is generally used. The preparation and cloning of such a recombinant cell line, gene amplification for high expression, and screening can be carried out by use of methods known in the art (see e.g., Omasa T.: J. Biosci. Bioeng., 94, 600-605, 2002).

The present invention includes an antibody composed of two heavy chains and two light chains as well as an antigen-binding fragment of the antibody of the present invention. The antigen-binding fragment includes, for example, Fab (Fragment of antigen binding), Fab', F(ab')2, and single chain Fv (scFv) and disulfide stabilized Fv (dsFv) as active fragments of the antibody linked via a linker or the like. Examples of peptides comprising the active fragments of the antibody include peptides containing CDRs. These fragments can be produced by a method known in the art such as a method of treating the antibody of the present invention with an appropriate protease or a gene recombination technique.

The antibody can be purified by use of purification means known in the art such as salting out, gel filtration, ion-exchange chromatography, or affinity chromatography.

Alternatively, according to a recently developed phage display antibody technique which involves expressing a recombinant antibody on phage surface by use of a genetic engineering technique, VH and VL genes may be artificially shuffled, and diversified scFv (single chain Fragment of variable region) antibodies can be expressed as phage-fused proteins to obtain specific antibodies. This technique can circumvent immunity and is highly appreciated as a humanized antibody preparation technique as an alternative for the cell fusion method. Any specific antibody or antigen-binding fragment thereof prepared by use of this technique with reference to the amino acid sequences of SEQ ID NOs: 2 to 6, 8 to 12, 14 to 18, and 20 to 24 described herein is included in the technical scope of the present invention.

Alternatively, an antibody obtained by the application of Potelligent technique drastically improving the ADCC activity of an antibody by the modification of the sugar chain moiety of the antibody prepared by a recently developed technique, to the antibody of the present invention (see Clin. Cancer Res., 10, 6248-6255 (2004)), and an antibody obtained by the application of Complegent technique improving CDC activity, to the antibody of the present invention (see Glycobiology, 17, 104-118 (2007)) are also included in the technical scope of the present invention. Likewise, an antibody prepared by a recently developed method for modifying ADCC activity (WO2007/039682 and WO2007/100083) or CDC activity (WO2007/011041 and WO2011/091078) by the modification of a partial amino acid sequence in the constant region of the antibody is also included in the technical scope of the present invention.

Furthermore, any antibody or antigen-binding fragment thereof obtained by the application of a partial Fc region substitution technique (see WO2006/071877) performed in order to impart protease resistance ability to the antibody and renders the antibody orally administrable is included in the technical scope of the present invention.

As an antibody preparation approach, a polyclonal antibody or a monoclonal antibody is usually obtained by use of a laboratory animal such as a mouse, a rabbit, or a goat. Since the antibody thus obtained has a sequence characteristic of the animal species used, the antibody administered directly to a human may be recognized as foreign matter by the human immune system to cause human anti-animal antibody response (i.e., to yield an antibody against the antibody).

The anti-hMPV monoclonal antibody according to the present invention or an antigen-binding fragment thereof can be obtained from antibody-producing cells derived from the blood of a healthy person or the like. In this case, the antibody is a completely human antibody. This completely human antibody probably neither has immunogenicity nor causes immune response even if administered as an antibody drug to a human body.

Particularly, the monoclonal antibody of the present invention against hMPV F protein has higher neutralizing ability than that of the conventional anti-hMPV antibodies and as such, can be expected to produce equivalent therapeutic effects at a lower dose.

### 5. Pharmaceutical composition containing antibody according to the present invention

The present invention further provides a pharmaceutical composition for the prevention or treatment of a respiratory disease caused by hMPV, comprising the antibody or an antigen-binding site thereof and a pharmaceutically acceptable carrier.

Particularly, the anti-hMPV antibody according to the present invention or an antigen-binding fragment thereof has, through specific binding to hMPV F protein, higher neutralizing ability than that of the conventional anti-hMPV F protein antibodies and as such, is useful as a preventive or therapeutic drug for a disease involving the hMPV.

The "pharmaceutically acceptable carrier" used herein includes any or every biologically compatible solvent, dispersion medium, coating, tonicity agent, and absorption-delaying agent, etc.

Examples of the pharmaceutically acceptable carrier include one or more carriers such as water, a salt solution, phosphate-buffered saline, dextrose, glycerol, and ethanol, and combinations thereof. For use as an injection or the like, the composition preferably contains a pH adjuster or a tonicity agent, for example, a sugar, a polyalcohol (mannitol, sorbitol, etc.), or sodium chloride. The pharmaceutically acceptable carrier can further include a small amount of an auxiliary substance that enhances the conservation or effectiveness of the antibody or a moiety of the antibody, such as a wetting agent, an emulsifier, an antiseptic, a buffer, or a stabilizer.

The composition of the present invention can be prepared in various dosage forms. Such a composition includes liquid, semisolid, and solid dosage forms, for example, solutions (e.g., injectable and transfusable solutions), dispersions, suspensions, tablets, capsules, troches, pills, powders, liposomes, and suppositories. Preferred forms differ depending on the intended mode of administration and a case to which treatment is applied. The composition is generally preferably in the form of an injectable or transfusable solution, as with, for example, compositions similar to those used for the passive immunization of humans with other antibodies. The preferred mode of administration is parenteral administration (e.g., intravenous, subcutaneous, intraperitoneal, or intramuscular administration). In a preferred embodiment, the antibody is administered through intravenous transfusion or intravenous injection. In another preferred embodiment, the antibody is administered through intramuscular injection or subcutaneous injection.

The antibody of the present invention or the antibody fragment can be incorporated into a pharmaceutical composition suitable for parenteral administration. When one type of antibody or moiety of the antibody is used, the antibody or the moiety is preferably prepared as an injectable preparation containing 0.1 to 250 mg/mL of the antibody (moiety). On the other hand, when plural types of antibodies are used as a mixture, these antibodies are preferably prepared as an injectable preparation containing 0.001 to 100 mg/mL of each antibody. The mixing ratio of the plural types of antibodies can be appropriately set.

Exemplary injectable preparations will be described below. However, the preparation of the present invention is not limited thereto as long as the preparation is preferred as an injection of the antibody drug of the present invention. The preparation can be constituted by, for example, a flint or amber vial, an ampule, or a prefilled syringe containing an active ingredient dissolved in a liquid or a freeze-dried active ingredient. The buffer can be L-histidine (1 to 50 mM) having a pH of 5.0 to 7.0 (optimally pH 6.0), optimally 5 to 10 mM L-histidine. Other appropriate buffers include, but are not limited to, sodium succinate, sodium citrate, sodium phosphate, and potassium phosphate. Sodium chloride can be used in order to change the osmotic pressure of a solution having a concentration of 0 to 300 mM (optimally 150 mM for the liquid dosage form,). The freeze-dried dosage form can contain a cryoprotectant, mainly, 0 to 10% (optimally 0.5 to 5.0%) sucrose. Other appropriate cryoprotectants include mannitol, trehalose, and lactose. The freeze-dried dosage form can contain an expander, mainly, 1 to 10% (optimally 2 to 4%) mannitol. In both of the liquid and freeze-dried dosage forms, a stabilizer, mainly, 1 to 50 mM (optimally 5 to 10 mM) L-methionine can be used. Other appropriate stabilizers include glycine, arginine, and polysorbate 80, etc. In the case of polysorbate 80, 0 to 0.05% (optimally 0.005 to 0.01%) can be contained therein. Other surfactants include, but are not limited to, polysorbate 20 and BRIJ surfactants.

In general, the pharmaceutical composition of the present invention must be sterile or stable under production and preservation conditions. This composition can be formulated as a solution, a microemulsion, a dispersion, a liposome, or other ordered structures suitable for high drug concentrations. The sterile injectable solution can be prepared by mixing a necessary amount of an active compound (i.e., the antibody or a moiety of the antibody), if necessary together with one or combination of the aforementioned components, into an appropriate solvent, followed by filtration sterilization. In general, the active compound is mixed with a sterile vehicle containing a basic dispersion medium and other necessary components selected from those listed above to prepare a dispersion. Preferred methods for preparing a sterile powder preparation for preparing the sterile injectable solution are the vacuum freeze drying and spray drying of the sterile filtrate thereof mentioned above. As a result, a composition comprising a powder of the active ingredient as well as arbitrary other desired components is obtained. The adequate flowability of the solution can be maintained, for example, by using coating with lecithin or the like, by maintaining a necessary particle size in the case of the dispersion, or by using a surfactant. The long-term absorption of the injectable composition can be achieved by means of absorption-delaying agent, for example, monostearate or gelatin, contained in the composition.

### 6. Process of obtaining anti-hMPV antibody according to the present invention and antigen-binding fragment thereof

Next, the processes by which the anti-hMPV monoclonal antibody according to the present invention and an antigen-binding fragment thereof were obtained will be described. However, the approaches of obtaining the antibody according to the present invention, etc., are not limited by the description below. As mentioned above, changes or modifications usually performed in the art can be made therein, as a matter of course.

The anti-hMPV antibody according to the present invention and an antigen-binding fragment thereof can be obtained by: separating a cell clone producing the antibody through various steps from the blood of a human; and subjecting the antibody obtained from the culture supernatant of the antibody-producing cell clone to affinity purification.

### 1) Separation of cell clone producing completely human antibody against F proteins of hMPV

B lymphocytes are separated from the blood of a human, and the growth of the B lymphocytes is induced. The method for inducing the growth is known in the art per se and can be carried out by, for example, a transformation method (D. Kozbor et al.) using "Epstein-Barr virus (EB virus)" (hereinafter, referred to as EBV), which is a factor triggering cancer.

Specifically, the B lymphocytes are infected by EBV to induce its growth. The cells that have grown are used as an antibody-producing cell library.

### 2) Recovery of monoclonal antibody from antibody-producing cell library

The method for recovering monoclonal antibodies from the cells that have grown by induction can be carried out by a well-known method routinely used in the preparation of monoclonal antibodies.

The antibody-producing cell library is screened for a lymphocyte clone producing an antibody binding to hMPV F protein. The antibody is isolated from the culture supernatant thereof. Specifically, a cell population (clone) producing the antibody binding to hMPV F protein is selected from the antibody-producing cell library by a limiting dilution method.

The clone binding to hMPV F protein can be detected by use of ELISA with the F protein as an antigen and ELISA using a labeled mouse anti-human IgG antibody. An alternative method that may be used involves introducing an F protein expression vector into cells, and after expression of the F protein, immobilizing the cells onto a screening plate, which is then used in the detection of the anti-hMPV antibody (cell fluorescent immunostaining screening method).

The selected antibody-positive cell population can be cultured and repeatedly screened to obtain a cell population (clone) producing only the antibody of interest. A flowchart representing these steps up to the separation of the antibody-producing cell clone is shown in Figure 1.

### 3) Affinity purification using protein A or G

For the purification of the anti-hMPV antibody, the selected cells can be allowed to grow in a dish, a roller bottle, a 2-L spinner flask, or a different culture system.

The obtained culture supernatant can be filtered, concentrated, and then subjected to affinity chromatography using protein A or protein G-Sepharose (GE Healthcare Japan Corp.) or the like to purify the protein. The buffer solution is replaced with PBS, and the concentration can be determined by OD280 or, preferably, nephelometer analysis. The isotype can be examined by a method specific for the isotype antigen. The anti-hMPV antibody thus obtained is a completely human antibody prepared from B lymphocytes sensitized in the human body and is therefore substantially unlike to cause immune response.

Another feature of this approach is that the EB virus having the activity of inducing the growth of B lymphocytes by infection is used in the preparation of the antibody-producing cell clone.

The EB virus method has the advantages that: a natural antibody produced in a human body can be prepared; and an antibody having high affinity can be obtained. For example, a human antibody against a certain kind of virus (e.g., human CMV) has been found to have approximately 10 to 100 times higher affinity than that of an antibody prepared from an artificially immunized mouse. The B lymphocyte population that has grown by EB virus infection serves as a library of antibody-producing cells. From this library, a particular antibody-producing cell clone is separated to obtain a human antibody.

### 7. Process of evaluating activity in vitro

The biological properties of the antibody or an antibody composition can be evaluated by testing the ability of the antibody to suppress *in vitro* the biological activity of the hMPV F protein. The *in vitro* assay method for the antibody includes a binding assay method such as ELISA, and a neutralization assay method, etc.

### 1) Binding activity

The term "specifically binding" or "specific binding" used herein refers to the recognition of the predetermined antigen so as to bind thereto. The binding affinity of the antibody for the hMPV F protein can be measured according to a method known in the art. For example, the binding affinity can be measured against F protein immobilized on a chip by use of a protein interaction analysis apparatus such as Biacore T200(R). The binding affinity (K_{D} value) is indicated by the ratio of Kd (dissociation constant) obtained by this measurement method to Ka (association constant) (K_{D}= Kd / Ka). The binding affinity (KD value) of the human anti-hMPV antibody according to the present invention or an antigen-binding fragment thereof against JPS02-76 (hMPV_type B1) F protein is preferably 1 × 10⁻⁸ M or lower, more preferably 1 × 10⁻⁹ M or lower, further preferably 6 × 10⁻¹⁰ M or lower.

### 2) In vitro neutralizing activity

The terms such as "neutralization", "inhibitory effect", "inhibition", "suppression", and "capable of inhibiting" used herein mean that biological activity attributed to the antigen (hMPV) is reduced by approximately 5 to 100%, preferably 10 to 100%, more preferably 20 to 100%, more preferably 30 to 100%, more preferably 40 to 100%, more preferably 50 to 100%, more preferably 60 to 100%, more preferably 70 to 100%, further preferably 80 to 100%.

The *in vitro* neutralizing ability of the anti-hMPV antibody can be evaluated using typical strains of four hMPV subgroups (types A1, A2, B1, and B2) (J. Virol. 2008 p. 8942-8946). The hMPV strains used are, for example, a JPS03-180 strain of type A1, a JPS03-178 strain of type A2, a JPS02-76 strain of type B1, and a JPS05-21 strain of type B2. The ability of each strain to infect LLC-MK2 cells can be examined in the presence of the anti-hMPV antibody to evaluate the neutralizing ability of the antibody.

For reference, among the previously reported anti-hMPV antibodies, an antibody mAb338 (WO2006/110214), albeit derived from a mouse, has been reported to have high neutralizing activity against all of the hMPV types A1, A2, B1, and B2, and a human antibody HMB3210 (WO2013/140247) exhibits high neutralizing activity against both RSV and hMPV. These antibodies were both used as positive controls.

The anti-hMPV antibody according to the present invention or an antigen-binding fragment thereof has 50% infection inhibitory activity (IC50) at preferably approximately 2.3 µg/mL (approximately 15 nM) or lower, more preferably approximately 1 µg/mL (approximately 6.7 nM) or lower, further preferably approximately 0.6 µg/mL (approximately 4 nM) or lower, most preferably approximately 0.3 µg/mL (approximately 2 nM) or lower, against all of the strains JPS03-180 (type A1), JPS03-178 (type A2), JPS02-76 (type B1), and JPS05-21 (type B2) in an evaluation system for the ability of hMPV to infect LLC-MK2 cells. Alternatively, the anti-hMPV antibody according to the present invention or an antigen-binding fragment thereof has 90% infection inhibitory activity (IC90) at preferably approximately 3.5 µg/mL (approximately 23 nM) or lower, more preferably approximately 2 µg/mL (approximately 13.3 nM) or lower, further preferably approximately 1 µg/mL (approximately 6.7 nM) or lower, most preferably approximately 0.7 µg/mL (approximately 4.7 nM) or lower, against all of the strains JPS03-180 (type A1), JPS03-178 (type A2), JPS02-76 (type B1), and JPS05-21 (type B2).

Alternatively, a human antibody having 50% infection inhibitory activity (IC50) at approximately 2 µg/mL (approximately 13.3 nM) or lower, preferably approximately 1 µg/mL (approximately 6.7 nM) or lower, more preferably approximately 0.5 µg/mL (approximately 3.3 nM) or lower, further preferably approximately 0.2 µg/mL (approximately 1.33 nM) or lower, still further preferably approximately 0.1 µg/mL (approximately 0.67 nM) or lower, most preferably approximately 0.03 µg/mL (approximately 0.2 nM) or lower, in an evaluation system using JPS03-178 (type A2), or an antigen-binding fragment thereof is included in the scope of the present invention.

Alternatively, a human antibody having 90% infection inhibitory activity (IC90) at approximately 6 µg/mL (approximately 40 nM) or lower, preferably approximately 1 µg/mL (approximately 6.7 nM) or lower, more preferably approximately 0.2 µg/mL (approximately 1.33 nM) or lower, further preferably approximately 0.1 µg/mL (approximately 0.67 nM) or lower, in an evaluation system using JPS03-178 (type A2), or an antigen-binding fragment thereof is included in the scope of the present invention.

### 3) Cell-to-cell spread inhibitory activity

The cell-to-cell spread of hMPV requires the cleavage of FP. LLC-MK2 cells or VERO cells, which serve as host cells for *in vitro* hMPV infection, lack the activity of cleaving FP. Accordingly, membrane-type serine protease (TMPRSS2) having the activity of cleaving FP is introduced into LLC-MK2 cells to prepare host cells stably expressing TMPRSS2. The obtained cells stably expressing TMPRSS2 are infected by hMPV. In this system, the cell-to-cell spread inhibitory activity of the anti-hMPV antibody can be evaluated. When the cell-to-cell spread inhibitory activity of the anti-hMPV antibody is evaluated in a system in which the host cells are infected by a JPS02-76 strain of hMPV type B1, a human antibody having 50% cell-to-cell spread inhibitory activity (IC50) at approximately 0.2 µg/mL (approximately 1.33 nM) or lower, preferably approximately 0.1 µg/mL (approximately 0.67 nM) or lower, more preferably approximately 0.05 µg/mL (approximately 0.33 nM) or lower, further preferably approximately 0.02 µg/mL (approximately 0.133 nM) or lower, or an antigen-binding fragment thereof is included in the scope the present invention.

### 8. Epitope grouping

The difference in epitope among anti-hMPV antibodies can be evaluated by the competition assay among the antibodies and the examination of the binding activity of each antibody against cells expressing a partially deleted peptide of the F protein (see the paragraph "12. Epitope mapping" in Examples and Figures 3 and 4). As a result, the anti-hMPV antibody of the present invention can be classified into any of 5 epitope groups (Group 1, Group 2, Group 3, Group 4, and Group 5). Specifically, Group 1 (group of antibodies binding to FP and HR1 deletion mutants, but not binding to F2, F1-1, F1-2, F1-3, F1-4, F1-5, and F1-6 deletion mutants) includes EV046115b, EV046130, and EV046147. Group 2 (group of antibodies binding to FP, HR1, F1-4, F1-5, and F1-6 deletion mutants, but not binding to F2, F1-1, F1-2, and F1-3 deletion mutants) includes mAb338, EV046113, EV046116, EV046141, and EV046142. Group 3 (group of antibodies binding to FP, F1-4, F1-5, and F1-6 deletion mutants, but not binding to F2, HR1, F1-1, F1-2, and F1-3 deletion mutants) includes EV046124, EV046143, and EV046150. Group 4 (group of antibodies binding to FP, HR1, and F1-6 deletion mutants, but not binding to F2, F1-1, F1-2, F1-3, F1-4, and F1-5 deletion mutants) includes EV046120, and EV046135. Group 5 (group of antibodies binding to FP, HR1, F1-1, F1-3, F1-4, F1-5, and F1-6 deletion mutants, but not binding to F2 and F1-2 deletion mutants) includes EV046136. Thus, the anti-hMPV antibody of the present invention is an antibody binding to hMPV F protein or an antigen-binding fragment thereof and is preferably an antibody binding to any of the epitope groups defined by Group 1, Group 2, Group 3, Group 4, and Group 5, or an antigen-binding fragment thereof, more preferably an antibody binding to any of the epitope groups defined by Group 2, Group 3, Group 4, and Group 5, or an antigen-binding fragment thereof, further preferably an antibody binding to any of the epitope groups defined by Group 3, Group 4, and Group 5, or an antigen-binding fragment thereof, most preferably an antibody binding to any of the epitope group defined by Group 3, or an antigen-binding fragment thereof.

For the epitope grouping, among the previously reported anti-hMPV antibodies, a mouse antibody mAb338 (WO2006/110214) having high neutralizing activity against all of the hMPV strains A1, A2, B 1, and B2 and already reported to be analyzed for its epitope (a.a. 238-245 of F protein), and a human antibody HMB3210 (WO2013/140247) binding both to the F proteins of hMPV and RSV were both used as controls for measurement.

Hereinafter, the present invention will be described further specifically with reference to Examples. However, the present invention is not intended to be limited by these Examples by any means. For the procedures used in these Examples, see Molecular Cloning: A Laboratory Manual (Third Edition) (Sambrook et al., Cold Spring Harbour Laboratory Press, 2001), unless otherwise specified.

### EXAMPLES

### 1. Separation of cell clone producing completely human antibody against hMPV

A flowchart for the separation of an antibody-producing cell clone is shown in Figure 1.

B lymphocytes were separated from human peripheral blood and infected by EBV. The infected cells were inoculated to a 96-well plate, cultured for 3 to 4 weeks, and then primarily screened for anti-hMPV antibodies in the culture supernatant. The cells in each well confirmed to contain the produced anti-hMPV antibodies were inoculated to a fresh 96-well plate. After culture for 3 to 4 weeks, the cells were secondarily screened for anti-hMPV antibodies. The cells in each antibody-positive well were inoculated at 1 to 30 cells/well to a fresh 96-well plate. After culture for 3 to 5 weeks, the cells were tertiarily screened for anti-hMPV antibodies. As a result of this cloning operation involving the limiting dilution culture method, etc., a cell clone producing each antibody of interest was obtained.

### 2. Cell fluorescent immunostaining screening of anti-hMPV antibody

The screening targeted antibodies against fusion protein (FP), which is a main neutralization target in hMPV. RNA was extracted from hMPV (JPS02-76), and cDNA was synthesized using random decamers. The full-length FP gene was amplified by PCR and cloned into an expression vector. The nucleotide sequence of the cloned FP gene and an amino acid sequence encoded thereby are shown in SEQ ID NOs: 131 and 132, respectively. The cloned FP gene differs by 2 bases from the original sequence registered in the gene bank (GenBank Accession No. AY530089) and resulted in one amino acid substitution.

CHO-K1 cells were transfected with the FP expression vector. After 16 hours, the cells were inoculated again to a 96-well plate or a 384-well plate. This transfection was carried out using Lipofectamine LTX (Invitrogen Corp.) and Plus reagent (Invitrogen Corp.) under conditions recommended by the manufacturer. The CHO-K1 cells then cultured for 24 hours were fixed using 4% paraformaldehyde and 0.1 M phosphate buffer (pH 7.4). Next, the cells were washed with PBS-0099PBS(-) and 0.1% Tween 20 and permeabilized using 1 x PBS(-) and 0.2% Triton X-100. The prepared plate was used as an anti-hMPV antibody screening plate.

The culture supernatant of the EBV-infected cells was added to the anti-hMPV antibody screening plate and reacted at room temperature for 1 hour, followed by washing with PBS-T three times. Next, anti-human IgG-Alexa 488 (Invitrogen Corp.) was diluted 1000-fold with PBS-T and 0.1% FCS, added thereto as a secondary antibody, and reacted at room temperature for 1 hour, followed by washing with PBS-T once. Next, 0.8 µg/ml DAPI and PBS-T were added thereto and reacted at room temperature for 10 minutes, followed by washing with PBS-T twice and the subsequent addition of 1 x PBS(-).

The stained cells were photographed using In Cell Analyzer 2000 (GE Healthcare Japan Corp.). Each well in which the positive cells were detected was used as an antibody-positive well.

### 3. Confirmation of antibody isotype and subclass

Each produced antibody was isotyped by the cell fluorescent immunostaining method using the culture supernatant of the separated antibody-producing cell clone. Specifically, the anti-hMPV antibody screening plate was used, and an antibody specific for each isotype and subclass was used as a secondary antibody. The isotypes and subclasses of the obtained 13 anti-hMPV antibodies are shown in Table 2 mentioned later.

### 4. Cloning of DNA encoding anti-hMPV antibody

The total RNA of the antibody-producing cells was reverse-transcribed using oligo-dT primers. The obtained cDNA was used as a template in the PCR amplification of each antibody gene. The primers used in PCR were designed on the basis of the database of cDNAs encoding human IgG antibody H and L chains. In order to amplify the full-length H chain cDNA and L chain cDNA, the 5' primer has a translation initiation point, and the 3' primer has a translation termination point.

### 5. Determination of amino acid sequence of antibody based on nucleotide sequence

The H chain and L chain cDNAs of each antibody amplified by PCR were inserted to plasmid vectors, and their nucleotide sequences were confirmed using an ABI sequencer. The signal sequence, H chain and L chain amino acid sequences, variable region amino acid sequences, and complementarity-determining region (CDR) amino acid sequences of the antibody were each determined from the obtained nucleotide sequences. In the CDR analysis, the method of Kabat (www.bioinf.org.uk: Dr. Andrew C.R. Martin's Group, Antibodies: General Information) was used. SEQ ID NOs of the amino acid sequences of the H and L chains of the obtained antibodies, the amino acid sequences of their variable regions, and the amino acid sequences of their complementarity-determining regions (CDRs) are each shown in Table 1.

### 6. Confirmation that obtained antibody gene encodes anti-hMPV antibody

The obtained H chain and L chain genes were respectively inserted to expression vectors. CHO-K1 cells were cotransfected with the expression vectors. The transfection was carried out using Lipofectamine LTX (Invitrogen Corp.) and Plus reagent (Invitrogen Corp.) under conditions recommended by the manufacturer. Two days later, the culture supernatant was recovered. Each antibody in the culture supernatant was confirmed to be human IgG by ELISA using a 96-well plate coated with an anti-human IgG antibody, and confirmed to bind to hMPV FP by the cell fluorescent immunostaining method using the anti-hMPV antibody screening plate.

### 7. Production of antibody protein

CHO-K1 cells were transfected with the obtained anti-hMPV antibody expression vector. The cells were cultured in the presence of a selection marker to obtain a CHO-K1 cell clone constitutively producing each antibody.

The CHO-K1 cells stably producing each antibody were cultured in a serum-free medium, and the culture supernatant was recovered. This culture supernatant was subjected to affinity purification using a protein A column to obtain a purified antibody. The column used was a prepack column of HiTrap rProtein A FF (GE Healthcare Japan Corp.). The purification was carried out under conditions recommended by the manufacturer. After the purification, the binding activity of each antibody against hMPV FP was confirmed using the anti-hMPV antibody screening plate. Also, an antibody H chain (approximately 50 kDa) and an antibody L chain (approximately 25 kDa) were confirmed by SDS-PAGE.

### 8. Neutralizing activity evaluation

### Immunostaining method:

The neutralizing activity was confirmed in the evaluation of each anti-hMPV antibody for its effectiveness. The neutralizing activity was evaluated on the basis of the rate of inhibition of hMPV infection of LLC-MK2 cells by the antibody. The hMPV strains used were JPS02-76 as a B 1 strain, JPS05-21 as a B2 strain, JPS03-180 as an A1 strain, and JPS03-178 as an A2 strain.

The virus was prepared by the following method:

A virus solution was added to LLC-MK2 cells washed with 1 × PBS(-) twice. After culture for 1 hour, EMEM containing 5 mM sucrose, 2 mM L-glutamine, and 0.5 µg/ml trypsin was added thereto as an infection medium, and the cells were cultured until CPE appeared. Then, uninfected LLC-ML2 cells and the infected cells were mixed at a ratio of 1:9 and cultured in an infection medium until CPE appeared to prepare seed-infected cells having 100% rate of infection. For a virus solution, uninfected LLC-MK2 cells and the seed-infected cells were mixed at a ratio of 1:9 and cultured in an infection medium until CPE appeared. The resulting culture supernatant was recovered. Then, the culture supernatant was centrifuged (20,000 g, 4°C, 2.5 hours), if necessary, to enrich the virus, which was then dispensed and cryopreserved at -80°C until use.

The neutralizing activity was evaluated by the following method:

Six 4-fold dilution series of each purified anti-hMPV antibody were prepared from 10 µg/ml. The virus solution (0.8 to 1.4 × 10⁴ pfu/ml) and the purified antibody were mixed at a ratio of 1:1 and left at 25°C for 1 hour. On the day before virus addition, LLC-MK2 cells were inoculated to a 96-well plate so as to reach 100% confluency. Before virus addition, the cells were washed with EMEM twice. The mixed solution of the virus solution and the purified antibody was added to LLC-MK2, and the cells were cultured for 1 hour in a CO₂ incubator. Then, the cells were washed with EMEM three times. EMEM containing 5 mM sucrose and 2 mM L-glutamine was added thereto, and the cells were cultured for 20 hours in a CO₂ incubator. In order to detect infected cells, the cells were fixed in 80% acetone, and the plate was blocked at room temperature for 1 hour by the addition of 1% BSA and 1 x PBS(-). Next, a mouse anti-hMPV antibody (HMPV123, AbD Serotec) was diluted to 1 µg/ml with PBS-T and 0.1% goat serum, added thereto, and reacted at room temperature for 1 hour. After washing PBS-T three times, anti-mouse IgG-Alexa 488 (Invitrogen Corp.) diluted 3000-fold as a secondary antibody and DAPI diluted to 0.4 µg/ml with PBS-T and 0.1% goat serum were added thereto and reacted at room temperature for 1 hour. After washing with PBS-T three times, 1 x PBS(-) was added thereto. The infected cells were photographed using In Cell Analyzer 2000 to count positive cells.

The negative control used in the neutralizing activity evaluation was a human monoclonal antibody (hIgG) having no specificity for hMPV. When the average number of infected cells in the presence of the negative control was defined as 0% rate of inhibition of infection, IC50 was calculated from the rate of inhibition of infection by each antibody at each concentration (calculated from antibody concentrations at two points spanning the 50% rate of inhibition, and the rates of inhibition) (Table 2).

Incidentally, a human-derived anti-hMPV antibody DSλ7 (Fab) has been shown to hardly exhibit neutralizing activity against B2 and A1 strains (neutralizing activity (IC60): > 59 µg/mL (1180 nM) and 9.8 µg/mL (196 nM), respectively) (WO2008/043052 and J. Virol., 2007 (vol. 81) p. 8315). According to this report, human antibodies (DS1, DS6, ACN044, etc. (all, Fab)) exhibit no neutralizing activity against 3 strains (types A1, B1, and B2) except for the A2 strain (IC60 value: >8 µg/mL (160 nM)). In consideration of these factors, the antibody of the present invention having an IC50 value of approximately 1 µg/mL (approximately 6.7 nM) or lower against at least all of types A1, B1 and B2 is significantly superior to the human antibodies disclosed in WO2008/043052.

**[Table 2]**

| Isotype of anti-hMPV antibody and IC50 against each hMPV strain | | | | |
|---|---|---|---|---|
| Ab ID. | isotype | IC50 (*µ*g /ml) | | |
| | | hMPV-B1 | hMPV-B2 | hMPV-A1 |
| | | JPS02-76 | JPS05-21 | JPS03-180 |
| EV046130 | IgG1/λ | 0.230 | 0.089 | 0.210 |
| EV046116 | IgG1/κ | 0.250 | 0.250 | 0.310 |
| EV046120 | IgG1/λ | 0.250 | 0.240 | 0.320 |
| EV046115b | IgG1/λ | 0.270 | 0.099 | 0.290 |
| EV046124 | IgG1/λ | 0.290 | 0.240 | 0.070 |
| EV046150 | IgG1/κ | 0.300 | 0.180 | 0.049 |
| EV046136 | IgG1/κ | 0.310 | 0.460 | 0.290 |
| EV046135 | IgG1/λ | 0.370 | 0.220 | 0.350 |
| EV046147 | IgG1/λ | 0.390 | 0.270 | 0.310 |
| EV046142 | IgG1/λ | 0.420 | 0.220 | 0.220 |
| EV046143 | IgG1/κ | 0.460 | 0.270 | 0.130 |
| EV046113 | IgG1/κ | 0.680 | 0.390 | 0.380 |
| EV046141 | IgG1/λ | 0.740 | 0.440 | 0.200 |

IC50, IC60, and IC90 further calculated from the data shown in Table 2 using data analysis software GraphPad Prism 6 are shown in Tables 3, 4, and 5, respectively. In these Tables 3, 4, and 5, neutralizing activity against JPS03-178 (type A2) was additionally shown, while positive controls used for neutralizing activity were prepared on the basis of information disclosed on a mouse antibody mAb338 against hMPV described in the patent literature JP2008-538353 (WO2006/110214) and a human antibody HMB3210 described in the patent literature WO2013/140247.

As a result of determining the IC50 (see Table 3) and IC60 (see Table 4) values by recalculation as mentioned above, all of the antibodies of the present invention also exhibited a neutralizing activity of approximately 1 µg/mL or lower (approximately 6.7 nM or lower) against types A1, B1, and B2. Thus, all of the antibodies of the present invention are significantly superior to the human antibodies disclosed in WO2008/043052. Their IC60 values against all types including type A2 were approximately 1 to 3 µg/mL or lower as a whole.

As is also evident from the results of Table 3, all of the antibodies of the present invention exhibited 50% inhibitory effect against all of the strains of hMPV type at approximately 2.3 µg/mL (approximately 15 nM) or lower. Thus, all of the antibodies of the present invention have high neutralizing activity. As seen from the results of Table 3, the anti-hMPV antibodies except for EV046115b, EV046130, EV046147, and EV046141 also exhibited 50% inhibitory effect (IC50) against all of the A1, A2, B1, and B2 strains at approximately 1 µg/mL (approximately 6.7 nM) or lower. Furthermore, all of EV046120, EV046124, EV046135, EV046142, EV046143, and EV046150 exhibited 50% inhibitory effect against hMPV type A2 at approximately 0.1 µg/mL (approximately 0.67 nM) or lower. Particularly, EV046135, EV046143, and EV046150 exhibited 50% inhibitory effect at approximately 0.03 µg /mL (approximately 0.2 nM) or lower. These antibodies were significantly superior even to HMB3210 (139.3 ng/mL).

As also seen from the results about the IC90 values in Table 5, all of the antibodies except for EV046115b exhibited 90% inhibitory effect (IC90) against all of types A1, A2, B1, and B2 at approximately 3.5 µg/mL (approximately 23 nM) or lower. Among them, particularly, EV046124 and EV046150 exhibited 90% inhibitory effect (IC90) against all of types A1, A2, B1, and B2 at approximately 1 µg/mL (approximately 6.7 nM) or lower. Thus, these antibodies have much higher neutralizing activity even than that of HMB3210 (approximately 3.6 µg/mL or lower). Furthermore, all of EV046124, EV046143, and EV046150 exhibited a neutralizing activity of 188.1 ng/mL, 96.71 ng/mL, and 101.1 ng/mL, respectively, against hMPV type A2 and thus had a neutralizing activity as very high as approximately 0.2 µg/mL (approximately 1.33 nM) or lower. Particularly, both EV046143 and EV046150 had an IC90 value of approximately 0.1 µg/mL

(approximately 0.67 nM). These antibodies were significantly superior even to HMB3210 (approximately 0.58 µg/mL).

**[Table 3]**

| IC50 of anti-hMPV antibody against each hMPV strain | | | | |
|---|---|---|---|---|
| | IC50 (ng/ml) | | | |
| Ab ID. | MPV-A1 | MPV-A2 | MPV-B1 | MPV-B2 |
| | JPS03-180 | JPS03-178 | JPS02-76 | JPS05-21 |
| EV046113 | 418.0 | 645.1 | 716.2 | 490.7 |
| EV046115b | 322.4 | 2284 | 249.7 | 107.6 |
| EV046116 | 351.1 | 189.8 | 330.9 | 279.0 |
| EV046120 | 300.1 | 97.13 | 241.3 | 249.4 |
| EV046124 | 76.73 | 40.67 | 352.8 | 254.9 |
| EV046130 | 219.5 | 1950 | 302.3 | 104.8 |
| EV046135 | 348.2 | 31.56 | 463.9 | 212.6 |
| EV046136 | 318.1 | 318.1 | 352.9 | 537.0 |
| EV046141 | 213.5 | 2113 | 718.7 | 524.0 |
| EV046142 | 211.8 | 61.35 | 523.3 | 272.6 |
| EV046143 | 156.6 | 19.81 | 605.7 | 272.4 |
| EV046147 | 326.1 | 1975 | 425.4 | 252.7 |
| EV046150 | 61.81 | 29.78 | 314.3 | 202.1 |
| mAb338 | 124.7 | 180.3 | 281.1 | 221.0 |
| HMB3210 | 388.8 | 139.3 | 138.8 | 40.81 |

**[Table 4]**

| IC60 of anti-hMPV antibody against each hMPV strain | | | | |
|---|---|---|---|---|
| | IC60 (ng/ml) | | | |
| Ah ID. | HMPV-AWL | MPV-A2 | MPV-B1 | MPV-B2 |
| | JPS03-180 | JPS03-178 | JPS02-76 | JPS05-21 |
| EV046113 | 521.8 | 759.6 | 818.8 | 652.1 |
| EV046115b | 399.5 | 2709 | 376.0 | 133.4 |
| EV046116 | 427.9 | 247.4 | 497.4 | 341.3 |
| EV046120 | 421.0 | 138.0 | 344.4 | 301.3 |
| EV046124 | 102.3 | 53.93 | 423.3 | 305.0 |
| EV046130 | 254.0 | 2010 | 354.0 | 131.6 |
| EV046135 | 440.0 | 47.12 | 571.1 | 268.4 |
| EV046136 | 358.5 | 358.5 | 419.3 | 669.8 |
| EV046141 | 276.1 | 2184 | 882.9 | 570.8 |
| EV046142 | 242.7 | 97.11 | 641.0 | 365.7 |
| EV046143 | 186.4 | 26.55 | 627.3 | 363.1 |
| EV046147 | 405.9 | 2060 | 513.8 | 297.7 |
| EV046150 | 82.31 | 37.32 | 360.5 | 229.4 |
| mAb338 | 160.0 | 239.0 | 338.0 | 261.1 |
| HMB3210 | 585.6 | 181.2 | 190.1 | 48.13 |

**[Table 5]**

| IC90 of anti-hMPV antibody against each hMPV strain | | | | |
|---|---|---|---|---|
| | IC90 (ng/ml) | | | |
| Ab ID. | MPV-A1 | MPV-A2 | MPV-B1 | MPV-B2 |
| | JPS03-180 | JPS03-178 | JPS02-76 | JPS05-21 |
| EV046113 | 1393 | 1558 | 1480 | 2289 |
| EV046115b | 1034 | 5745 | 2304 | 344.7 |
| EV046116 | 1030 | 800.4 | 3005 | 828.3 |
| EV046120 | 1883 | 652.6 | 1651 | 693.5 |
| EV046124 | 364.6 | 188.1 | 952.3 | 676.8 |
| EV046130 | 485.8 | 2296 | 714.4 | 358.2 |
| EV046135 | 1235 | 277.2 | 1437 | 748.0 |
| EV046136 | 605.3 | 605.3 | 895.0 | 1778 |
| EV046141 | 863.0 | 2523 | 2182 | 835.9 |
| EV046142 | 443.1 | 737.6 | 1567 | 1346 |
| EV046143 | 401.6 | 96.71 | 734.5 | 1295 |
| EV046147 | 1072 | 2407 | 1182 | 612.1 |
| EV046150 | 292.0 | 101.1 | 662.8 | 403.6 |
| mAb338 | 498.4 | 856.3 | 772.0 | 547.0 |
| HMB3210 | 3569 | 578.7 | 847.7 | 99.53 |

### 9. Evaluation of cell-to-cell spread inhibitory activity

The cell-to-cell spread inhibitory activity was further evaluated as the evaluation of each anti-hMPV antibody for its effectiveness.

hMPV infection requires the cleavage of FP. LLC-MK2 cells or VERO cells, which serve as host cells for *in vitro* hMPV infection, lack the activity of cleaving FP. For this reason, trypsin is generally added to a medium. However, antibody deactivation by trypsin is of concern to a system in which antibodies are continuously added in order to evaluate cell-to-cell spread inhibitory activity. In this respect, membrane-type serine protease (TMPRSS2) reportedly having the activity of cleaving FP was introduced into LLC-MK2 cells to prepare host cells stably expressing TMPRSS2 (Shirogane et al. 2008 J. Virol. 82: 8942-8946).

The obtained cells stably expressing TMPRSS2 were infected by hMPV. As a result of spreading the hMPV infection under trypsin-free conditions, the number of infected cells was increased, and foci were confirmed.

The actual evaluation of the cell-to-cell spread inhibitory activity was conducted as follows: the cells stably expressing TMPRSS2 were infected by hMPV. After a lapse of 4 hours, six 4-fold dilution series of each antibody starting at 40 µg/ml were added to the cells. After 72 hours, the cells were fixed in the same way as in the neutralizing activity evaluation, and the infected cells were detected.

The negative control used in the cell-to-cell spread inhibitory activity evaluation was a human monoclonal antibody (hIgG) having no specificity for hMPV. The positive control used in this evaluation was a synthesized antibody mAb338 against hMPV as described in the patent literature JP2008-538353. When the average number of infected cells in the presence of the negative control at respective concentrations was defined as 0% rate of inhibition of cell-to-cell spread and the number of infected cells in the presence of the positive control at 40 µg/ml was defined as 100% rate of inhibition of cell-to-cell spread, the rate of inhibition of infection by each antibody at each concentration was calculated. Its IC50 and IC90 (ng/ml) were calculated using data analysis software GraphPad Prism 6. The results are shown in Table 6.

**[Table 6]**

| Cell-to-cell spread inhibitory activity of anti-hMPV antibody | | |
|---|---|---|
| Ab ID. | MPV-B1 (JPS02-76) | |
| | IC50 (ng/ml) | IC90 (ng/ml) |
| EV046113 | 107.6 | 707.4 |
| EV046115b | 134.5 | 617.0 |
| EV046116 | 54.00 | 132.2 |
| EV046120 | 13.16 | 66.88 |
| EV046124 | 48.18 | 259.5 |
| EV046130 | 34.86 | 147.3 |
| EV046135 | 30.77 | 230.5 |
| EV046136 | 52.22 | 211.9 |
| EV046141 | 36.27 | 171.0 |
| EV046142 | 12.34 | 66.41 |
| EV046143 | 57.19 | 885.7 |
| EV046147 | 113.9 | 506.1 |
| EV046150 | 16.64 | 64.95 |
| mAb338 | 21.54 | 93.75 |

### 10. Affinity analysis

The affinity analysis was conducted as the evaluation of each anti-hMPV antibody for its binding activity.

The antigens used in the analysis were prepared as follows: a gene encoding FP-TM(-)-His, which was His-Tag-added hMPV (JPS02-76) FP lacking the transmembrane region (amino acid residues 491 to 539 of FP), was cloned into an expression vector. CHO-K1 cells were transfected with the FP-TM(-)-His expression vector. The cells were cultured in the presence of a selection marker to obtain a CHO-K1 cell clone stably expressing FP-TM(-)-His. The culture supernatant of the cells stably expressing FP-TM(-)-His was recovered. FP-TM(-)-His was purified using Ni-Sepharose 6 Fast Flow carrier (GE Healthcare Japan Corp.). After the purification, imidazole was removed from FP-TM(-)-His by gel filtration, and the resulting protein was quantified and then used in affinity analysis.

The affinity analysis was conducted using Biacore T-200. Anti-human IgG was immobilized onto a sensor chip using human IgG capture kit (GE Healthcare Japan Corp.). Each anti-hMPV antibody was captured thereon and used as a ligand. The analyte used was the FP-TM(-)-His thus purified. The affinity of each anti-hMPV antibody for hMPV (JPS02-76) FP is shown in Table 7. All of the anti-hMPV antibodies exhibited binding activity as very high as a K_{D} value of 10 nM or lower against FP (Table 7). The affinity of EV046115b and EV046147 was immeasurable (ND) due to strong aggregation reaction on the sensor chip.

**[Table 7]**

| Results of affinity analysis of anti-hMPV antibody | | | |
|---|---|---|---|
| Ab ID. | Affiniy (MPV-B1 : JPS02-76) | | |
| | Ka (1/Ms) | Kd (1/s) | KD (M) |
| EV046113 | 4.632E+5 | 1.227E-4 | 2.648E-10 |
| EV046115b | N.D. | N.D. | N.D. |
| EV046116 | 2.521E+5 | 4.077 E-4 | 1.618E-9 |
| EV046120 | 2.898E+5 | 6.169E-4 | 2.115E-9 |
| EV046124 | 3.768E+5 | 2.255E-4 | 5.993E-10 |
| EV046130 | 5.873E+5 | 1.532E-3 | 2.642E-9 |
| EV046135 | 4.139E+5 | 3.649E-4 | 9.291E-10 |
| EV046136 | 3.914E+5 | 3.651E-4 | 9.501E-10 |
| EV046141 | 7.156E+5 | 1.179E-3 | 1.810E-9 |
| EV046142 | 2.755E+5 | 1.085E-4 | 4.249E-10 |
| EV046143 | 5.809E+5 | 9.379E-5 | 1.640E-10 |
| EV046147 | N.D. | N.D. | N.D. |
| EV046150 | 3.914E+5 | 9.635E-5 | 2.484E-10 |
| mAb338 | 4.344E+5 | 2.283E-4 | 5.315E-10 |

| | | | |
|---|---|---|---|
| (N.D.: immeasurable due to antibody aggregation reaction on the sensor chip) | | | |

### 11. Competition assay

The competition assay of each anti-hMPV antibody was conducted.

The antigen used in in the analysis was the culture supernatant of the cells stably expressing FP-TM(-)-His as shown in the affinity analysis. The assay was conducted using Biacore T-200. After capturing of FP-TM(-)-His onto Sensor Chip NTA (GE Healthcare Japan Corp.), an anti-hMPV antibody was bound as Sample 1 to the sensor chip until a saturation level. Next, another anti-MPV antibody was bound thereto as Sample 2. The presence or absence of competition was determined on the basis of whether or not the binding of the anti-hMPV antibody as Sample 2 was inhibited. The competition assay results are shown in Figure 2. The binding of EV046147 was immeasurable due to strong aggregation reaction on the sensor chip.

### 12. Epitope mapping

For the epitope grouping of the anti-hMPV antibodies, deletion mutant binding assay was conducted.

The prepared deletion mutants are shown in Figure 3. HA-V5-Full is hMPV (JPS02-76) FP containing HA-Tag (HA; TMYPYDVPDYA) added downstream of the signal peptide (SP) and a linker moiety (SLEGPRFE) and V5-Tag (V5; GKPIPNPLLGLDST) added downstream of the C-tail (C). The full-length nucleotide sequence of the HA-V5-Full used and an amino acid sequence encoded thereby are shown in SEQ ID NOs: 133 and 134, respectively.

The gene of each deletion mutant was cloned into an expression vector. The binding of each anti-hMPV antibody was detected in the same way as in the cell fluorescent immunostaining screening. Also, an anti-HA antibody and an anti-V5 antibody were used to confirm the expression of the deletion mutants themselves. The deletion mutant binding assay results are shown in Figure 4. The epitope grouping results together with the competition assay results are shown in Figure 5.

For the competition assay and the epitope mapping, a mouse anti-hMPV antibody mAb338 (J. Virol., 2008 (vol. 89) p. 3113) and a human antibody HMB3210 were used as controls.

These results demonstrated that: HMB3210 does not belong to any of the epitope groups of the antibody of the present invention; and mAb338 belongs to the epitope group 2 of the antibody of the present invention.

### INDUSTRIAL APPLICABILITY

The anti-human metapneumovirus (hMPV) antibody of the present invention is useful in uses such as a pharmaceutical composition for the prevention or treatment of a disease involving the hMPV.

## Claims

1. An antibody capable of specifically binding to human metapneumovirus F protein and neutralizing its biological activity, or an antigen-binding fragment thereof, comprising a heavy chain variable region and a light chain variable region wherein
(i) the heavy chain variable region comprises:
(a) the amino acid sequence of heavy chain CDR1 selected from the group consisting of the amino acid sequences of SEQ ID NOs: 3, 13, 23, 33, 43, 53, 63, 73, 83, 93, 103, 113, and 123 and amino acid sequences derived from any of these amino acid sequences by the deletion, substitution, insertion, and/or addition mutations of one to several amino acid residues,
(b) the amino acid sequence of heavy chain CDR2 selected from the group consisting of the amino acid sequences of SEQ ID NOs: 4, 14, 24, 34, 44, 54, 64, 74, 84, 94, 104, 114, and 124 and amino acid sequences derived from any of these amino acid sequences by the deletion, substitution, insertion, and/or addition mutations of one to several amino acid residues, and
(c) the amino acid sequence of heavy chain CDR3 selected from the group consisting of the amino acid sequences of SEQ ID NOs: 5, 15, 25, 35, 45, 55, 65, 75, 85, 95, 105, 115, and 125 and amino acid sequences derived from any of these amino acid sequences by the deletion, substitution, insertion, and/or addition mutations of one to several amino acid residues, and
(ii) the light chain variable region comprises:
(a) the amino acid sequence of light chain CDR1 selected from the group consisting of the amino acid sequences of SEQ ID NOs: 8, 18, 28, 38, 48, 58, 68, 78, 88, 98, 108, 118, and 128 and amino acid sequences derived from any of these amino acid sequences by the deletion, substitution, insertion, and/or addition mutations of one to several amino acid residues,
(b) the amino acid sequence of light chain CDR2 selected from the group consisting of the amino acid sequences of SEQ ID NOs: 9, 19, 29, 39, 49, 59, 69, 79, 89, 99, 109, 119, and 129 and amino acid sequences derived from any of these amino acid sequences by the deletion, substitution, insertion, and/or addition mutations of one to several amino acid residues, and
(c) the amino acid sequence of light chain CDR3 selected from the group consisting of the amino acid sequences of SEQ ID NOs: 10, 20, 30, 40, 50, 60, 70, 80, 90, 100, 110, 120, and 130 and amino acid sequences derived from any of these amino acid sequences by the deletion, substitution, insertion, and/or addition mutations of one to several amino acid residues.

2. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 3,
(b) the amino acid sequence of heavy chain CDR2 comprising the amino acid sequence of SEQ ID NO: 4, and
(c) the amino acid sequence of heavy chain CDR3 comprising the amino acid sequence of SEQ ID NO: 5, and
(ii) the light chain variable region comprises:
(a) the amino acid sequence of light chain CDR1 comprising the amino acid sequence of SEQ ID NO: 8,
(b) the amino acid sequence of light chain CDR2 comprising the amino acid sequence of SEQ ID NO: 9, and
(c) the amino acid sequence of light chain CDR3 comprising the amino acid sequence of SEQ ID NO: 10.

3. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 13,
(b) the amino acid sequence of heavy chain CDR2 comprising the amino acid sequence of SEQ ID NO: 14, and
(c) the amino acid sequence of heavy chain CDR3 comprising the amino acid sequence of SEQ ID NO: 15, and
(ii) the light chain variable region comprises:
(a) the amino acid sequence of light chain CDR1 comprising the amino acid sequence of SEQ ID NO: 18,
(b) the amino acid sequence of light chain CDR2 comprising the amino acid sequence of SEQ ID NO: 19, and
(c) the amino acid sequence of light chain CDR3 comprising the amino acid sequence of SEQ ID NO: 20.

4. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 23,
(b) the amino acid sequence of heavy chain CDR2 comprising the amino acid sequence of SEQ ID NO: 24, and
(c) the amino acid sequence of heavy chain CDR3 comprising the amino acid sequence of SEQ ID NO: 25, and
(ii) the light chain variable region comprises:
(a) the amino acid sequence of light chain CDR1 comprising the amino acid sequence of SEQ ID NO: 28,
(b) the amino acid sequence of light chain CDR2 comprising the amino acid sequence of SEQ ID NO: 29, and
(c) the amino acid sequence of light chain CDR3 comprising the amino acid sequence of SEQ ID NO: 30.

5. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 33,
(b) the amino acid sequence of heavy chain CDR2 comprising the amino acid sequence of SEQ ID NO: 34, and
(c) the amino acid sequence of heavy chain CDR3 comprising the amino acid sequence of SEQ ID NO: 35, and
(ii) the light chain variable region comprises:
(a) the amino acid sequence of light chain CDR1 comprising the amino acid sequence of SEQ ID NO: 38,
(b) the amino acid sequence of light chain CDR2 comprising the amino acid sequence of SEQ ID NO: 39, and
(c) the amino acid sequence of light chain CDR3 comprising the amino acid sequence of SEQ ID NO: 40.

6. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 43,
(b) the amino acid sequence of heavy chain CDR2 comprising the amino acid sequence of SEQ ID NO: 44, and
(c) the amino acid sequence of heavy chain CDR3 comprising the amino acid sequence of SEQ ID NO: 45, and
(ii) the light chain variable region comprises:
(a) the amino acid sequence of light chain CDR1 comprising the amino acid sequence of SEQ ID NO: 48,
(b) the amino acid sequence of light chain CDR2 comprising the amino acid sequence of SEQ ID NO: 49, and
(c) the amino acid sequence of light chain CDR3 comprising the amino acid sequence of SEQ ID NO: 50.

7. The antibody or an antigen-binding fragment thereof according to claim 1, wherein
(i) the heavy chain variable region comprises
(a) the amino acid sequence of heavy chain CDR1 comprising the amino acid sequence of SEQ ID NO: 53,
(b) the amino acid sequence of heavy chain CDR2 comprising the amino acid sequence of SEQ ID NO: 54, and
(c) the amino acid sequence of heavy chain CDR3 comprising the amino acid sequence of SEQ ID NO: 55, and
(ii) the light chain variable region comprises:
(a) the amino acid sequence of light chain CDR1 comprising the amino acid sequence of SEQ ID NO: 58,
(b) the amino acid sequence of light chain CDR2 comprising the amino acid sequence of SEQ ID NO: 59, and
(c) the amino acid sequence of light chain CDR3 comprising the amino acid sequence of SEQ ID NO: 60.

8. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 63,
(b) the amino acid sequence of heavy chain CDR2 comprising the amino acid sequence of SEQ ID NO: 64, and
(c) the amino acid sequence of heavy chain CDR3 comprising the amino acid sequence of SEQ ID NO: 65, and
(ii) the light chain variable region comprises:
(a) the amino acid sequence of light chain CDR1 comprising the amino acid sequence of SEQ ID NO: 68,
(b) the amino acid sequence of light chain CDR2 comprising the amino acid sequence of SEQ ID NO: 69, and
(c) the amino acid sequence of light chain CDR3 comprising the amino acid sequence of SEQ ID NO: 70.

9. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 73,
(b) the amino acid sequence of heavy chain CDR2 comprising the amino acid sequence of SEQ ID NO: 74, and
(c) the amino acid sequence of heavy chain CDR3 comprising the amino acid sequence of SEQ ID NO: 75, and
(ii) the light chain variable region comprises:
(a) the amino acid sequence of light chain CDR1 comprising the amino acid sequence of SEQ ID NO: 78,
(b) the amino acid sequence of light chain CDR2 comprising the amino acid sequence of SEQ ID NO: 79, and
(c) the amino acid sequence of light chain CDR3 comprising the amino acid sequence of SEQ ID NO: 80.

10. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 83,
(b) the amino acid sequence of heavy chain CDR2 comprising the amino acid sequence of SEQ ID NO: 84, and
(c) the amino acid sequence of heavy chain CDR3 comprising the amino acid sequence of SEQ ID NO: 85, and
(ii) the light chain variable region comprises:
(a) the amino acid sequence of light chain CDR1 comprising the amino acid sequence of SEQ ID NO: 88,
(b) the amino acid sequence of light chain CDR2 comprising the amino acid sequence of SEQ ID NO: 89, and
(c) the amino acid sequence of light chain CDR3 comprising the amino acid sequence of SEQ ID NO: 90.

11. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 93,
(b) the amino acid sequence of heavy chain CDR2 comprising the amino acid sequence of SEQ ID NO: 94, and
(c) the amino acid sequence of heavy chain CDR3 comprising the amino acid sequence of SEQ ID NO: 95, and
(ii) the light chain variable region comprises:
(a) the amino acid sequence of light chain CDR1 comprising the amino acid sequence of SEQ ID NO: 98,
(b) the amino acid sequence of light chain CDR2 comprising the amino acid sequence of SEQ ID NO: 99, and
(c) the amino acid sequence of light chain CDR3 comprising the amino acid sequence of SEQ ID NO: 100.

12. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 103,
(b) the amino acid sequence of heavy chain CDR2 comprising the amino acid sequence of SEQ ID NO: 104, and
(c) the amino acid sequence of heavy chain CDR3 comprising the amino acid sequence of SEQ ID NO: 105, and
(ii) the light chain variable region comprises:
(a) the amino acid sequence of light chain CDR1 comprising the amino acid sequence of SEQ ID NO: 108,
(b) the amino acid sequence of light chain CDR2 comprising the amino acid sequence of SEQ ID NO: 109, and
(c) the amino acid sequence of light chain CDR3 comprising the amino acid sequence of SEQ ID NO: 110.

13. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 113,
(b) the amino acid sequence of heavy chain CDR2 comprising the amino acid sequence of SEQ ID NO: 114, and
(c) the amino acid sequence of heavy chain CDR3 comprising the amino acid sequence of SEQ ID NO: 115, and
(ii) the light chain variable region comprises:
(a) the amino acid sequence of light chain CDR1 comprising the amino acid sequence of SEQ ID NO: 118,
(b) the amino acid sequence of light chain CDR2 comprising the amino acid sequence of SEQ ID NO: 119, and
(c) the amino acid sequence of light chain CDR3 comprising the amino acid sequence of SEQ ID NO: 120.

14. The antibody or an antigen-binding fragment thereof according to claim 1, wherein
(i) the heavy chain variable region comprises:
(a) the amino acid sequence of heavy chain CDR1 comprising the amino acid sequence of SEQ ID NO: 123,
(b) the amino acid sequence of heavy chain CDR2 comprising the amino acid sequence of SEQ ID NO: 124, and
(c) the amino acid sequence of heavy chain CDR3 comprising the amino acid sequence of SEQ ID NO: 125, and
(ii) the light chain variable region comprises:
(a) the amino acid sequence of light chain CDR1 comprising the amino acid sequence of SEQ ID NO: 128,
(b) the amino acid sequence of light chain CDR2 comprising the amino acid sequence of SEQ ID NO: 129, and
(c) the amino acid sequence of light chain CDR3 comprising the amino acid sequence of SEQ ID NO: 130.

15. The antibody or an antigen-binding fragment thereof according to any one of claims 1 to 14, wherein the antibody comprises:
(a) a heavy chain variable region (VH) selected from the group consisting of the amino acid sequences of SEQ ID NOs: 2, 12, 22, 32, 42, 52, 62, 72, 82, 92, 102, 112, and 122 and amino acid sequences having 80% or higher identity to any of these amino acid sequences, and
(b) a light chain variable region (VL) selected from the group consisting of the amino acid sequences of SEQ ID NOs: 7, 17, 27, 37, 47, 57, 67, 77, 87, 97, 107, 117, and 127 and amino acid sequences having 80% or higher identity to any of these amino acid sequences.

16. The antibody or an antigen-binding fragment thereof according to claim 15, wherein the antibody comprises:
(a) a heavy chain variable region (VH) comprising the amino acid sequence of SEQ ID NO: 2 or an amino acid sequence having 80% or higher identity to the amino acid sequence of SEQ ID NO: 2, and
(b) a light chain variable region (VL) comprising the amino acid sequence of SEQ ID NO: 7 or an amino acid sequence having 80% or higher identity to the amino acid sequence of SEQ ID NO: 7.

17. The antibody or an antigen-binding fragment thereof according to claim 15, wherein the antibody comprises:
(a) a heavy chain variable region (VH) comprising the amino acid sequence of SEQ ID NO: 12 or an amino acid sequence having 80% or higher identity to the amino acid sequence of SEQ ID NO: 12, and
(b) a light chain variable region (VL) comprising the amino acid sequence of SEQ ID NO: 17 or an amino acid sequence having 80% or higher identity to the amino acid sequence of SEQ ID NO: 17.

18. The antibody or an antigen-binding fragment thereof according to claim 15, wherein the antibody comprises:
(a) a heavy chain variable region (VH) comprising the amino acid sequence of SEQ ID NO: 22 or an amino acid sequence having 80% or higher identity to the amino acid sequence of SEQ ID NO: 22, and
(b) a light chain variable region (VL) comprising the amino acid sequence of SEQ ID NO: 27 or an amino acid sequence having 80% or higher identity to the amino acid sequence of SEQ ID NO: 27.

19. The antibody or an antigen-binding fragment thereof according to claim 15, wherein the antibody comprises:
(a) a heavy chain variable region (VH) comprising the amino acid sequence of SEQ ID NO: 32 or an amino acid sequence having 80% or higher identity to the amino acid sequence of SEQ ID NO: 32, and
(b) a light chain variable region (VL) comprising the amino acid sequence of SEQ ID NO: 37 or an amino acid sequence having 80% or higher identity to the amino acid sequence of SEQ ID NO: 37.

20. The antibody or an antigen-binding fragment thereof according to claim 15, wherein the antibody comprises:
(a) a heavy chain variable region (VH) comprising the amino acid sequence of SEQ ID NO: 42 or an amino acid sequence having 80% or higher identity to the amino acid sequence of SEQ ID NO: 42, and
(b) a light chain variable region (VL) comprising the amino acid sequence of SEQ ID NO: 47 or an amino acid sequence having 80% or higher identity to the amino acid sequence of SEQ ID NO: 47.

21. The antibody or an antigen-binding fragment thereof according to claim 15, wherein the antibody comprises:
(a) a heavy chain variable region (VH) comprising the amino acid sequence of SEQ ID NO: 52 or an amino acid sequence having 80% or higher identity to the amino acid sequence of SEQ ID NO: 52, and
(b) a light chain variable region (VL) comprising the amino acid sequence of SEQ ID NO: 57 or an amino acid sequence having 80% or higher identity to the amino acid sequence of SEQ ID NO: 57.

22. The antibody or an antigen-binding fragment thereof according to claim 15, wherein the antibody comprises:
(a) a heavy chain variable region (VH) comprising the amino acid sequence of SEQ ID NO: 62 or an amino acid sequence having 80% or higher identity to the amino acid sequence of SEQ ID NO: 62, and
(b) a light chain variable region (VL) comprising the amino acid sequence of SEQ ID NO: 67 or an amino acid sequence having 80% or higher identity to the amino acid sequence of SEQ ID NO: 67.

23. The antibody or an antigen-binding fragment thereof according to claim 15, wherein the antibody comprises:
(a) a heavy chain variable region (VH) comprising the amino acid sequence of SEQ ID NO: 72 or an amino acid sequence having 80% or higher identity to the amino acid sequence of SEQ ID NO: 72, and
(b) a light chain variable region (VL) comprising the amino acid sequence of SEQ ID NO: 77 or an amino acid sequence having 80% or higher identity to the amino acid sequence of SEQ ID NO: 77.

24. The antibody or an antigen-binding fragment thereof according to claim 15, wherein the antibody comprises:
(a) a heavy chain variable region (VH) comprising the amino acid sequence of SEQ ID NO: 82 or an amino acid sequence having 80% or higher identity to the amino acid sequence of SEQ ID NO: 82, and
(b) a light chain variable region (VL) comprising the amino acid sequence of SEQ ID NO: 87 or an amino acid sequence having 80% or higher identity to the amino acid sequence of SEQ ID NO: 87.

25. The antibody or an antigen-binding fragment thereof according to claim 15, wherein the antibody comprises:
(a) a heavy chain variable region (VH) comprising the amino acid sequence of SEQ ID NO: 92 or an amino acid sequence having 80% or higher identity to the amino acid sequence of SEQ ID NO: 92, and
(b) a light chain variable region (VL) comprising the amino acid sequence of SEQ ID NO: 97 or an amino acid sequence having 80% or higher identity to the amino acid sequence of SEQ ID NO: 97.

26. The antibody or an antigen-binding fragment thereof according to claim 15, wherein the antibody comprises:
(a) a heavy chain variable region (VH) comprising the amino acid sequence of SEQ ID NO: 102 or an amino acid sequence having 80% or higher identity to the amino acid sequence of SEQ ID NO: 102, and
(b) a light chain variable region (VL) comprising the amino acid sequence of SEQ ID NO: 107 or an amino acid sequence having 80% or higher identity to the amino acid sequence of SEQ ID NO: 107.

27. The antibody or an antigen-binding fragment thereof according to claim 15, wherein the antibody comprises:
(a) a heavy chain variable region (VH) comprising the amino acid sequence of SEQ ID NO: 112 or an amino acid sequence having 80% or higher identity to the amino acid sequence of SEQ ID NO: 112, and
(b) a light chain variable region (VL) comprising the amino acid sequence of SEQ ID NO: 117 or an amino acid sequence having 80% or higher identity to the amino acid sequence of SEQ ID NO: 117.

28. The antibody or an antigen-binding fragment thereof according to claim 15, wherein the antibody comprises:
(a) a heavy chain variable region (VH) comprising the amino acid sequence of SEQ ID NO: 122 or an amino acid sequence having 80% or higher identity to the amino acid sequence of SEQ ID NO: 122, and
(b) a light chain variable region (VL) comprising the amino acid sequence of SEQ ID NO: 127 or an amino acid sequence having 80% or higher identity to the amino acid sequence of SEQ ID NO: 127.

29. The antibody or an antigen-binding fragment thereof according to any one of claims 1 to 28, wherein the epitope of the antibody belongs to any of epitope groups Group 1 typified by anti-hMPV antibodies EV046115b, EV046130, and EV046147, Group 2 typified by anti-hMPV antibodies EV046113, EV046116, EV046141, and EV0461142, Group 3 typified by anti-hMPV antibodies EV046124, EV046143, and EV046150, Group 4 typified by anti-hMPV antibodies EV046120 and EV046135, and Group 5 typified by an anti-hMPV antibody EV046136 in epitope grouping according to binding activity against a partially deleted peptide of the F protein.

30. The antibody or an antigen-binding fragment thereof according to any one of claims 1 to 28, wherein the epitope of the antibody belongs to any of epitope groups Group 2 typified by anti-hMPV antibodies EV046113, EV046116, EV046141, and EV0461142, Group 3 typified by anti-hMPV antibodies EV046124, EV046143, and EV046150, Group 4 typified by anti-hMPV antibodies EV046120 and EV046135, and Group 5 typified by an anti-hMPV antibody EV046136 in epitope grouping according to binding activity against a partially deleted peptide of the F protein.

31. The antibody or an antigen-binding fragment thereof according to any one of claims 1 to 28, wherein the epitope of the antibody belongs to an epitope group Group 3 typified by anti-hMPV antibodies EV046124, EV046143, and EV046150 in epitope grouping according to binding activity against a partially deleted peptide of the F protein.

32. The antibody or an antigen-binding fragment thereof according to any one of claims 1 to 31, wherein the antibody is of IgG1 (κ) or IgG1 (λ) class (subclass).

33. The antibody or an antigen-binding fragment thereof according to any one of claims 1 to 32, wherein the antibody has a neutralizing activity (IC50) of 1 µg/mL (approximately 6.7 nM) or lower against any of human metapneumovirus strains JPS02-76 (type B1), JPS05-21 (type B2), and JPS03-180 (type A1).

34. The antibody or an antigen-binding fragment thereof according to any one of claims 1 to 32, wherein the antibody has a neutralizing activity (IC50) of approximately 1 µg/mL (approximately 6.7 nM) or lower against any of human metapneumovirus strains JPS03-180 (type A1), JPS03-178 (type A2), JPS02-76 (type B1), and JPS05-21 (type B2).

35. The antibody or an antigen-binding fragment thereof according to any one of claims 1 to 32, wherein the antibody has a neutralizing activity (IC90) of approximately 2 µg/mL (approximately 13.3 nM) or lower against any of human metapneumovirus strains JPS03-180 (type A1), JPS03-178 (type A2), JPS02-76 (type B1), and JPS05-21 (type B2).

36. The antibody or an antigen-binding fragment thereof according to any one of claims 1 to 32, wherein the antibody has a neutralizing activity (IC50) of approximately 0.1 µg/mL (approximately 0.67 nM) or lower against a human metapneumovirus JPS03-178 strain (type A2).

37. A pharmaceutical composition comprising an antibody or an antigen-binding fragment thereof according to any one of claims 1 to 36 and a pharmaceutically acceptable carrier.

38. The pharmaceutical composition according to claim 37, wherein the pharmaceutical composition is intended for the treatment or prevention of human metapneumovirus infection.

39. An isolated nucleic acid encoding the amino acid sequence of an antibody or an antigen-binding fragment thereof according to any one of claims 1 to 36, an isolated nucleic acid encoding an amino acid sequence of any of SEQ ID NOs: 1, 6, 11, 16, 21, 26, 31, 36, 41, 46, 51, 56, 61, 66, 71, 76, 81, 86, 91, 96, 101, 106, 111, 116, 121, and 126, or an isolated nucleic acid hybridizing under high stringent conditions to any of these nucleic acids.

40. A recombinant expression vector comprising an isolated nucleic acid according to claim 39 incorporated therein.

41. A host cell comprising a recombinant expression vector according to claim 40 introduced thereinto.
